# EUROPEAN PATENT APPLICATION

(11) **EP 4 230 618 A1**
(43) Date of publication of application: **23.08.2023**
(21) Application number: 21902224.1
(22) Date of filing: 20.10.2021
(51) Int. Cl.: C07D 213/56, C07D 213/81, C07D 213/79, C07D 213/63, C07D 233/64, C07D 237/10, A61K 31/44, A61K 31/65, A61K 31/33, A61P 29/00

(54) **BENZYLAMINE OR BENZYL ALCOHOL DERIVATIVE AND USE THEREOF**

(30) Priority: 08.12.2020 CN 202011424997; 08.05.2021 CN 202110501583
(71) Applicant: Chengdu Hyperway Pharmaceuticals Co., Ltd., Chengdu, Sichuan 610041 (CN); Shenzhen Hyperway Pharmaceuticals Co., Ltd., Shenzhen, Guangdong 518116 (CN)
(72) Inventor: REN, Junfeng, Chengdu, Sichuan 610000 (CN); LIU, Guanfeng, Chengdu, Sichuan 610000 (CN); WU, Xiancai, Chengdu, Sichuan 610000 (CN); DU, Nan, Chengdu, Sichuan 610000 (CN); LI, Pengwu, Chengdu, Sichuan 610000 (CN); YANG, Xiaoyun, Chengdu, Sichuan 610000 (CN); ZHOU, Qingsong, Chengdu, Sichuan 610000 (CN); YUAN, Chenguang, Chengdu, Sichuan 610000 (CN); LI, Yingfu, Chengdu, Sichuan 610000 (CN)
(74) Representative: Ipside
(86) International application number: PCT/CN2021/124838
(87) International publication number: WO 2022/121517

(57) **Abstract**

The present invention discloses a compound as a sodium channel regulator, and this type of compound can effectively inhibit and block the activity of Navl.8 ion channel, a subtype of voltage-gated sodium channels, and can be used as a specific Navl.8 inhibitor and to prepare drugs for the treatment of pain diseases mediated by Navl.8 such as intestinal pain, neuropathic pain, musculoskeletal pain, acute pain, inflammatory pain, cancer pain, primary pain, multiple sclerosis, Charcot-Marie-Tooth syndrome, incontinence, and cardiac arrhythmia, with broad application prospects.

## Description

### Technical Field

The present invention relates to pharmaceutical technology and, in particular, to a compound as a specific inhibitor voltage-gated sodium ion channel subtype Nav1.8 and the uses.

### Technical Background

With the advent of the aging global population, the scope of chronic pain diseases has gradually expanded, more than one-third of the world's population suffer from chronic pain, and the number of arthritis patients in China is as high as 120 million; meanwhile, there are at least 80 to 90 million patients with neuropathic chronic pain in China, and the population is growing; in addition, cancer will eventually become a chronic disease, with the cancer pain tormenting such patients. It is reported that more than half of the cancer patients in China have not received cancer pain treatment. Pain is originally a mechanism for self-protection in animals or human body, however, many persistent pains are beyond their usefulness and bring great pain to patients' physical or mental health. Therefore, pain management is an important factor in improving the quality of life of cancer patients, and its market prospect is huge.

In 2005, the global market for pain relievers totaled more than $80 billion, with prescription drug sales of approximately $20 billion. According to the National Institutes of Health (NIH) statistics, as many as 25 million people in the United States struggle with pain every day, of which 23 million adults suffer from severe pain. In China, with the rising manufacturing industry, the number of industrial workers has expanded dramatically, the long-term labor is prone to various strains and arthralgia, and this group has become another large group of pain medication consumption. Data shows that over-the-counter drug market for painkillers is growing rapidly in China, and its sales are second only to cold medicines, accounting for about 20% of the proportion.

However, the abuse of opioid painkillers has a staggering impact on society. According to WHO estimates, 69,000 people die of opioid overdose and 15 million people are dependent on opioids (i.e., opioid addiction) each year worldwide, and the importance of developing new safe and effective pain medications cannot be overstated.

The inward current of voltage-gated sodium channels (Nav) is an important link in the generation and conduction of action potentials in central and peripheral neurons, and increased neuron excitability or reactivity to stimuli is an important mechanism for the development of various pains. Therefore, the role of voltage-gated sodium channels in pain conduction pathways, especially in peripheral sensory neurons, has been a hot topic in pain research.

There are nine human sodium channels, Nav1.1 to Nav1.9. the Nav1.5, Nav1.8 and Nav1.9 are tetrodotoxin (TTX) insensitive sodium channels, among which Nav1.8 is involved in important ion channels in chronic pain, atrial fibrillation, and Buga syndrome, and are highly selective targets for pain treatment. Nav1.8 is mainly expressed in small-diameter nociceptive neurons and participates in the action potentials and rhythmic firing of sensory neurons. Nav1.8 is regulated by inflammatory mediators and is also upregulated in sciatic nerve injury models, and studies of knockdown and silencing Nav1.8 suggest that it is involved in the regulation of neuropathic and inflammatory pain. Since Nav1.8 is primarily localized to pain-sensing neurons, selective Nav1.8 blockers are unlikely to induce the adverse effects common to non-selective Nav blockers. Therefore, research on screening specific inhibitor on Nav1.8 pain target has become a hot topic; however, the known Nav inhibitors mainly suffer from disadvantages such as narrow therapeutic window, which may be due to their lack of subtype selectivity.

Currently known Nav1.8 inhibitors include PF-01247324, A803467, PF-06305591, and VX150. Among these, the first three compounds were abandoned in the preclinical phase due to insufficient selectivity, poor pharmacokinetic data and low bioavailability. The study of VX-150 is currently in phase II of clinical trials and has met primary clinical endpoints including acute post-surgical pain, chronic pain in osteoarthritis, and neuropathic pain. VX-150 was well tolerated, with no serious adverse events. The FDA has granted VX-150 as breakthrough therapy for the treatment of moderate to severe pain. However, due to the large clinical dose of VX-150 at 1500 mg first dose followed by 750 mg every 12 hours, its activity needs to be further improved. Also, the dissolution and absorption of the compound has been more problematic, and although partially addressed by the prodrug approach, it is still not ideal.

Therefore, the development of Nav1.8 inhibitors with higher affinity, high specificity, and better pharmacokinetics has great social and economic value.

### Contents of the invention

The main technical problem addressed by the present invention is to provide a benzylamine or benzyl alcohol derivative with strong selective inhibition on Nav1.8.

To solve the above technical problems, the present invention provides a compound characterized by having the structure shown in formula I or its tautomer, mesomer, racemate, enantiomer, diastereoisomer or its mixture form, and pharmaceutically acceptable hydrate, solvate or salt. 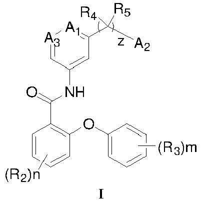
A₁ and A₃ are independently selected from CR₁or N;
A₂ is selected from NR₁₀R₁₁or OR₁₂;
R₁, R₂, R₃, R₄, R₅ are independently selected from hydrogen, halogen, hydroxyl, cyano group, amino, substituted or unsubstituted alkyl, substituted or unsubstituted cycloalkyl, substituted or unsubstituted heteroalkyl, substituted or unsubstituted heterocycloalkyl substituted or unsubstituted aryl, substituted or unsubstituted heteroaryl, substituted or unsubstituted amido, ester, acyl, carboxyl, sulfonyl, sulfonamido, boronic acid group, boronic acid ester group, phosphoryl, substituted or unsubstituted alkenyl, substituted or unsubstituted alkynyl.
R₁₀, R₁₁ and R₁₂ are independently selected from hydrogen, halogen, hydroxy, cyano group, amino, substituted or unsubstituted alkyl, substituted or unsubstituted cycloalkyl, substituted or unsubstituted heteroalkyl, substituted or unsubstituted heterocycloalkyl, substituted or unsubstituted aryl, substituted or unsubstituted heteroaryl, ester, acyl, carboxyl, acylamino, sulfonyl, sulfonamido, boronic acid group, boronic acid ester group, phosphoryl;
Or, R₄, R₅ and their connected atoms constitute substituted or non-substituted 3 to 10-membered cycloalkyls and =O; or, R₄, R₅ and their connected atoms constitute substituted or non-substituted 3 to 10-membered heterocycloalkyls, or R₅, Rn and their connected atoms constitute substituted or non-substituted 3 to 10-membered heterocycloalkyls, or R₅, R₁₂ and their connected atoms constitute substituted or non-substituted 3 to 10-membered heterocycloalkyls.
Wherein R₁, R₂, R₃, R₄, R₅, R₁₀, R₁₁, R₅, R₁₁ and the substituents in the heterocycloalkyl group constituted by the connected atoms are independently selected from deuterium, halogen, hydroxyl, amino, alkyl, heteroalkyl, cycloalkyl, heterocycloalkyl, cyano group, ester, acyl, carboxyl, amido, aryl, heteroaryl, sulfonyl, sulfonamido;
Z is selected from 0, 1, 2, 3, 4, and 5, n is selected from 0, 1, 2, 3, and 4, and m is selected from 0, 1, 2, 3, 4, and 5.

The present invention also provides the structure shown in formula II or II' or their tautomers, mesomers, racemates, enantiomers, diastereoisomers or its mixture forms, and pharmaceutically acceptable hydrates, solvates or salts.

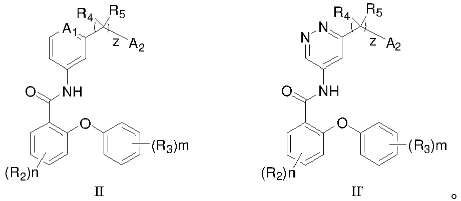

The present invention also provides the structure shown in formula III or III' or their tautomers, mesomers, racemates, enantiomers, diastereoisomers or its mixture forms, and pharmaceutically acceptable hydrates, solvates or salts: 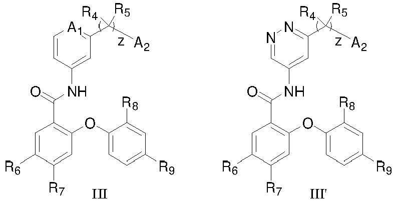
A₁ is selected from CR₁ or N; A₂ is selected from NR₁₀R₁₁or OR₁₂;
R₁ is selected from hydrogen, halogen, hydroxyl, cyano group, amino, substituted or unsubstituted alkyl, substituted or unsubstituted cycloalkyl, substituted or unsubstituted heteroalkyl, substituted or unsubstituted heterocycloalkyl, ester, acyl, carboxyl, acylamino, sulfonyl, sulfonamido, boronic acid group, boronic acid ester, and phosphoryl;
R₆, R₇, R₈, R₉ are independently selected from hydrogen, halogen, hydroxyl, cyano group, amino, substituted or unsubstituted alkyl, substituted or unsubstituted cycloalkyl, substituted or unsubstituted heteroalkyl, substituted or unsubstituted heterocycloalkyl, substituted or unsubstituted aryl, substituted or unsubstituted substituted heteroaryl, ester, acyl, carboxyl, acylamino, sulfonyl, sulfonamido, boronic acid group, boronic acid ester group, phosphoryl group, substituted or non-substituted alkenyl group, and substituted or non-substituted alkynyl group; wherein, the substituents are selected from deuterium, halogen, hydroxyl, amino, alkyl, heteroalkyl, cycloalkyl, heterocycloalkyl, cyano group, ester, acyl, carboxyl, acylamino, aryl, heteroaryl, sulfonyl, sulfonamido.

Further, n is selected from 0, 1, and 2.

Further, m is selected from 0, 1, and 2.

Further, z is selected from 0, 1, 2, and 3.

Wherein, the values of n, m and z can be arbitrarily combined.
R₁ is selected from hydrogen, halogen, hydroxyl, cyano group, amino, substituted or unsubstituted alkyl, substituted or unsubstituted cycloalkyl, substituted or unsubstituted heteroalkyl, substituted or unsubstituted heterocycloalkyl, substituted or unsubstituted acylamino, ester, acyl, carboxyl, sulfonyl, sulfonamido, boronic acid group, boronic acid ester group, and phosphoryl; wherein, the substituents are selected from halogen, hydroxyl, amino , C1-C6 alkyl, 3 to 6-membered cycloalkyls, 2 to 7-membered heteroalkyls, 3 to 6-membered heterocycloalkyls, cyano group, ester, acyl, carboxyl, amido, aryl, heteroaryl, sulfonyl, sulfonamido;
Wherein, R₁ is selected from hydrogen, halogen, hydroxyl, cyano group, amino, substituted or unsubstituted C1-C6 alkyl, substituted or unsubstituted 3 to 6-membered cycloalkyls, substituted or unsubstituted 2 to 7-membered heteroalkyls, substituted or unsubstituted 3 to 6-membered heterocycloalkyls, substituted or unsubstituted acylamino, ester, acyl; wherein the substituent is selected from halogen, hydroxyl, amino, C1~ C6 alkyl, 3 to 6-membered cycloalkyl, 2 to 7-membered heteroalkyl, 3 to 6-membered heterocycloalkyl, cyano, ester, acyl, carboxyl, acylamino, sulfonyl, and sulfonamido;

Further, R₁ is selected from hydrogen, halogen, substituted or non-substituted C1-C3 alkyl, substituted or non-substituted 2- to 4-membered heteroalkyls; wherein, the substituents are selected from halogen, hydroxyl, amino, C1-C3 alkyl, 2- to 4-membered heteroalkyls, and acyl.

Further, R₁ is selected from hydrogen and halogen.

Further, R₁ is selected from H and F.

In one aspect, n is 1 or 2 and each R₂ is independently selected from hydrogen, halogen, substituted or non-substituted C1-C3 alkyl, and substituted or non-substituted cyclopropyl.

Further, each R₂ is independently selected from hydrogen, halogen, trifluoromethyl, substituted or non-substituted cyclopropyl; when n is 1, R₂ is not H; when n is 2, both R₂ are not H simultaneously.

In one aspect, m is 2 or 3, each R₃ is independently selected from halogen, substituted or unsubstituted alkyl, substituted or unsubstituted cycloalkyl, substituted or unsubstituted heteroalkyl, and substituted or unsubstituted heterocycloalkyl, wherein, the substituents are selected from deuterium, C1-C6 alkyl, 2 to 7-membered heteroalkyls, 3 to 6-membered cycloalkyls, 3 to 6-membered heterocycloalkyls, halogen, hydroxyl, and acylamino; each R₃ is different.

Further, each R₃ is independently selected from hydrogen, halogen, substituted or non-substituted C1-C6 alkyl, substituted or non-substituted C1-C6 alkoxy, wherein, the substituents are selected from deuterium, halogen, C1-C6 alkyl, 2 to 7-membered heteroalkyls, 3 to 6-membered cycloalkyls, 3 to 6-membered heterocycloalkyls, hydroxyl, and acylamino.

Further, each R₃ is independently selected from halogen, substituted or non-substituted C1-C3 alkyl, substituted or non-substituted C1-C3 alkoxy.

Further, when R₄, R₅ and their connected atoms constitute cycloalkyl or heterocycloalkyl, it can be that a cycloalkyl or a heterocycloalkyl (e.g., 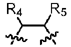 constituting 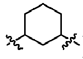 ) is constituted by a R₄ and a R₅ located in two adjacent substitution sites, and their connected atoms, or that a cycloalkyl or a heterocycloalkyl (e.g., 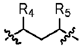 constituting 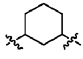 ) is constituted by a R₄ and a R₅ located in two non-adjacent substitution sites, and their connected atoms, or that a cycloalkyl or a heterocycloalkyl (e.g., 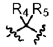 constituting 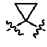 ) is constituted by a R₄ and a R₅ located in the same substitution site, and their connected atoms.

R₄, R₅ and their connected atoms constitute = O, say, R₄, R₅ and their connected atoms in the same substitution site constitute a carbonyl group 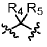 constituting 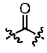 ).

In one aspect , the present invention also provides the structure shown in formula IV, IV' or IV" or their isomers, tautomers, mesomers, racemates, enantiomers, diastereoisomers or its mixture forms, pharmaceutically acceptable hydrates, solvates or salts.

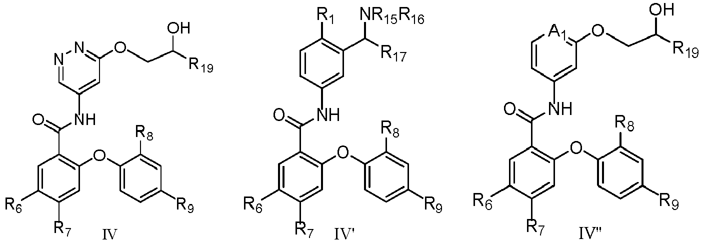

Wherein,
A₁ is selected from N or -CR₁; R₁₅ and R₁₆ are independently selected from hydrogen, C1-C3 alkyl, further selected from hydrogen, and methyl;
R₁₇ is selected from C1-C5 acylamino, 4-membered or 5-membered heterocyclic ring containing lactam, C1-C5 alkyl substituted by at least hydroxyl or amino; or, one of R₁₅ or R₁₆, and R₁₇ and its connected atoms constitute 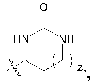 Z₃ is selected from 0, 1 or 2; further, R₁₇ is selected from C1-C3 acylamino, 4-membered or 5-membered heterocyclic ring containing lactam, C1-C3 alkyl groups substituted by at least hydroxyl or amino; or, either R₁₅ or R₁₆, R₁₇ and their connected atoms constitute 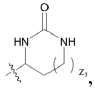 Z₃ is selected from 0, 1 or 2;
R₁₉ is selected from 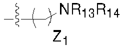 and 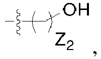 R₁₃ and R₁₄ are independently selected from hydrogen, acyl, substituted or non-substituted alkyl, substituted or non-substituted cycloalkyl, substituted or non-substituted heterocycloalkyl, substituted or non-substituted heteroaryl, Z₁ and Z₂ are independently 1-5.

Further, R₁₃ and R₁₄ are independently selected from hydrogen, acyl, 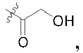 C1-C5 alkyl or cycloalkyl, thiazole.

On the other hand, the present invention also provides the structure shown in formula V or V' or their tautomers, mesomers, racemates, enantiomers, diastereoisomers or its mixture forms, and pharmaceutically acceptable hydrates, solvates or salts:

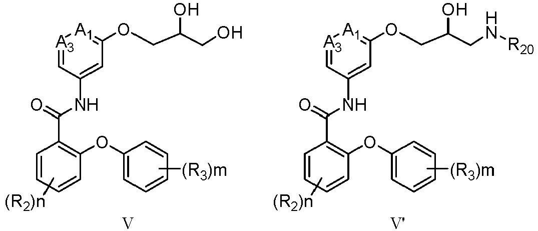

Wherein, A₁ and A₃ is selected from CR1; or, A₁ is selected from N and A₃ is selected from N or CR1;

R₂₀ are independently selected from hydrogen, acyl, 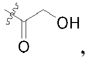 C1-C5 alkyl or cycloalkyl, and thiazole.

The present invention also provides a pharmaceutical composition, the active ingredient of which is selected from the compounds described in any one of claims 1 to 21, or their stereoisomers, solvates, hydrates, and pharmaceutically acceptable salts or co-crystals.

The present invention also provides the uses of the compounds or their stereoisomers, solvates, hydrates, and pharmaceutically acceptable salts or co-crystals in the preparation of sodium ion channel regulators.

Further, the stated sodium ion channel regulator is a Nav1.8 inhibitor.

The present invention also provides the uses of the compounds or their stereoisomers, solvates, hydrates, and pharmaceutically acceptable salts or co-crystals in the preparation a drug for the treatment of diseases causing Nav1.8 overexpression.

The present invention also provides the uses of the compounds or their stereoisomers, solvates, hydrates, and pharmaceutically acceptable salts or co-crystals in the preparation a drug for the treatment of diseases caused by Nav1.8 overexpression.

The present invention also provides the uses of the compounds or their stereoisomers, solvates, hydrates, and pharmaceutically acceptable salts or co-crystals in the preparation of a drug for one or more of the following diseases: chronic pain, intestinal pain, neuropathic pain, musculoskeletal pain, acute pain, inflammatory pain, cancer pain, primary pain, multiple sclerosis, Charcot-Marie-Tooth syndrome, incontinence, and cardiac arrhythmia.

Further, the stated neuropathic pain is selected from one or more of the following diseases: postherpetic neuralgia, diabetic neuralgia, painful HIV-associated sensory neuropathy, trigeminal neuralgia, burning mouth syndrome, post-amputation pain, phantom pain, painful neuroma, traumatic neuroma, Morton's neuroma, nerve crush injury, spinal canal stenosis, carpal tunnel syndrome, radiculalgia, sciatica, nerve avulsion injury, brachial plexus avulsion, complex regional pain syndrome, neuralgia caused by drug therapy, cancer chemotherapy and antiretroviral therapy, pain after spinal cord injury, primary small fiber neuropathy, primary sensory neuropathy, trigeminal autonomic headache;
The stated musculoskeletal pain is selected from one or more of the following: osteoarthritis pain, back pain, cold pain, burn pain, and dental pain;
The stated inflammatory pain is selected from rheumatoid arthritis pain and/or vulvodynia;

The stated primary pain is selected from fibromyalgia.

The pharmaceutical compositions containing the compounds provided by the present invention or their stereoisomer, solvates, hydrates, and pharmaceutically acceptable salts or a co-crystals, may contain pharmaceutically acceptable excipients.

The term "pharmaceutically acceptable" in the present invention refers to any material that does not interfere with effectiveness of biological activity of active ingredients and is not toxic to the host to which it is administered.

Pharmacologically acceptable excipients mentioned in the present invention is a general term for all additional materials in the drug other than the active pharmaceutical ingredient, the excipients should have the following properties: (1) It has no toxicity to the human body, and almost no side effects; (2) It's chemically stable, not easily affected by temperature, pH, and storage time, etc.; (3) It has no incompatibility with the active pharmaceutical ingredient, and does not affect efficacy and quality analysis thereof; (4) It shows no interaction with packaging materials. The excipients in this invention include but not limited to fillers (diluents), lubricants (glidants or anti-adhesive agents), dispersants, wetting agents, adhesives, regulators, solubilizers, antioxidants, antibacterial agents, emulsifiers, and disintegrants, etc. Adhesives include sugar syrup, Arabic gum, gelatin, sorbitol, astragalus gum, cellulose and its derivatives (such as microcrystalline cellulose, sodium carboxymethyl cellulose, ethyl cellulose or hydroxypropyl methyl cellulose, etc.), gelatin syrup, sugar syrup, starch syrup or polyvinylpyrrolidone, etc.; fillers contain lactose, powdered sugar, dextrin, starch and its derivatives, cellulose and its derivatives, inorganic calcium salts (such as calcium sulfate, calcium phosphate, calcium phosphate, calcium hydrogen phosphate, precipitated calcium carbonate, etc.), sorbitol or glycine, etc.; lubricants include micronized silica gel, magnesium stearate, talc, aluminum hydroxide, boric acid, hydrogenated vegetable oil, polyethylene glycol, etc.; disintegrants include starch and its derivatives (such as sodium carboxymethyl starch, sodium starch glycolate, pre-gelatinized starch, modified starch, hydroxypropyl starch, corn starch, etc.), polyvinylpyrrolidone or microcrystalline cellulose, etc. Wetting agents include sodium dodecyl sulfate, water or alcohol, etc.; antioxidants include sodium sulfite, sodium bisulfite, sodium metabisulfite, dibutylbenzene acid, etc.; bacterial inhibitors include 0.5% phenol, 0.3% cresol, 0.5% trichloro-tert-butanol, etc.; regulators include hydrochloric acid, citric acid, potassium (sodium) hydroxide, sodium citrate and buffers (including sodium dihydrogen phosphate and disodium hydrogen phosphate), etc.; emulsifiers include polysorbate 80, sorbitan oleate (span 80) , Pluronic^{™} F-68, lecithin, soy phospholipids, etc.; solubilizers include tween-80, bile, glycerol, etc.

The term "pharmaceutically acceptable salt" refers to salt formed by the compound of the present invention with acid or alkali suitable for use as a drug. The acids and bases mentioned above are broadly defined as lewis acid and lewis alkali. Acids suitable for salt formation include but not limited to inorganic acids such as hydrochloric, hydrobromic, hydrofluoric, sulfuric, nitric, and phosphoric acids; organic acids such as formic, acetic, propionic, oxalic, malonic, succinic, fumaric, maleic, lactic, malic, tartaric, citric, picric, methanesulfonic, benzenemethanesulfonic, and benzenesulfonic acids; and acidic amino acids such as aspartic and glutamic acids.

There are no particular limitations on the administration method of the compounds or pharmaceutical compositions of the invention, representative methods include but not limited to oral, parenteral (intravenous, intramuscular or subcutaneous), and topical administration.

Solid dosage forms used for oral administration include capsules, tablets, pills, powders, and granules. In these solid dosage forms, the active compound is mixed with at least one conventional inert excipient (or carrier), such as sodium citrate or dicalcium phosphate, or with (a) fillers or bulking agents, e.g., starch, lactose, sucrose, glucose, mannitol, and silicic acid; (b) binders, e.g., hydroxymethyl cellulose, alginate, gelatin, polyvinylpyrrolidone, sucrose, and arabic gum; (c) humectants, e.g., glycerin; (d) disintegrants, e.g., agar, calcium carbonate, potato starch or tapioca starch, alginic acid, certain composite silicates, and sodium carbonate; (e) retarders, e.g., paraffin; (f) absorption accelerator agents, e.g., quaternary amine compounds; (g) wetting agents, e.g., cetearyl alcohol and glycerol monostearate; (h) adsorbents, e.g., kaolin; and (i) lubricants, e.g., talc, calcium stearate, talc, calcium stearate, magnesium stearate, solid polyethylene glycol, sodium dodecyl sulfate, or their mixtures. As for capsules, tablets, and pills, the dosage form may also contain a buffering agent.

Solid dosage forms such as tablets, sugar pills, capsules, pills and granules can be prepared using coating and shell materials such as enteric coatings and other materials well known in the field. They may contain opacifiers, and the active compound or compounds in such compositions may be released in a delayed manner in a part of digestive tract. Examples of embedded components used are polymeric substances and waxes. If necessary, the active compound combined with one or more of the above excipients may also be made in microcapsule form.

Liquid dosage forms for oral administration includes pharmaceutically acceptable emulsions, solutions, suspensions, syrups, or tinctures. In addition to the active compound, the liquid dosage form may include inert diluents routinely adopted in the area, such as water or other solvents, and solubilizers and emulsifiers, e.g., ethanol, isopropyl alcohol, ethyl carbonate, ethyl acetate, propylene glycol, 1,3-butanediol, dimethylformacylamino, and oils, particularly cottonseed oil, peanut oil, maize germ oil, olive oil, castor oil and sesame oil or mixtures of these substances.

In addition to these inert diluents, the compositions may also comprise adjuvants such as wetting agents, emulsifiers and suspensions, sweeteners, flavorings, and fragrances.

In addition to the active compounds, the suspension liquid may comprise suspending agents such as ethoxylated isooctadecanol, polyoxyethylene sorbitol and dehydrated sorbitol esters, microcrystalline cellulose, and methanolic aluminum and agar or mixtures of these substances.

Compositions for parenteral injection may comprise physiologically acceptable sterile aqueous or anhydrous solutions, dispersions, suspension liquid or emulsions, as well as sterile powders to be redissolved into sterile injectable solutions and dispersions. Suitable aqueous and non-aqueous carriers, diluent, and solvent or excipients including water, ethanol, polyols, and their suitable mixtures.

Dosage forms of the compounds of the present invention for topical administration include ointment, powders, patch, sprays and inhalants. The active ingredient is mixed under sterile conditions with carrier and any preservative and buffering agent which are physiologically acceptable, or with propellants that may be required if necessary.

The compounds of the present invention can likewise be used in injectable preparations. Wherein, the stated injection is selected from liquid injection (injection), sterile powder for injection (powder injection) or tablet for injection (refer to the molded or machine-pressed tablet made in aseptic operation, dissolved in water for injection for subcutaneous or intramuscular injection when ready for use).

Wherein, the stated powder for injection contains at least an excipient in addition to the above compounds. The excipient, in the invention, is a composition purposively added to drug, and should not have pharmacological properties in the amount used. However, the excipient may contribute to the processing, dissolving or dissoluting of the drug, and may contribute to stability through targeted administration route.

Functional Group isomers resulted from the rapid movement of an atom in a molecule from one position to another position is called tautomer.

Mesomer contains asymmetric atoms in the molecule, but has a symmetry factor that makes the total optical rotation in the molecule zero, that is, no optical rotation.

Racemate is an equimolar mixture of a chiral molecule (see chirality) with its enantiomer (see optical isomerism).

Stereoisomers with real object and mirror image of each other are of non-overlappable, and called enantiomers. The enantiomeric isomers all have optical activity, of real object and mirror image in an enantiomeric isomer, one is levorotatory and the other is dextorotatory, so the enantiomeric isomers are also called optical isomers.

Diastereoisomers refers to stereisomer that has two or more chiral centers which are of non-mirroring relationship between the molecules thereof.

The term "Substitution" refers to the replacement of a hydrogen atom in a molecule by a different atom or molecule.

The term "member" refers to the number of skeleton atoms that make up ring or heteroalkyl group.

The term "one bond" in the present invention refers to only one linkage bond therein, which can also be understood as "none".

The term "Alkyl" means an hydrocarbon group, refers to saturated hydrocarbyl. The alkyl portion may be straight-chain alkyl or branched alkyl. Typical alkyl groups include but are not limited to methyl, ethyl, propyl, isopropyl, butyl, isobutyl, tert-butyl, pentyl, and hexyl.

C1-Cn in the present invention includes C1-C2, C1-C3 ......C1-Cn, with n being an integer greater than one; the prefixes as substituents indicate the minimum and maximum number of carbon atoms in substituent, for example, "C1-C6 alkyl" refers to a straight or branched alkyl group containing one to six carbon atoms.

The term "heteroalkyl" refers to alkyl group containing heteroatoms, including alkoxy.

The term "alkenyl" refers to aliphatic hydrocarbon group having at least one carbon-carbon double bond. The stated alkenyl may be straight-chain or branched.

The term "alkynyl" refers to aliphatic hydrocarbon group having at least one carbon-carbon triple bond. The stated alkynyl may be straight-chain or branched.

The term "acylamino" is a chemical structure having the formula -C(O)NHR or -NHC(O)R, wherein R can be selected from alkyl, heteroalkyl, cycloalkyl, heterocycloalkyl, aryl, heteroaryl, etc.

The term "sulfonyl" is a chemical structure having the formula -S(=O)₂R, including sulfonamido, wherein R can be selected from alkyl, heteroalkyl, cycloalkyl, heterocycloalkyl, aryl, heteroaryl, amino, etc.

The term "phosphoryl" is a chemical structure having the formula -P(=O)RR', wherein R and R' are independently selected from alkyl, heteroalkyl, cycloalkyl, heterocycloalkyl, aryl, heteroaryl, hydroxy, amino, etc.

The term "ester" is a chemical structure having the formula -COOR, wherein R is selected from alkyl, heteroalkyl, cycloalkyl, heterocycloalkyl, aryl, heteroaryl, etc.

The term "acyl" is a chemical structure having the formula -C (O)R, wherein R is selected from alkyl, heteroalkyl, cycloalkyl, heterocycloalkyl, aryl, heteroaryl, etc.

The term "cycloalkyl" refers to saturated or unsaturated cyclic hydrocarbon substituent.

The term "heterocycloalkyl" refers to cycloalkyl having at least one heteroatom in the cyclic skeleton.

Heteroatoms include but are not limited to O, S, N, P, and Si.

The "cyclo" refers to any covalent closed structure, including carbocycle (e.g., aryl or cycloalkyl), heterocycle (e.g., heteroaryl or heterocycloalkyl), aromatic group (e.g., aryl or heteroaryl), or non-aromatic group (e.g., cycloalkyl or heterocycloalkyl). The "cyclo" referred to in the present invention may be monocyclic or polycyclic, and to be parallel, spiro or bridge ring.

The typical cycloalkyl include but are not limited to cyclopropyl, cyclobutyl, cyclopentyl, 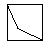 , and

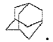

Typical heterocycloalkyl include but are not limited to:

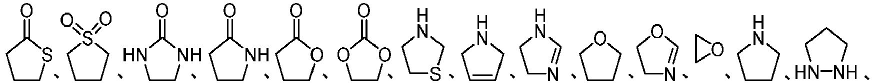

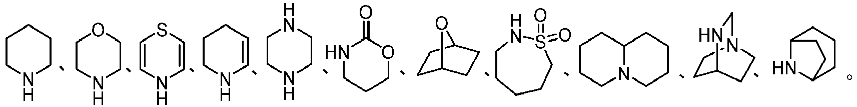

The term "aryl" refers to that a planar ring having a delocalized π-electron system and 4n+2π electrons, where n is an integer. Aryl ring may comprise five, six, seven, eight, nine or more than nine atoms. Aryl group include but are not limited to phenyl, naphthyl, phenanthryl, anthryl, fluorenyl, and indenyl.

Typical heteroaryl include but are not limited to:

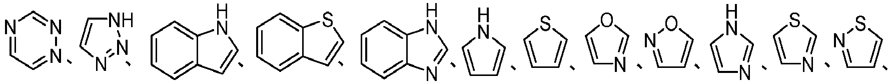

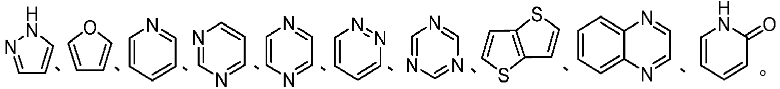

The term "halogen" refers to fluorine, chlorine, bromine or iodine.

The term "deuterium" refers to the isotope of hydrogen (H), also known as heavy hydrogen, generally with the elemental symbol D or ²H. Deuterium may be used to replace position of H in the invention.

The alkyl, heteroalkyl, cyclic group, heterocyclic, amino, ester, carbonyl, acylamino, sulfonyl, phosphoryl, boronic acid group, boronic acid ester group, aryl, and heteroaryl, as stated in the invention, may be non-substituted- alkyl, heteroalkyl, cyclic group, heterocyclic, amino, ester, carbonyl, acylamino, sulfonyl, phosphoryl, boronic acid group, boronic acid ester group, aryl, and heteroaryl, or substituted- alkyl, heteroalkyl, cyclic group, heterocyclic, amino, ester, carbonyl, acylamino, sulfonyl, phosphoryl, boronic acid group, boronic acid ester group, aryl, and heteroaryl.

In the foregoing, except as already indicated, the stated "substituted-" means that the mentioned group could be substituted by one or more additional groups, the additional groups are independently selected from alkyl, cycloalkyl, aryl, carboxyl, heteroaryl, heterocycloalkyl, hydroxy, alkoxy, alkylthio, aryloxy, O=, guanidinyl, cyano group, nitro , acyl, halogen, haloalkyl, amino, etc.

Inhibitors refers to a products that cause a reduction in biological activity of protein, such as the compounds in the present invention reduces Nav1.8 ion channel activity, namely Nav1.8 inhibitors.

The beneficial effect of the present invention is that the present invention provides a series of compounds with significant inhibitory effect on Nav1.8 ion channel activity, and provides a new treatment means for the disesase such as chronic pain, intestinal pain, neuropathic pain, musculoskeletal pain, acute pain, inflammatory pain, cancer pain, primary pain, multiple sclerosis, Charcot-Marie-Tooth syndrome, incontinence or arrhythmia. This series of compounds can be used to prepare drugs for the treatment of related diseases and have broad applications.

### Description of the preferred examples

The technical solutions of the present invention are clearly and completely described below, and it is clear that the described examples are only a part of the examples of the invention. Based on the examples in the invention, all other examples obtained by general technicians in this field without making creative work shall fall within the scope of protection of the invention.

In the invention, the structures of the compounds are determined by mass spectrum (MS) and/or nuclear magnetic resonance (1HNMR). The chemical abbreviations have the following meanings:
DMF: N, N- dimethylformamide
THF: Tetrahydrofuran
DIAD: Diisopropyl azodicarboxylate
DIPEA: N, N-diisopropylethylamine
PE: Petroleum ether
EA: Ethyl acetate
DCM: Dichloromethane
HATU: O-(7-azabenzotriazol-1-yl)-N, N, N', N'- tetramethylurea
DMSO: Dimethyl sulfoxide
DAST: Diethylamino sulfur trifluoride
TBAF: Tetrabutylammonium fluoride
NMP: N-methyl-pyrrolidone
CDI: N, N'-carbonyl diimidazole
LiHMDS: Lithium bis (trimethylsilyl) amine

### Intermediate synthesis

### Intermediate 1

### 2-(4-fluoro-2-methylphenoxy)-4-(trifluoromethyl) benzoic acid

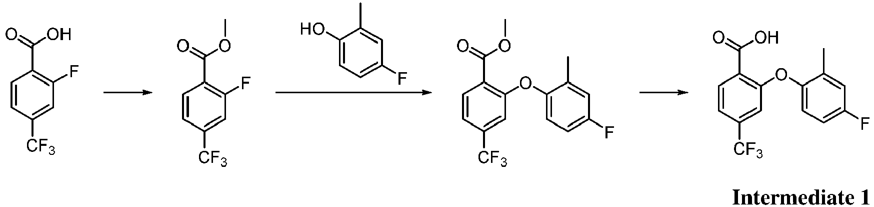

### Step 1: Synthesis of methyl 2-fluoro-4-(trifluoromethyl) benzoate

Add 2-fluoro-4-(trifluoromethyl) benzoic acid (2.08 g, 10 mmol), potassium carbonate (2.76 g, 20 mmol) and 20 mL acetonitrile to a reaction flask, and then add iodomethane (1.70 g, 12 mmol) at room temperature. Heat the reaction mixture to reflux and stirr for 6 h. After TLC shows that the reaction is complete, cool the reaction mixture to room temperature and filter, collect the filtrat, remove the solvent by rotary evaporation to give 2.10 g of the target product, with the 95% yield.

### Step 2: Synthesis of methyl 2-(4-fluoro-2-methylphenoxy)-4-(trifluoromethyl) benzoate

Add Methyl 2-fluoro-4-(trifluoromethyl) benzoate (1.11 g, 5 mmol), 4-fluoro-2-methylphenol (0.63 g, 5 mmol), potassium carbonate (1.38 g, 10 mmol), and 10 mL N-methylpyrrolidone to a reaction flask, heat the reaction mixture to 120°C and stir for 20 h. After TLC shows that the reaction is complete, cool the reaction mixture to room temperature and pour into 50 mL water, extract with ethyl acetate, dry, remove the solvent by rotary evaporation, and purify by silica gel column chromatography (PE/EA=20/1) to give 1.3 g of the target product, with the yield of 79%.

### Step 3: Synthesis of 2-(4-fluoro-2-methylphenoxy)-4-(trifluoromethyl) benzoic acid

Add 2-(4-fluoro-2-methylphenoxy)-4-(trifluoromethyl) methyl benzoate (656 mg, 2 mmol), sodium hydroxide (320 mg, 8 mmol), 10 mL of water and 10 mL of methanol to a reaction flask, stir the reaction mixture at room temperature for 8 h. After TLC shows that the reaction is complete, poure the reaction mixture into 50 mL of water, adjust pH to 5 with 1 M hydrochloric acid, extract with ethyl acetate, dry, remove the solvent by rotary evaporation to give 620 mg of the target product, with the yield of 99%.

### Intermediate 2

### 2-(4-fluoro-2-methylphenoxy)-5-(trifluoromethyl) benzoic acid

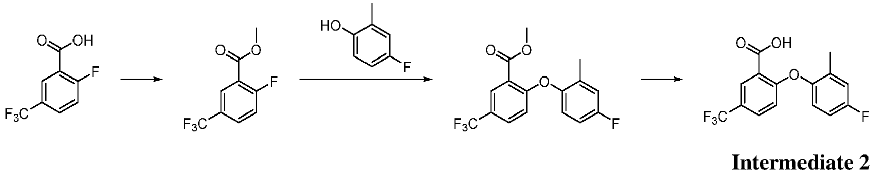

The operation was the same as that of intermediate 1.

### Intermediate 3

### 4-cyclopropyl-2-(4-fluoro-2-methylphenoxy)-5-(trifluoromethyl) benzoic acid

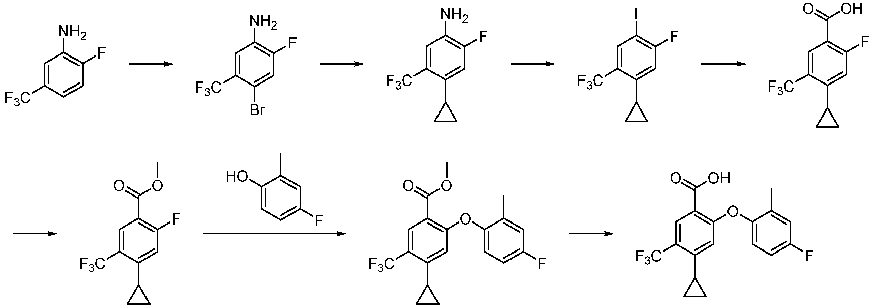

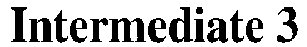

### Step 1: Synthesis of 4-bromo-2-fluoro-5-(trifluoromethyl) aniline

Dissolve 2-fluoro-5-(trifluoromethyl) aniline (3.58 g, 20 mmol) in 50 mL DMF, add NBS (3.56 g, 20 mmol) at room temperature, stir the reaction mixture at room temperature overnight. Pour reaction mixture into 200 mL of water, extract with ethyl acetate, dry, remove the solvent by rotary evaporation, and purify by silica gel column chromatography (PE/EA=2/1) to give 4.80 g of the target product, with the yield of 93%.

### Step 2: Synthesis of 4-cyclopropyl-2-fluoro-5-(trifluoromethyl) aniline

Add 4-bromo-2-fluoro-5-(trifluoromethyl) aniline (2.58 g, 10 mmol), cyclopropylboronic acid (3.44 g, 40 mmol), sodium carbonate (5.30 g, 50 mmol) and 30 mL DMF to a reaction flask, charge with nitrogen, add Pd(dppf)Cl₂ (220 mg, 0.3 mmol). Heat the reaction mixture under nitrogen protection to 90 °C and stirr overnight. Cool the reaction mixture to room temperature and pour into 200 mL of water, extract with ethyl acetate, dry the organic phase, remove the solvent by rotary evaporation, and purify by silica gel column chromatography (PE/EA=2/1) to give 1.62 g of the target product, with the yield of 74%.

### Step 3: Synthesis of 1-iodo-4-cyclopropyl-2-fluoro-5-(trifluoromethyl) benzene

Dissolve 4-cyclopropyl-2-fluoro-5-(trifluoromethyl) aniline (1.10 g, 5 mmol) in 15 mL acetonitrile, drop isoamyl nitrite (0.70 g, 6 mmol) at 0°C, stir at room temperature for 1 h. Add Potassium iodide (1.66 g, 10 mmol) in 5 mL of aqueous solution, and stir the reaction mixture at room temperature for 6 h. Poured into 50 mL of water, extract with ethyl acetate, and wash the organic phase with 1 M dilute hydrochloric acid, 1 M aqueous sodium carbonate and saturated NaCl solution successively, dry, and remove the solvent by rotary evaporation to give 1.05 g of the target product, with the yield of 61%.

### Step 4: Synthesis of 4-cyclopropyl-2-fluoro-5-(trifluoromethyl) benzoic acid

Dissolve 1-iodo-4-cyclopropyl-2-fluoro-5-(trifluoromethyl) benzene (660 mg, 2 mmol) and triethylamine (404 mg, 4 mmol) in DMF/H₂O (10 mL/1 mL), then add Pd(dppf)Cl₂(73 mg, 0.1 mmol), charge carbon monoxide to replace the air in the system, heat the reaction mixture to 90 °C under 1 MPa pressure of carbon monoxide and stirr overnight. Cool the reaction mixture to room temperature and pour into 50 mL of water, extract with ethyl acetate, and dry the organic phase, remove the solvent by rotary evaporation, and purify by silica gel column chromatography (DCM/MeOH=10/1) to give 190 mg of the target product, with the yield of 38%.

### Step 5: Synthesis of 4-cyclopropyl-2-(4-fluoro-2-methylphenoxy)-5-(trifluoromethyl) benzoic acid

The product of the previous step was used as the substrate to obtain the compound of intermediate 3 by the same method as that of intermediate 1

### Intermediate 4

### 5-Chloro-2-(4-fluoro-2-methylphenoxy)-4-(trifluoromethyl) benzoic acid

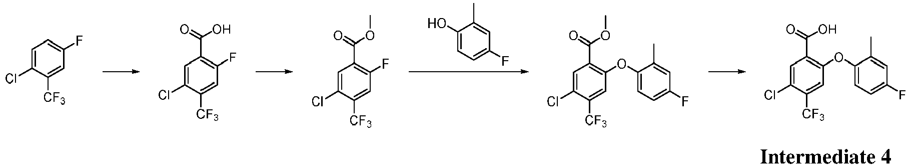

### Step 1: Synthesis of 4-bromo-2-fluoro-5-(trifluoromethyl) aniline

Dissolve 1-Chloro-4-fluoro-2-(trifluoromethyl) benzene (2.0 g, 10.1 mmol) in 20 mL of dry THF and cool to -70 °C under nitrogen protection. Drop 2,2,6,6-tetramethylpiperidinolithium (11.1 mL, 1.0 M hexane solution, 11.1 mmol), stir the reaction solution at -70 °C for 0.5 h, add excess amount of dry ice. Stand, till the temperature reaches room temperature, add 100 ml of water, adjust the pH of the mixture to acidic, and extract with ethyl acetate. Dry the organic phase, remove the solvent by rotary evaporation, and purify by silica gel column chromatography (DCM/MeOH=10/1) to give 1.8 g of the target product, with the yield of 75%.

### Step 2: Synthesis of 5-chloro-2-(4-fluoro-2-methylphenoxy)-4-(trifluoromethyl) benzoic acid

The product of the previous step is used as the substrate to obtain the compound of intermediate 3 by the same method as that of intermediate 1

### Example 1

### N-(3-(1,3-diamino-3-oxopropyl)-4-fluorophenyl)-2-(4-fluoro-2-methylphenoxy)-5-(trifluoro methyl) benzamide

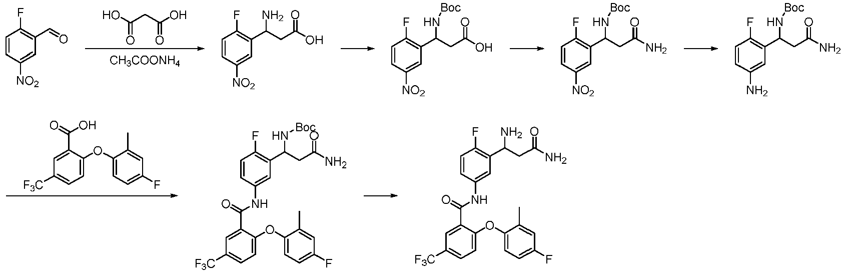

### Step 1: Synthesis of 3-amino-3-(2-fluoro-5-nitrophenyl) propionic acid

Add 2-fluoro-5-nitrobenzaldehyde (2000 mg, 11.8 mmol), malonic acid (1477 mg, 14.2 mmol), ammonium acetate (1823 mg, 10 mmol) and 20 mL of ethanol to a reaction flask, heat the reaction mixture to 80°C and stirr overnight. After TLC shows that the reaction is complete, cool the reaction mixture to room temperature and filter, wash the filter cake three times with ethanol, dry, and get about 3000 mg of yellow solid, the crude product is used directly in the next reaction step.

### Step 2: Synthesis of 3-((tert-butoxycarbonyl) amino)-3-(2-fluoro-5-nitrophenyl) propanoic acid

Add 3-amino-3-(2-fluoro-5-nitrophenyl) propionic acid (3000 mg, crude), (Boc)₂O (4716 mg, 21.6 mmol) and 30 mL of water to a reaction flask, then add sodium hydroxide (1154 mg, 28.9 mmol), stir the reaction mixture at room temperature overnight, remove the solvent by rotary evaporation, and purify by silica gel column chromatography (DCM/MeOH=20/1) to give 780 mg of pale yellow solid, with the two-step yield of 20%.

### Step 3: Synthesis of (3-amino-1-(2-fluoro-5-nitrophenyl)-3-oxopropyl) tert-butyl carbamate

Add 3-((tert-butoxycarbonyl) amino)-3-(2-fluoro-5-nitrophenyl) propanoic acid (100 mg, 0.35 mmol), ammonium chloride (185 mg, 3.5 mmol), DIPEA (89 mg, 0.69 mmol) and 2 mL tetrahydrofuran to a reaction flask, then add HBTU (261 mg, 0.69 mmol), and stir the reaction mixture at room temperature overnight. Poured into 10 mL of water, extract with ethyl acetate, dry the organic phase, remove the solvent by rotary evaporation, and purify by Prep-TLC (DCM/MeOH=20/1) to give 75 mg of pale yellow solid, with the yield of 75%.

### Step 4: Synthesis of (3-amino-1-(5-amino-2-fluorophenyl)-3-oxopropyl) tert-butyl carbamate

Add (3-amino-1-(2-fluoro-5-nitrophenyl)-3-oxopropyl) tert-butyl carbamate (75 mg, 0.23 mmol) and 2 mL of ethanol to a reaction flask, then add 10% Pd/C (50 mg), and stir the reaction mixture at room temperature under hydrogen atmosphere overnight. After vacuum filtration, wash the filter cake with ethanol, collect the filtrate, remove the solvent by rotary evaporation to give 70 mg of white solid, with the yield of 103%.

### Step 5: Synthesis of tert-butyl (3-amino-1-(2-fluoro-5-(2-(4-fluoro-2-methylphenoxy)-5-(trifluoromethyl) benzamido) phenyl)-3-oxopropyl) carbamate

Add 2-(4-fluoro-2-methylphenoxy)-5-(trifluoromethyl) benzoic acid (89 mg, 0.28 mmol), (3-amino-1-(5-amino-2-fluorophenyl)-3-oxopropyl) tert-butyl carbamate (70 mg, 0.23 mmol), DIPEA (61 mg, 0.47 mmol) and 2 mL of tetrahydrofuran to a reaction flask, then add HBTU (179 mg, 0.47 mmol), and stir the reaction mixture at room temperature overnight, then poured into 10 mL of water, extract with ethyl acetate. Dry the organic phase and remove the solvent by rotary evaporation, and purify by Prep-TLC (DCM/MeOH=50/1) to give 50 mg of pale yellow solid, with the yield of 36%.

### Step 6: Synthesis of N-(3-(1,3-diamino-3-oxopropyl)-4-fluorophenyl)-2-(4-fluoro-2-methylphenoxy)-5-(trifluorometh yl) benzoyl

Dissolve Tert-butyl (3-amino-1-(2-fluoro-5-(2-(4-fluoro-2-methylphenoxy)-5-(trifluoromethyl) benzamido) phenyl)-3-oxopropyl) carbamate (50 mg, 0.084 mmol) in 2 mL of dichloromethane, add trifluoroacetic acid (2 mL) at room temperature, stir overnight. Pour the mixture into 10 mL of water, adjust pH to basic by sodium carbonate, extract with ethyl acetate, dry the organic phase, remove the solvent by rotary evaporation, and purify by Prep-TLC (DCM/MeOH=20/1) to give 8 mg as a white solid, with yield of 20%.
LC/MS: m/z=494.2 [M+H]⁺.
LC/MS: m/z=494.2 [M+H]⁺.
1H NMR (400 MHz, d₆-DMSO) δ 2.16 (3H, s), 2.53-2.55 (2H, m), 4.46 (1H, brs), 6.77 (1H, d, *J* = 8.4 Hz), 7.10-7.24 (4H, m), 7.17-7.19 (1H, m), 7.27 (1H, d, *J* = 8.8 Hz), 7.80-7.85 (1H, m), 7.95 (1H, s), 10.61 (1H, s).
1H NMR (400 MHz, d₆-DMSO) δ 2.16 (3H, s), 2.53-2.55 (2H, m), 4.46 (1H, brs), 6.77 (1H, d, J= 8.4 Hz), 7.10-7.24 (4H, m), 7.17-7.19 (1H, m), 7.27 (1H, d, J= 8.8 Hz), 7.80-7.85 (1H, m), 7.95 (1H, s), 10.61 (1H, s).

### Example 2

### 3-Amino-3-(2-fluoro-5-(2-(4-fluoro-2-methylphenoxy)-5-(trifluoromethyl)benzamido) phenyl) propanoic acid

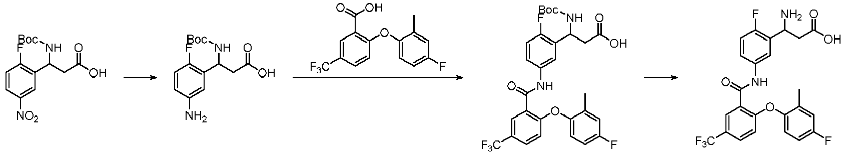

### Step 1: Synthesis of 3-(5-amino-2-fluorophenyl)-3-((tert-butoxycarbonyl) amino) propionic acid

Add 3-((tert-butoxycarbonyl) amino)-3-(2-fluoro-5-nitrophenyl) propionic acid (100 mg, 0.34 mmol) and 5 mL of ethanol to a reaction flask, then add 10% Pd/C (70 mg). Stir the reaction mixture at room temperature overnight under hydrogen atmosphere. Filter, wash the filter cake with ethanol, collect the filtrate and remove the solvent by rotary evaporation to give 95 mg of white solid, with the yield of 100%.

### Step 2: Synthesis of 3-((tert-butoxycarbonyl) amino)-3-(2-fluoro-5-(2-(4-fluoro-2-methylphenoxy)-5-(trifluoromethyl) benzamido) phenyl) propanoic acid

Add 2-(4-fluoro-2-methylphenoxy)-5-(trifluoromethyl) benzoic acid (110 mg, 0.35 mmol) and 2 mL of sulfoxide chloride to a reaction flask, stirr at 80 °C for 2 h and remove the solvent by rotary evaporation. Dissolve the resulting solid in 2 mL of tetrahydrofuran and drop into 1 mL of tetrahydrofuran solution containing 3-(5-amino-2-fluorophenyl)-3-(tert-butoxycarbonyl) amino) propanoic acid (95 mg, 0.31 mmol) and DIPEA (82 mg, 0.64 mmol) at room temperature. Stir the reaction mixture at room temperature for 2 hours, pour into 10 mL of water, extract with ethyl acetate. Dry the organic phase, remove the solvent by rotary evaporation, and purify by Prep-TLC (DCM/MeOH=20/1) to give 100 mg of pale yellow solid, with the yield of 53%.

### Step 3: Synthesis of

### (3-Amino-3-(2-fluoro-5-(2-(4-fluoro-2-methylphenoxy)-5-(trifluoromethyl) benzamido) phenyl) propanoic acid

Dissolve 3-((tert-butoxycarbonyl) amino)-3-(2-fluoro-5-(2-(4-fluoro-2-methylphenoxy)-5-(trifluoromethyl) benzamido) phenyl) propanoic acid (100 mg, 0.17 mmol) in 1 mL of dichloromethane, add trifluoroacetic acid (1 mL) at room temperature, then stir overnight. Pour the reaction mixture into 10 mL of water, adjust pH to basic using sodium carbonate, extract with ethyl acetate, dry the organic phase, remove the solvent by rotary evaporation, and purify by Prep-TLC (DCM/MeOH=20/1) to give 9 mg of white solid, with the yield of 11%.

LC/MS: m/z=495.1 [M+H]⁺.

1H NMR (400 MHz, d₆-DMSO) δ 2.16 (3H, s), 2.47-2.49 (2H, m), 4.45 (1H, brs), 6.78 (1H, d, *J*= 8.4 Hz), 7.10-7.24 (5H, m), 7.17-7.19 (1H, m), 7.25 (1H, d, *J*= 8.4 Hz), 7.82-7.85 (1H, m), 7.95 (1H, s), 10.55 (1H, s), 13.08 (1H, brs).

### Example 3

### N-(3-(1-Amino-3-hydroxypropyl)-4-fluorophenyl)-4-cyclopropyl-2-(4-fluoro-2-methylpheno xy)-5-(trifluoromethyl) benzamide

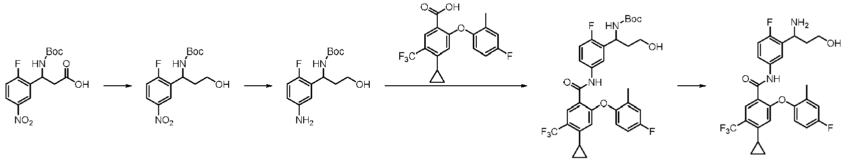

### Step 1: Synthesis of tert-butyl (1-(2-fluoro-5-nitrophenyl)-3-hydroxypropyl) carbamate

Dissolve 3-((tert-butoxycarbonyl) amino)-3-(2-fluoro-5-nitrophenyl) propionic acid (50 mg, 0.15 mmol) in 1 mL of tetrahydrofuran, add borane tetrahydrofuran solution (1 M, 1 mL) at 0 °C. Stir the reaction mixture at room temperature overnight. pour into 10 mL of water, and extract with ethyl acetate. Dry the organic phase and remove the solvent by rotary evaporation and purify by Prep-TLC (DCM/MeOH=30/1) to give 30 mg of white solid, with the yield of 63%.

### Step 2: Synthesis of tert-butyl (1-(5-amino-2-fluorophenyl)-3-hydroxypropyl) carbamate

Add Tert-butyl (1-(2-fluoro-5-nitrophenyl)-3-hydroxypropyl) carbamate (30 mg, 0.096 mmol) and 2 mL of ethanol to a reaction flask, then add 10% Pd/C (20 mg). Stir the reaction mixture at room temperature under hydrogen atmosphere overnight. Filter, wash the cake with ethanol, combine the filtrate, remove the solvent by rotary evaporation to give 25 mg of white solid, with the yield of 93%.

### Step 3: Synthesis of tert-butyl (1-(5-(4-(4-cyclopropyl-2-(4-fluoro-2-methylphenoxy)-5-(trifluoromethyl) benzamido)-2-fluorophenyl)-3-hydroxypropyl) carbamate

Add 4-cyclopropyl-2-(4-fluoro-2-methylphenoxy)-5-(trifluoromethyl) benzoic acid (30 mg, 0.085 mmol), 1-(5-amino-2-fluorophenyl)-3-hydroxypropyl) carbamic acid tert-butyl ester (25 mg, 0.088 mmol), DIPEA (23 mg, 0.18 mmol) and 2 mL of tetrahydrofuran to a reaction flask, then add HATU (67 mg, 0.18 mmol). Stir the reaction mixture at room temperature overnight, pour into 10 mL of water, and extract with ethyl acetate. Dry the organic phase, remove the solvent by rotary evaporation, and purify by Prep-TLC (DCM/MeOH=50/1) to give 35 mg as a white solid, with the yield of 66%.

### Step 4: Synthesis of N-(3-(1-amino-3-hydroxypropyl)-4-fluorophenyl)-4-cyclopropyl-2-(4-fluoro-2-methylphenoxy)-5 -(trifluoromethyl) benzamide

Dissolve Tert-butyl (1-(5-(4-(4-cyclopropyl-2-(4-fluoro-2-methylphenoxy)-5-(trifluoromethyl) benzamido)-2-fluorophenyl)-3-hydroxypropyl) carbamate (35 mg, 0.056 mmol) in 1 mL of dichloromethane, add trifluoroacetic acid (1 mL) at room temperature, then stir overnight. Pour the reaction mixture into 10 mL of water, adjust pH to basic using sodium carbonate, extract with ethyl acetate. Dry the organic phase, remove the solvent by rotary evaporation and purify by Prep-TLC (DCM/MeOH=20/1) to give 18 mg of white solid, with the yield of 62%.

LC/MS: m/z=521.2 [M+H]⁺.

1H NMR (400 MHz, d₆-DMSO) δ 0.55-0.57 (2H, m), 1.01-1.04 (2H, m), 1.79-1.82 (2H, m), 2.12-2.14 (1H, m), 2.16 (3H, s), 3.37-3.40 (2H, m), 4.14- 4.17 (1H, m), 6.35 (1H, s), 7.02-7.12 (3H, m), 7.20 (1H, d, *J*= 8.4 Hz), 7.49-7.52 (1H, m), 7.74-7.76 (1H, m), 7.90 (1H, s), 10.43 (1H, s).

### Example 4

### N-(3-(1,3-Diamino-2-methyl-3-oxopropyl)-4-fluorophenyl)-2-(4-fluoro-2-methylphenoxy)-5 -(trifluoromethyl) benzamide

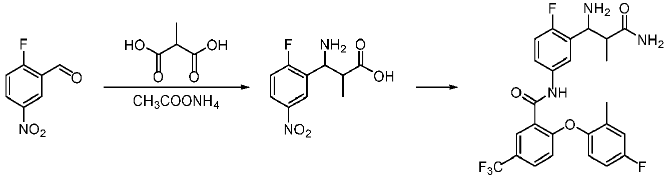

### Step 1: Synthesis of (3-amino-3-(2-fluoro-5-nitrophenyl)-2-methylpropanoic acid

Add 2-fluoro-5-nitrobenzaldehyde (2000 mg, 11.8 mmol), 2-methylmalonic acid (1676 mg, 14.2 mmol), ammonium acetate (1823 mg, 10 mmol) and 20 mL of ethanol to a reaction flask. Heat the reaction mixture to 80 °C and stir overnight. After TLC shows that the reaction is complete, cool the reaction mixture to room temperature and filter, wash the filter cake three times with ethanol. The resulting solid was purify by Prep-HPLC to give 620 mg of white solid, with the yield of 23%.

### Step 2: Synthesis of N-(3-(1,3-diamino-2-methyl-3-oxopropyl)-4-fluorophenyl)-2-(4-fluoro-2-methylphenoxy)-5-(trifl uoromethyl) benzamide

The product of the previous step is used as the substrate to obtain the compound of Example 4 by the same method as that of Example 1

### LC/MS: m/z=508.2 [M+H]⁺.

1H NMR (400 MHz, d₆-DMSO) δ 1.04 (3H, d, *J*= 7.2 Hz), 2.16 (3H, s), 2.48-2.53 (1H, m), 4.25 (1H, d,*J*= 6.0 Hz), 6.70 (1H, s), 6.81 (1H, d,*J*= 8.4 Hz), 7.07-7.30 (5H, m), 7.60-7.64 (1H, m), 7.76-7.79 (2H, m), 7.96 (1H, d,*J*= 2.4 Hz), 10.51 (1H, s).

### Example 5

### N-(3-(1-Amino-3-methoxypropyl)-4-fluorophenyl)-4-cyclopropyl-2-(4-fluoro-2-methylphen oxy)-5-(trifluoromethyl) benzamide

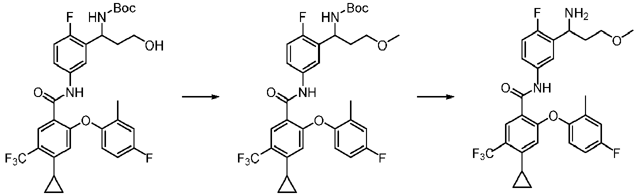

### Step 1: Synthesis of (tert-butyl (1-(5-(4-cyclopropyl-2-(4-fluoro-2-methylphenoxy)-5-(trifluoromethyl) benzamido)-2-fluorophenyl)-3-methoxypropyl) carbamate

Add 1-(5-(4-(4-cyclopropyl-2-(4-fluoro-2-methylphenoxy)-5-(trifluoromethyl)benzamido)-2-fluorophe nyl)-3-hydroxypropyl) tert-butyl carbamate (100 mg, 0.16 mmol), DIPEA (41 mg, 0.32 mmol) and 5 mL of dichloromethane to a reaction flask, and drop methylflavonyl chloride (22 mg, 0.19 mmol) at room temperature. Stir the reaction mixture for 1 h at room temperature, remove the solvent by rotary evaporation, then add 5 mL of methanolic solution of sodium methanol (7 M). Heat the resulting mixture to 70 °C and stir overnight, then pour into 10 mL of water, extract with ethyl acetate. Dry the organic phase and remove the solvent by rotary evaporation to give 80 mg of yellow oily substance, the substance was directly used in the next step.

### Step 2: Synthesis of (N-(3-(1-amino-3-methoxypropyl)-4-fluorophenyl)-4-cyclopropyl-2-(4-fluoro-2-methylphenoxy)-5-(trifluoromethyl) benzamide

Dissolve (Tert-butyl (1-(5-(4-cyclopropyl-2-(4-fluoro-2-methylphenoxy)-5-(trifluoromethyl) benzamido)-2-fluorophenyl)-3-methoxypropyl) carbamate (70 mg, crude product from previous step) in 2 mL of hydrogen chloride-dioxane solution (2 M), stir at room temperature for 2 h. Remove the solvent by rotary evaporation, then add 10 mL of water, adjust pH to basic by sodium carbonate, and extracted with ethyl acetate. Dry the organic phase, remove the solvent by rotary evaporation and purify by Prep-HPLC to give 3 mg of white solid, with two-step yield of 4%.

LC/MS: m/z=535.2 [M+H]⁺.

1H NMR (400 MHz, d₆-DMSO) δ 0.56-0.57 (2H, m), 1.02-1.04 (2H, m), 1.74-1.76 (2H, m), 2.13-2.15 (1H, m), 2.16 (3H, s), 3.19 (3H, s), 3.20-3.30 (2H m), 4.14-4.16 (1H, m), 6.35 (1H, s), 7.05-7.10 (3H, m), 7.20 (1H, d, *J*= 8.8 Hz), 7.49-7.52 (1H, m), 7.74-7.76 (1H, m), 7.90 (1H, s), 10.37 (1H, s).

### Example 6

### N-(3-(1-Acetylamino-3-hydroxypropyl)-4-fluorophenyl)-4-cyclopropyl-2-(4-fluoro-2-methyl phenoxy)-5-(trifluoromethyl) benzamide

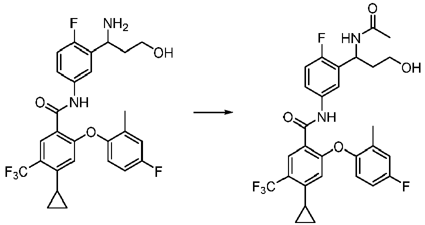

Dissolve N-(3-(1-amino-3-hydroxypropyl)-4-fluorophenyl)-4-cyclopropyl-2-(4-fluoro-2-methylphenoxy)-5 -(trifluoromethyl) benzamide (50 mg, 0.096 mmol) and DIPEA (32 mg, 0.29 mmol) in 2 mL of dichloromethane, drop acetyl chloride (9 mg, 0.12 mmol) at room temperature. After stir for 1 h, add 10 mL of water, and extracted with ethyl acetate. Dry the organic phase, remove the solvent by rotary evaporation, and purify by Prep-HPLC to give 8 mg of white solid, with yield of 15%.

LC/MS: m/z=563.2 [M+H]⁺.

1H NMR (400 MHz, d₆-DMSO) δ 0.55-0.57 (2H, m), 1.01-1.04 (2H, m), 1.75-1.80 (2H, m), 1.82 (3H, s), 2.07-2.10 (1H, m), 2.16 (3H, s), 3.37-3.39 (2H m), 4.51-4.53 (1H, m), 5.04-5.09 (1H, m), 6.33 (1H, s), 7.06-7.10 (3H, m), 7.20 (1H, d,*J*= 8.8 Hz), 7.49-7.52 (1H, m), 7.62-7.66 (1H, m), 7.90 ( 1H, s), 8.33 (1H, d,*J*= 7.6 Hz), 10.40 (1H, s).

### Example 7

### 4-Cyclopropyl-2-(4-fluoro-2-methylphenoxy)-N-(4-fluoro-3-(3-hydroxy-1-(methylamino) propyl) phenyl)-5-(trifluoromethyl) benzamide

### Example 8

### 4-Cyclopropyl-N-(3-(1-(dimethylamino)-3-hydroxypropyl)-4-fluorophenyl)-2-(4-fluoro-2-m ethylphenoxy)-5-(trifluoromethyl) benzamide

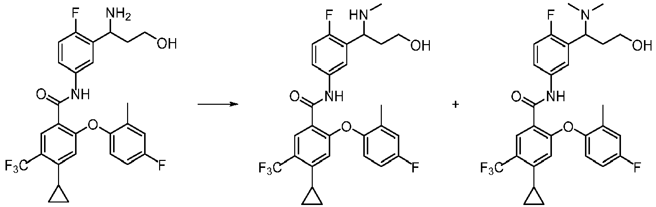

Dissolve N-(3-(1-amino-3-hydroxypropyl)-4-fluorophenyl)-4-cyclopropyl-2-(4-fluoro-2-methylphenoxy)-5 -(trifluoromethyl) benzamide (50 mg, 0.096 mmol) and potassium carbonate (26 mg, 0.19 mmol) in 2 mL acetonitrile, add iodomethane (13 mg, 0.096 mmol). Stir at room temperature for 6 h, pour into 10 mL water, and extracted with ethyl acetate. Dry the organic phase, remove the solvent by rotary evaporation, and purify by Prep-HPLC to obtain the product.

Example 7 data: 5 mg white solid, with yield of 10%

LC/MS: m/z=535.2 [M+H]⁺.

1H NMR (400 MHz, d₆-DMSO) δ 0.56-0.58 (2H, m), 1.03-1.05 (2H, m), 1.74-1.77 (1H, m), 1.90-2.00 (1H, m), 2.13-2.15 (1H, m), 2.16 (3H, s), 2.24 (3H , s), 3.35-3.43 (2H, m), 4.08 (1H, brs), 6.36 (1H, s), 7.06-7.10 (2H, m), 7.15-7.22 (2H, m), 7.51-7.55 (1H, m), 7.83 (1H, d,J= 4.4 Hz), 7.92 (1H, s ), 10.45 (1H, s).

Example 8 data: 6 mg white solid, with yield of 12%

LC/MS: m/z=549.2 [M+H]⁺.

1H NMR (400 MHz, d₆-DMSO) δ 0.56-0.58 (2H, m), 1.04-1.07 (2H, m), 1.74-1.83 (2H, m), 2.13-2.15 (1H, m), 2.16 (3H, s), 2.20 (6H, s), 3.35-3.43 (2H , m), 4.11 , m), 4.11 (1H, brs), 6.37 (1H, s), 7.06-7.10 (2H, m), 7.16-7.22 (2H, m), 7.51-7.55 (1H, m), 7.81-7.83 (1H, m), 7.91 (1H, s), 10.39 (1H, s).

### Example 9

### 4-Cyclopropyl-N-(3-(1,2-diamino-2-oxoethyl)-4-fluorophenyl)-2-(4-fluoro-2-methylphenox y)-5-(trifluoromethyl) benzamide

### Example 10

### N-(3-(2-amino-1-(methylamino)-2-oxoethyl)-4-fluorophenyl)-4-cyclopropyl-2-(4-fluoro-2-m ethylphenoxy)-5-(trifluoromethyl) benzamide

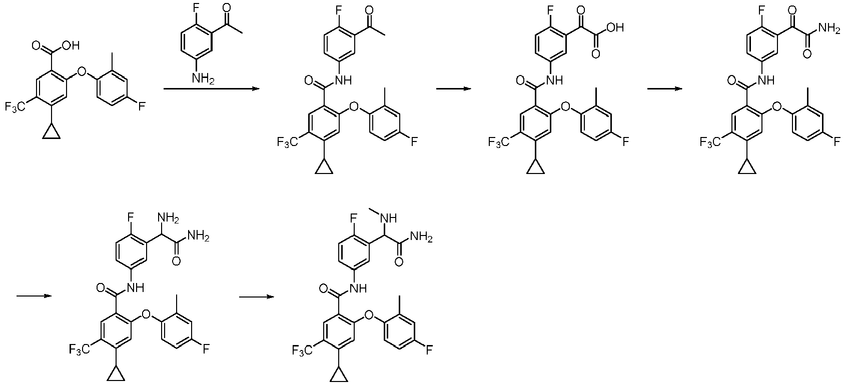

### Step 1: Synthesis of N-(3-acetyl-4-fluorophenyl)-4-cyclopropyl-2-(4-fluoro-2-methylphenoxy)-5-(trifluoromethyl) benzamide

Dissolve 4-cyclopropyl-2-(4-fluoro-2-methylphenoxy)-5-(trifluoromethyl) benzoic acid (81 mg, 0.23 mmol), 5-amino-2-fluoroacetophenone (70 mg, 0.46 mmol) and DIPEA (89 mg, 0.69 mmol) in 3 mL DMF, add HATU (175 mg, 0.46 mmol). Stir the reaction mixture at room temperature overnight, pour into 10 mL of water, and extract with ethyl acetate. Dry the organic phase, remove the solvent by rotary evaporation, and purify by TLC (PE/EA=10/1) to give 125 mg of white solid.

### Step 2: Synthesis of 2-(5-(4-cyclopropyl-2-(4-fluoro-2-methylphenoxy)-5-(trifluoromethyl) benzamido)-2-fluorophenyl)-2-oxoacetic acid

Dissolve N-(3-acetyl-4-fluorophenyl)-4-cyclopropyl-2-(4-fluoro-2-methylphenoxy)-5-(trifluoromethyl) benzamide (125 mg, 0.26 mmol) in 3 mL pyridine, add selenium dioxide (57 mg, 0.51 mmol). Stir the reaction mixture at 100 °C overnight. Remove the solvent by rotary evaporation, then add 10 mL water, adjust pH to acidic with 1 M dilute hydrochloric acid, and extract with ethyl acetate. Dry the organic phase, remove the solvent by rotary evaporation, and purify by Prep-TLC (DCM/MeOH=10/1) to give 100 mg of white solid, with the yield of 75%.

### Step 3: Synthesis of N-(3-(2-amino-2-oxoacetyl)-4-fluorophenyl)-4-cyclopropyl-2-(4-fluoro-2-methylphenoxy)-5-(trifl uoromethyl) benzamide

Dissolve 4-cyclopropyl-2-(5-(4-cyclopropyl-2-(4-fluoro-2-methylphenoxy)-5-(trifluoromethyl) benzamido)-2-fluorophenyl)-2-oxoacetic acid (100 mg, 0.19 mmol), ammonium chloride (102 mg, 1.9 mmol) and DIPEA (50 mg, 0.38 mmol) in 3 mL DMF, add HATU (144 mg, 0.38 mmol). Stir the reaction mixture at room temperature overnight, pour into 10 mL of water, and extract with ethyl acetate. Dry the organic phase, remove the solvent by rotary evaporation, and purify by Prep-TLC (PE/EA=10/1) to give 50 mg of white solid, with the yield of 50%.

### Step 4: Synthesis of 4-cyclopropyl-N-(3-(1,2-diamino-2-oxoethyl)-4-fluorophenyl)-2-(4-fluoro-2-methylphenoxy)-5-(t rifluoromethyl)benzamide

Dissolve N-(3-(2-amino-2-oxoacetyl)-4-fluorophenyl)-4-cyclopropyl-2-(4-fluoro-2-methylphenoxy)-5-(trifl uoromethyl) benzamide (50 mg, 0.096 mmol) and hydroxylamine hydrochloride (27 mg, 0.38 mmol) in 3 mL methanol, stirr at 60°C for 20 min, add sodium acetate (32 mg, 0.38 mmol). Cool the reaction mixture to room temperature, add 2 mL formic acid and 100 mg of zinc powder, stir at room temperature for 2 h, pour into 10 mL of water, and extract with ethyl acetate. Dry the organic phase, remove the solvent by rotary evaporation, and purify by Prep-TLC (DCM/MeOH=10/1) to give 12 mg of white solid, with the yield of 25%.

LC/MS: m/z=520.2 [M+H]⁺.

1H NMR (400 MHz, d₆-DMSO) δ 0.55-0.57 (2H, m), 1.01-1.04 (2H, m), 2.10-2.12 (1H, m), 2.14 (3H, s), 4.48 (1H, s), 6.33 (1H, s), 7.07-7.13 (3H, m), 7.18-7.22 (2H, m), 7.46 (1H, s), 7.55-7.58 (1H, m), 7.68-7.71 (1H, m), 7.89 (1H, s), 10.44 (1H, s).

### Step 5: Synthesis of N-(3-(2-amino-1-(methylamino)-2-oxoethyl)-4-fluorophenyl)-4-cyclopropyl-2-(4-fluoro-2-methyl phenoxy)-5-(trifluoromethyl) benzamide

Dissolve 4-cyclopropyl-N-(3-(1,2-diamino-2-oxoethyl)-4-fluorophenyl)-2-(4-fluoro-2-methylphenoxy)-5-(t rifluoromethyl) benzamide (10 mg, 0.019 mmol) and potassium carbonate (6 mg, 0.043 mmol) in 2 mL acetonitrile, add iodomethane (2.6 mg, 0.018 mmol). Stir the mixture at room temperature for 6 h, then add 10 mL of water, and extract with ethyl acetate. Dry the organic phase, remove the solvent by rotary evaporation, and purify by Prep-HPLC to give 4 mg of white solid, with the yield of 33%.

LC/MS: m/z=534.2 [M+H]⁺.

1H NMR (400 MHz, d₆-DMSO) δ 0.55-0.57 (2H, m), 1.02-1.04 (2H, m), 2.10-2.12 (1H, m), 2.14 (3H, s), 2.22 (3H, s), 4.20 (1H, s), 6.33 (1H, s), 7.07-7.27 (5H, m), 7.47 (1H, s), 7.55-7.58 (1H, m), 7.68-7.71 (1H, m), 7.90 (1H, s), 10.42 (1H, s).

### Example 11

### N-(3-(1-Amino-2-hydroxyethyl)-4-fluorophenyl)-4-cyclopropyl-2-(4-fluoro-2-methylphenox y)-5-(trifluoromethyl) benzamide

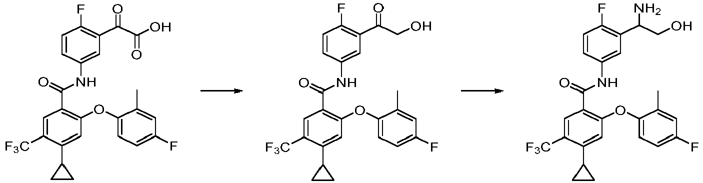

### Step 1: Synthesis of 4-cyclopropyl-2-(4-fluoro-2-methylphenoxy)-N-(4-fluoro-3-(2-hydroxyacetyl) phenyl)-5-(trifluoromethyl) benzamide

Dissolve 2-(5-(4-Cyclopropyl-2-(4-fluoro-2-methylphenoxy)-5-(trifluoromethyl) benzamido)-2-fluorophenyl)-2-oxoacetic acid (52 mg, 0.1 mmol) in 1 mL tetrahydrofuran, then add borane tetrahydrofuran solution (1 M, 1 mL) at 0°C. Stir the reaction mixture for 2h at room temperature, poured into 10 mL of water, and extract with ethyl acetate. Dry the organic phase and remove the solvent by rotary evaporation and purify by Prep-TLC (DCM/MeOH=30/1) to give 23 mg of white solid in 45% yield.

### Step 2: Synthesis of N-(3-(1-amino-2-hydroxyethyl)-4-fluorophenyl)-4-cyclopropyl-2-(4-fluoro-2-methylphenoxy)-5-( trifluoromethyl) benzamide

Dissolve 4-cyclopropyl-2-(4-fluoro-2-methylphenoxy)-N-(4-fluoro-3-(2-hydroxyacetyl) phenyl)-5-(trifluoromethyl) benzamide (25 mg, 0.05 mmol) and hydroxylamine hydrochloride (14 mg, 0.2 mmol) in 2 mL methanol, stir at 60°C for 20 min, then add sodium acetate (16 mg, 0.2 mmol). Cool the reaction mixture to room temperature, add 2 mL of formic acid and 100 mg of zinc powder, and stir for 2 h at room temperature. Pour the reaction mixture into 10 mL water, and extract with ethyl acetate. Dry the organic phase, remove the solvent by rotary evaporation, and purify by Prep-TLC (DCM/MeOH=10/1) to give 7 mg of white solid, with the yield of 28%.

LC/MS: m/z=507.2 [M+H]⁺.

1H NMR (400 MHz, d₆-DMSO) δ 0.53-0.56 (2H, m), 1.01-1.05 (2H, m), 2.12-2.14 (1H, m), 2.15 (3H, s), 3.44-3.47 (2H, m), 4.19-4.21 (1H, m), 6.37 (1H, s), 7.03-7.12 (3H, m), 7.21 (1H, d, *J*= 8.4 Hz), 7.50-7.52 (1H, m), 7.74-7.76 (1H, m), 7.90 (1H, s), 10.40 (1H, s).

### Example 12

### N-(3-(Aminomethyl)-4-fluorophenyl)-2-(4-fluoro-2-methylphenoxy)-5-(trifluoromethyl) benzamide

### Example 13

N-(3-( ( ( (2-amino-2-oxoethyl)amino)methyl)-4-fluorophenyl)-2-(4-fluoro-2-methylphenoxy)-5-(t rifluoromethyl) benzamide

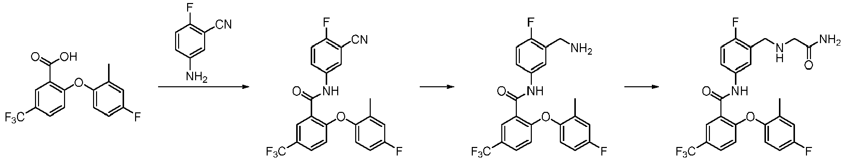

### Step 1: Synthesis of N-(3-cyano group-4-fluorophenyl)-2-(4-fluoro-2-methylphenoxy)-5-(trifluoromethyl) benzamide

Add 2-(4-fluoro-2-methylphenoxy)-5-(trifluoromethyl) benzoic acid (94 mg, 0.3 mmol), 5-amino-2-fluorobenzonitrile (41 mg, 0.3 mmol), DIPEA (77 mg, 0.6 mmol) and 2 mL tetrahydrofuran to a reaction flask, then add HATU (190 mg, 0.5 mmol). Stir the reaction mixture at room temperature overnight, pour into 10 mL of water, and extract with ethyl acetate. Dry the organic phase, remove the solvent by rotary evaporation, and purify by Prep-TLC (PE/EA=3/1) to give 110 mg of white solid, with the yield of 85%.

### Step 2: Synthesis of N-(3-(aminomethyl)-4-fluorophenyl)-2-(4-fluoro-2-methylphenoxy)-5-(trifluoromethyl) benzamide

Add N-(3-cyano group-4-fluorophenyl)-2-(4-fluoro-2-methylphenoxy)-5-(trifluoromethyl) benzamide (110 mg, 0.25 mmol) and 2 mL of methanol to a reaction flask, then add 10% Pd/C (30 mg). Stir the reaction mixture at 50 °C under hydrogen atmosphere (1 MPa) overnight. Filter, wash the filter cake with methanol, combine the filtrat, and remove the solvent by rotary evaporation to give 100 mg of white solid, with the yield of 91%.

LC/MS: m/z=437.1 [M+H]⁺.

1H NMR (400 MHz, d₆-DMSO) δ 2.15 (3H, s), 3.73 (2H, s), 6.81 (1H, d, J= 8.4 Hz), 7.09-7.26 (4H, m), 7.57-7.59 (1H, m), 7.76-7.79 (2H, m) , 7.97 (1H, d,*J* = 1.6 Hz), 10.55 (1H, s).

### Step 3: Synthesis of N-(3-( ( ( (2-amino-2-oxoethyl)amino)methyl)-4-fluorophenyl)-2-(4-fluoro-2-methylphenoxy)-5-(t rifluoromethyl) benzamide

Dissolve N-(3-(aminomethyl)-4-fluorophenyl)-2-(4-fluoro-2-methylphenoxy)-5-(trifluoromethyl) benzamide (44 mg, 0.1 mmol) and potassium carbonate (28 mg, 0.2 mmol) in 2 mL acetonitrile, add acetbromamide (14 mg, 0.1 mmol), stir at room temperature overnight. Filter and collect the filtrate, remove the solvent by rotary evaporation, and purify by Prep-TLC (PE/EA=1/1) to give 15 mg of white solid, with the yield of 31%.

LC/MS: m/z=494.1 [M+H]⁺.

1H NMR (400 MHz, d₆-DMSO) δ 2.15 (3H, s), 3.06 (2H, s), 3.70 (2H, s), 6.81 (1H, d, *J*= 8.8 Hz), 7.08-7.27 (6H, m), 7.61-7.63 (1H, m), 7.76 -7.78 (2H, m), 7.95 (1H, s), 10.55 (1H, s).

### Example 14

### N-(3-(1-aminoethyl)-4-fluorophenyl)-2-(4-fluoro-2-methylphenoxy)-5-(trifluoromethyl) benzamide

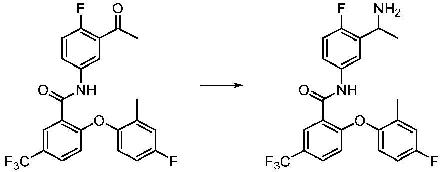

Dissolve N-(3-acetyl-4-fluorophenyl)-4-cyclopropyl-2-(4-fluoro-2-methylphenoxy)-5-(trifluoromethyl) benzamide (45 mg, 0.1 mmol, obtained by a method similar to the first step of example 9 reacting with the corresponding raw material) and 0.1 mL of ammonia in 2 mL tetrahydrofuran, stir for 6 h. Add sodium triacetoxy borohydride (42 mg, 0.2 mmol), stir the reaction mixture for 2 h, and remove the solvent by rotary evaporation directly. The crude product was purify by Prep-TLC (PE/EA=1/1) to give 6 mg of white solid, with the yield of 13%.

LC/MS: m/z=451.1 [M+H]⁺.

1H NMR (400 MHz, d₆-DMSO) δ 1.26 (3H, d, J= 6.4 Hz), 2.15 (3H, s), 4.28 (1H, q, *J*= 6.4 Hz), 6.82 (1H, d, *J*= 8.8 Hz), 7.07-7.26 (4H, m), 7.56-7.60 (1H, m), 7.77 (1H, dd, *J*= 8.4 Hz, 1.6 Hz), 7.88 (1H, dd, *J*= 8.0 Hz, 2.4 Hz), 7.96 (1H, d, *J=* 2.4 Hz), 10.52 (1H, s).

### Example 15

### 2-(4-fluoro-2-methylphenoxy)-N-(4-fluoro-3-(pyrrolidin-2-yl) phenyl)-5-(trifluoromethyl) benzamide

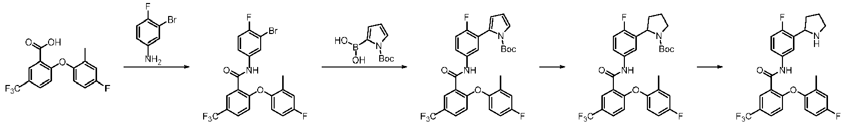

### Step 1: Synthesis of N-(3-bromo-4-fluorophenyl)-2-(4-fluoro-2-methylphenoxy)-5-(trifluoromethyl) benzamide

Add 2-(4-fluoro-2-methylphenoxy)-5-(trifluoromethyl) benzoic acid (94 mg, 0.3 mmol), 3-bromo-4-fluoroaniline (57 mg, 0.3 mmol), DIPEA (77 mg, 0.6 mmol), and 2 mL tetrahydrofuran to a reaction flask, then add HATU (190 mg, 0.5 mmol). Stir the reaction mixture at room temperature overnight, poured into 10 mL of water, and extract with ethyl acetate. Dry the organic phase, remove the solvent by rotary evaporation, and purify by Prep-TLC (PE/EA=10/1) to give 120 mg of white solid, with the yield of 82%.

### Step 2: Synthesis of 2-(2-fluoro-5-(2-(4-fluoro-2-methylphenoxy)-5-(trifluoromethyl) benzamido) phenyl) tert-butyl-1-H-pyrrole-1-carboxylate

Add N-(3-bromo-4-fluorophenyl)-2-(4-fluoro-2-methylphenoxy)-5-(trifluoromethyl) benzamide (97 mg, 0.2 mmol), (1-(tert-butoxycarbonyl)-1H-pyrrol-2-yl) boronic acid (42 mg, 0.2 mmol), potassium carbonate (55 mg, 0.4 mmol), and 2 mL DMF to a reaction flask. Charge with nitrogen, add Pd(dppf)Cl₂(7 mg, 0.01 mmol). Heat the reaction mixture to 90°C and stir for 6 h under nitrogen protection. Cool to room temperature, pour into 10 mL of water, then extract with ethyl acetate. Dry the organic phase, remove the solvent by rotary evaporation and purify by Prep-TLC (PE/EA=4/1) to give 90 mg white solid, with the yield of 79%.

### Step 3: Synthesis of 2-(2-fluoro-5-(2-(4-fluoro-2-methylphenoxy)-5-(trifluoromethyl) benzamido) phenyl) pyrrolidine-1-carboxylate

Add 2-(2-fluoro-5-(2-(4-fluoro-2-methylphenoxy)-5-(trifluoromethyl) benzamido) phenyl) tert-butyl-1-H-pyrrolidine-1-carboxylate (90 mg, 0.16 mmol) and 5 mL of methanol to a reaction flask, then add 10% Pd/C (20 mg). Stir the reaction mixture at room temperature under hydrogen atmosphere (1 MPa) overnight. Filter, wash the filter cake with methanol, and combine the filtrate, remove the solvent by rotary evaporation to give 85 mg white solid, with the yield of 94%.

### Step 4: Synthesis of 2-(4-fluoro-2-methylphenoxy)-N-(4-fluoro-3-(pyrrolidin-2-yl) phenyl)-5-(trifluoromethyl) benzamide

Dissolve 2-(2-fluoro-5-(2-(4-fluoro-2-methylphenoxy)-5-(trifluoromethyl) benzamido) phenyl) pyrrolidine-1-carboxylate (58 mg, 0.1 mmol) in 2 mL hydrogen chloride/dioxane (2 M), stir at room temperature for 2 h. Remove the solvent by rotary evaporation, add 10 mL of water, adjust pH to basic by sodium carbonate, and extract with ethyl acetate. Dry the organic phase, remove the solvent by rotary evaporation, then purify by Prep-HPLC to give 25 mg of white solid, with the yield of 52%.

LC/MS: m/z=477.2 [M+H]⁺.

1H NMR (400 MHz, d₆-DMSO) δ 1.44-1.53 (1H, m), 1.74-1.78 (2H, m), 2.16 (3H, s), 2.17-2.20 (1H, m), 2.97-3.00 (2H, m), 4.33 (1H, t, *J*= 7.6 Hz), 6.81 (1H, d, *J*= 8.4 Hz), 7.09-7.26 (4H, m), 7.58-7.61 (1H, m), 7.77 (1H, dd, *J*= 8.8 Hz, 2.4 Hz), 7.77 (1H, dd, *J*= 8.4 Hz, 4.0 Hz), 7.96 (1H, d, *J*= 2.8 Hz), 10.51 (1H, s).

### Example 16

### 5-(2-fluoro-5-(2-(4-fluoro-2-methylphenoxy)-5-(trifluoromethyl) benzamido) phenyl) pyrrolidine-2-carboxacylamino

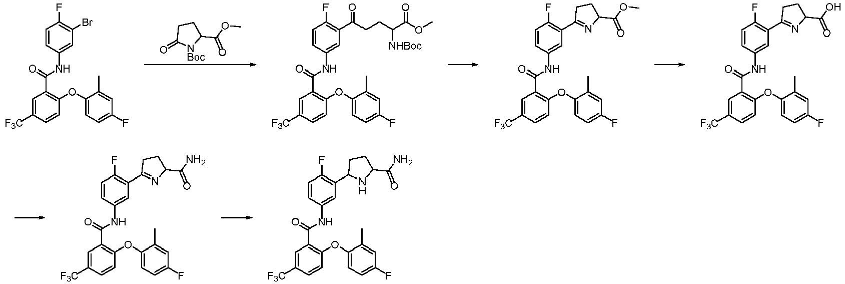

### Step 1: Synthesis of 2-((tert-butoxycarbonyl) amino)-5-(2-fluoro-5-(2-(4-fluoro-2-methylphenoxy)-5-(trifluoromethyl) benzamido) phenyl)-5-oxovaleric acid methyl ester

Dissolve N-(3-bromo-4-fluorophenyl)-2-(4-fluoro-2-methylphenoxy)-5-(trifluoromethyl) benzamide (486 mg, 1.0 mmol) in 5 mL anhydrous tetrahydrofuran, drop n-butyllithium (1.6 M, 3.1 mL) at -70°C, then stir for 1 h at that temperature. Drop 2 mL of tetrahydrofuran solution containing N-tert-butoxycarbonyl-DL-pyroglutamate methyl ester (1215 mg, 5.0 mmol), stir for 1 h, pour into 20 mL of water, and extract with ethyl acetate. Dry the organic phase, remove the solvent by rotary evaporation, and purify by silica gel column chromatography (PE/EA=4/1) to give 210 mg of white solid, with the yield of 32%.

### Step 2: Synthesis of 5-(2-fluoro-5-(2-(4-fluoro-2-methylphenoxy)-5-(trifluoromethyl) benzamido) phenyl)-3,4-dihydro-2H-pyrrole-2-carboxylic acid methyl ester

Dissolve 2- ((tert-butoxy-carbonyl) amino) -5- (2-fluoro-5 - (2- (4-fluoro-2-methylphenoxy) -5- (trifluoromethyl) benzenamide) phenyl) -5-oxy-valerate methyl ester (210 mg, 0.32 mmol) in 5 mL of dichloromethane, then add 2 mL of trifluoroacetic acid and stir at room temperature overnight. Remove the solvent by rotary evaporation directly to give ~200 mg crude product.

### Step 3: Synthesis of 5-(2-fluoro-5-(2-(4-fluoro-2-methylphenoxy)-5-(trifluoromethyl) benzamido) phenyl)-3,4-dihydro-2H-pyrrole-2-carboxylic acid

Dissolve the crude product obtained in the previous step in 5 mL of tetrahydrofuran, add 2 mL of aqueous sodium hydroxide solution with a concentration of 1 M, stir for 6 h at room temperature. Adjust pH to 5 with dilute hydrochloric acid, and purify the reaction mixture directly by Prep-HPLC after to obtain 90 mg of the target product, with the two-step yield of 54%.

### Step 4: Synthesis of 5-(2-fluoro-5-(2-(4-fluoro-2-methylphenoxy)-5-(trifluoromethyl) benzamido) phenyl)-3,4-dihydro-2H-pyrrole-2-carboxamide

Add 5-(2-fluoro-5-(2-(4-fluoro-2-methylphenoxy)-5-(trifluoromethyl) benzamido) phenyl)-3,4-dihydro-2H-pyrrole-2-carboxylic acid (52 mg, 0.1 mmol), ammonium chloride (53 mg, 1.0 mmol), DIPEA (39 mg, 0.3 mmol) and 2 mL of tetrahydrofuran to a reaction flask, then add HBTU (76 mg, 0.2 mmol). Stir the reaction mixture at room temperature overnight, pour into 10 mL of water, and extract with ethyl acetate. Dry the organic phase, remove the solvent by rotary evaporation, and purify by Prep-TLC (DCM/MeOH=20/1) to give 30 mg of white solid, with the yield of 58%.

### Step 5: Synthesis of 2-(4-fluoro-2-methylphenoxy)-N-(4-fluoro-3-(pyrrolidin-2-yl) phenyl)-5-(trifluoromethyl) benzamide

Dissolve 5-(2-fluoro-5-(2-(4-fluoro-2-methylphenoxy)-5-(trifluoromethyl) benzamido) phenyl)-3,4-dihydro-2H-pyrrole-2-carboxamide (30 mg, 0.058 mmol) in 2 mL methanol, add 20 mg of sodium borohydride, then stir at room temperature. Pour. the reaction mixture into 10 mL of water, and extract with ethyl acetate. Dry the organic phase, remove the solvent by rotary evaporation, and purify by Prep-TLC (DCM/MeOH=10/1) to give 6 mg of white solid, with the yeild of 20%.

LC/MS: m/z=520.2 [M+H]⁺.

1H NMR (400 MHz, d₆-DMSO) δ 1.55-1.74 (2H, m), 1.87-2.00 (1H, m), 2.05 (3H, s), 2.16-2.22 (1H, m), 3.45-3.46 (0.6H, m), 3.65-3.66 (0.4H, m), 4.10-4.42 (1H, m), 6.57-6.64 (1H, m), 7.01-7.11 (2H, m), 7.18-7.22 (2H, m), 7.25-7.45 (1H, m), 7.50-7.69 (1H, m), 7.71-7.77 (3H, m), 7.98 (1H, s), 10.63- 10.74 (1H, m).

### Example 17

### N-(3-(2,3-Diamino-3-oxopropyl)-4-fluorophenyl)-2-(4-fluoro-2-methylphenoxy)-5-(trifluoro methyl) benzamide

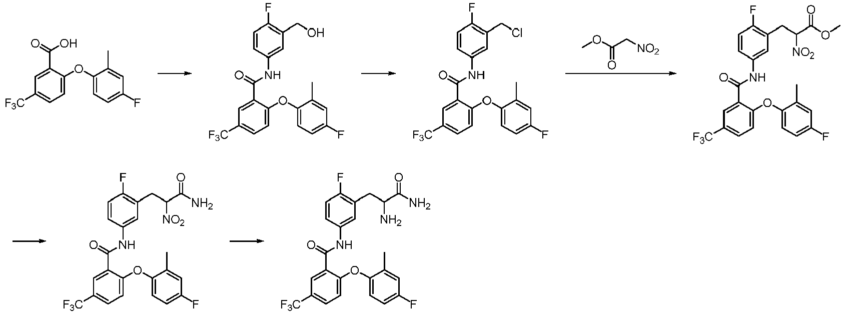

### Step 1: Synthesis of 2-(4-fluoro-2-methylphenoxy)-N-(4-fluoro-3-(hydroxymethyl) phenyl)-5-(trifluoromethyl) benzamide

Add 2-(4-fluoro-2-methylphenoxy)-5-(trifluoromethyl) benzoic acid (94 mg, 0.3 mmol), (5-amino-2-fluorophenyl) methanol (42 mg, 0.3 mmol), DIPEA (77 mg, 0.6 mmol) and 2 mL of tetrahydrofuran to a reaction flask, then add HATU (190 mg, 0.5 mmol). Stir the reaction mixture at room temperature overnight, then pour into 10 mL of water, and extract with ethyl acetate. Dry the organic phase, remove the solvent by rotary evaporation, and purify by Prep-TLC (PE/EA=10/1) to give 95 mg of white solid, with the yeild of 73%.

### Step 2: Synthesis of N-(3-(chloromethyl)-4-fluorophenyl)-2-(4-fluoro-2-methylphenoxy)-5-(trifluoromethyl) benzamide

Dissolve 2-(4-fluoro-2-methylphenoxy)-N-(4-fluoro-3-(hydroxymethyl) phenyl)-5-(trifluoromethyl) benzamide (95 mg, 0.22 mmol) in phosphorus oxychloride, stir at 60 °C for 1 h. Remove the solvent by rotary evaporation directly and the residual trichloroxaphos was processed with trichloromethane twice to give 100 mg of yellow solid, with the yeild of 100%.

### Step 3: Synthesis of methyl 3-(2-fluoro-5-(2-(4-fluoro-2-methylphenoxy)-5-(trifluoromethyl) benzamido) phenyl)-2-nitropropanoate

Dissolve N-(3-(chloromethyl)-4-fluorophenyl)-2-(4-fluoro-2-methylphenoxy)-5-(trifluoromethyl) benzamide (100 mg, 0.22 mmol), methyl nitroacetate (52 mg, 0.44 mmol), benzyltriethylammonium chloride (50 mg, 0.22 mmol) and potassium bicarbonate (44 mg, 0.44 mmol) in 3 mL DMF, stir at 60°C overnight. Pour the reaction mixture into 10 mL water, and extract with ethyl acetate. Dry the organic phase, remove the solvent by rotary evaporation, and purify by Prep-TLC (PE/EA=3/1) to give 25 mg white solid, with the yeild of 21%.

### Step 4: Synthesis of N-(3-(3-amino-2-nitro-3-oxopropyl)-4-fluorophenyl)-2-(4-fluoro-2-methylphenoxy)-5-(trifluorom ethyl) benzamide

Dissolve Methyl 3-(2-fluoro-5-(2-(4-fluoro-2-methylphenoxy)-5-(trifluoromethyl) benzamido) phenyl)-2-nitropropanoate (25 mg, 0.046 mmol) in 4 mL of ammonia-methanol solution (1 M), stir at room temperature overnight. Remove the solvent by rotary evaporation to give 25 mg of white solid, with the yeild of 100%.

### Step 5: Synthesis of N-(3-(2,3-diamino-3-oxopropyl)-4-fluorophenyl)-2-(4-fluoro-2-methylphenoxy)-5-(trifluorometh yl)benzamide

Add N-(3-(3-amino-2-nitro-3-oxopropyl)-4-fluorophenyl)-2-(4-fluoro-2-methylphenoxy)-5-(trifluorom ethyl) benzamide (25 mg, 0.046 mmol) and 5 mL methanol to a reaction flask, then add 10% Pd/C (10 mg). Stir the reaction mixture at room temperature under hydrogen atmosphere (1 MPa) overnight. Filter, wash the filter cake with methanol, combine the filtrate and remove the solvent by rotary evaporation, purify by Prep-HPLC to give 9 mg as a white solid, with the yeild of 38%.

LC/MS: m/z=494.1 [M+H]⁺.

### Example 18

### 2-(4-Fluoro-2-methylphenoxy)-N-(4-fluoro-3-(3-oxopiperazin-2-yl) phenyl)-5-(trifluoromethyl) benzamide

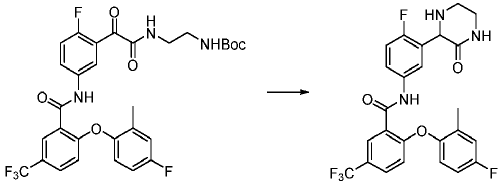

Dissolve (2-(2-(2-fluoro-5-(2-(4-fluoro-2-methylphenoxy)-5-(trifluoromethyl) benzamido) phenyl)-2-oxoacetamido) ethyl) carbamic acid tert-butyl ester (45 mg, 0.072 mmol, prepared by a method similar to example 9 with the corresponding materials) in 3 mL HCl/EA (4 M), stir at room temperature for 2 h, then remove the solvent by rotary evaporation, add 5 mL of methanol and 50 mg of sodium cyano borohydride. Stir at room temperature overnight and pour into 20 mL of water, extract with ethyl acetate. Dry the organic phase, remove the solvent by rotary evaporation and purify by Prep-HPLC to give 8 mg as a white solid, with the yield of 22%.

LC/MS: m/z=506.1 [M+H]⁺.

1H NMR (400 MHz, d₆-DMSO) δ 2.16 (3H, s), 2.78-2.82 (1H, m), 2.88-3.01 (2H, m), 3.15-3.19 (1H, m), 4.52 (1H, s), 6.78 (1H, d, *J=* 8.0 Hz), 7.10-7.26 (4H, m), 7.63-7.66 (1H, m), 7.74-7.78 (2H, m), 7.81 (1H, s), 7.93 (1H, s), 10.52 (1H, s).

### Example 19

### N-(3-(amino (2-oxopyrrolidin-3-yl) methyl)-4-fluorophenyl)-4-cyclopropyl-2-(4-fluoro-2-methylphenoxy)-5-(trifluoromethyl) benzamide

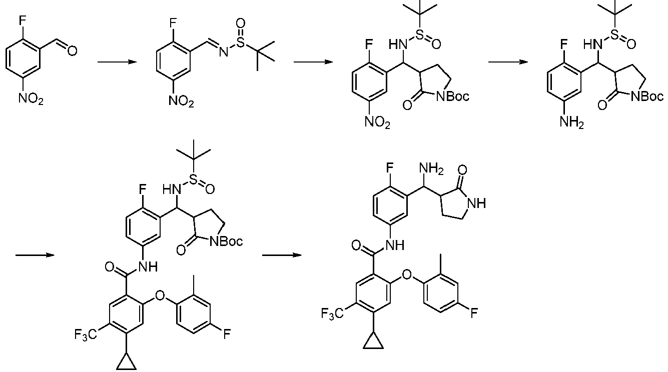

### Step 1: Synthesis of N-(2-fluoro-5-nitrobenzylidene)-2-methylpropane-2-sulfinamide

Dissolve 2-fluoro-5-nitrobenzaldehyde (333 mg, 2.0 mmol), tert-butyl tert-butylsulfonamide (268 mg, 2.2 mmol) and cesium carbonate (987 mg, 3.0 mmol) in 10 mL dichloromethane, stir at room temperature overnight, pour into 10 mL of water, then extract with ethyl acetate. Dry the organic phase and remove the solvent by rotary evaporation to give 573 mg of yellow solid.

### Step 2: Synthesis of tert-butyl 3-( ( ( ( tert-butylsulfinyl)amino)(2-fluoro-5-nitrophenyl)methyl)-2-oxopyrrolidine-1-carboxylate

At -70 °C and under nitrogen protection, drop N-butyllithium (2.5 M, 0.9 mL) into 5 mL of anhydrous tetrahydrofuran solution of N-BOC-2-pyrrolidone (545 mg, 2.94 mmol), then stir for 0.5 h. Drop N-(2-fluoro-5-nitrobenzylidene)-2-methylpropane-2-sulfinacylamino (200 mg, 0.73 mmol) in 1 mL of tetrahydrofuran to the reaction mixture, and the temperature is controlled below -60 °C. After stir of 1.5 h, pour the reaction mixture into 20 mL of aqueous ammonium chloride solution, extracte with ethyl acetate. Dry the organic phase, remove the solvent by rotary evaporation, and purify by silica gel column (DCM/MeOH=20/1) to obtain 280 mg of yellow oily substance, with the yield of 83%.

### Step 3: Synthesis of tert-butyl 3-((5-amino-2-fluorophenyl) ((tert-butylthio) amino) methyl)-2-oxopyrrolidine-1 -carboxylate

Add 3-((((tert-butylsulfonyl) amino) (2-fluoro-5-nitrophenyl) methyl) -2-oxy-pyrrolidine 1-tert-butyl carboxylate (110 mg, 0.24 mmol) and 5 mL of ethanol to a reaction flask, then add 10% Pd/C (20 mg). Stir the reaction mixture at room temperature under hydrogen atmosphere overnight. Filter, collect the filtrate and wash with ethanol, remove the solvent by rotary evaporation to give 100 mg of yellow solid, with the yield of 97%.

### Step 4. Synthesis of tert-butyl 3-(((tert-butylsulfinyl) amino) (5-(4-cyclopropyl-2-(4-fluoro-2-methylphenoxy)-5-(trifluoromethyl) benzamido)-2-fluorophenyl) methyl)-2-oxopyrrolidine-1 -carboxylate

Add 4-cyclopropyl-2-(4-fluoro-2-methylphenoxy)-5-(trifluoromethyl) benzoic acid (82 mg, 0.23 mmol), 3-((5-amino-2-fluorophenyl) ((tert-butylthio) amino) methyl)-2-oxopyrrolidine-1-carboxylic acid tert-butyl ester (98 mg, 0.23 mmol), DIPEA (106 mg, 0.82 mmol) and 5 mL of tetrahydrofuran to a reaction flask, then add HATU (163 mg, 0.42 mmol). Stir the reaction mixture at room temperature overnight, pour into 20 mL of water, and extract with ethyl acetate. Dry the organic phase, remove the solvent by rotary evaporation, and purify by Prep-TLC (DCM/MeOH=15/1) to give 25 mg of white solid, with the yeild of 14%.

### Step 5: Synthesis of N-(3-(amino (2-oxopyrrolidin-3-yl) methyl)-4-fluorophenyl)-4-cyclopropyl-2-(4-fluoro-2-methylphenoxy)-5-(trifluoromethyl) benzamide

Dissolve 3-(((tert-butylsulfinyl) amino) (5-(4-cyclopropyl-2-(4-fluoro-2-methylphenoxy)-5-(trifluoromethyl) benzamido)-2-fluorophenyl) methyl)-2-oxopyrrolidine-1-carboxylic acid tert-butyl ester (25 mg, 0.033 mmol) in 2 mL of hydrogen chloride/ethyl acetate solution (2 M), stir at room temperature overnight, remove the solvent by rotary evaporation. Add 10 mL of water, adjust pH to alkaline by sodium carbonate, and extract with ethyl acetate. Remove the solvent by rotary evaporation and purify by Prep-HPLC to give 12 mg as a white solid, with the yield of 67%.

LC/MS: m/z=560.2 [M+H]⁺.

1H NMR (400 MHz, d₆-DMSO) δ 0.54-0.60 (2H, m), 1.00-1.06 (2H, m), 1.61-1.71 (1H, m), 2.13-2.12 (2H, m), 2.15 (3H, s), 2.52-2.58 (1H, m), 3.07- 3.13 (2H, m), 4.60 (1H, d,*J*= 3.2 Hz), 6.37 (1H, s), 7.05-7.09 (3H, m), 7.20 (1H, dd,*J*= 9.2 Hz, 2.7 Hz), 7.51-7.57 (1H, m), 7.69 (1H, s), 7.79 (1H, dd, *J*= 6.6 Hz, 2.4 Hz), 7.90 (1H, s), 10.40 (1H, s).

### Example 20

### N-(3-(1-Amino-3-hydroxybutyl)-4-fluorophenyl)-4-cyclopropyl-2-(4-fluoro-2-methylphenox y)-5-(trifluoromethyl) benzamide

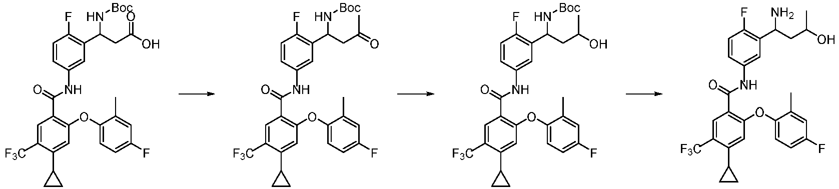

### Step 1: Synthesis of tert-butyl (1-(5-(4-cyclopropyl-2-(4-fluoro-2-methylphenoxy)-5-(trifluoromethyl) benzamido)-2-fluorophenyl)-3-oxobutyl) carbamate

Dissolve 3-((tert-butoxycarbonyl) amino)-3-(5-(4-cyclopropyl-2-(4-fluoro-2-methylphenoxy)-5-(trifluoromethyl) benzamido)-2-fluorophenyl) propanoic acid (63 mg, 0.10 mmol) in 2 mL anhydrous tetrahydrofuran, add methyl lithium (1 M, 0.4 mL) at 0 °C. Stir for 1 h at room temperature and then pour into 10 mL of water. Extract wih ethyl acetate, dry, remove the solvent by rotary evaporation, and purify by Prep-TLC (PE/EA=2/1) to give 30 mg of white solid, with the yeild of 47%.

### Step 2. Synthesis of tert-butyl (1-(5-(4-cyclopropyl-2-(4-fluoro-2-methylphenoxy)-5-(trifluoromethyl) benzamido)-2-fluorophenyl)-3-hydroxybutyl) carbamate

Dissolve tert-butyl (1-(5-(4-cyclopropyl-2-(4-fluoro-2-methylphenoxy)-5-(trifluoromethyl) benzamido)-2-fluorophenyl)-3-oxobutyl) carbamate (30 mg, 0.047 mmol) in 2 mL methanol, add 5 mg of sodium borohydride at room temperature, stir for 1 h, and pour into 10 mL of water. Extract with ethyl acetate. dry the organic phase, remove the solvent by rotary evaporation, and purify by Prep-TLC (PE/EA=1/1) to give 18 mg as a white solid, with the yield of 60%.

### Step 3: Synthesis of N-(3-(1-amino-3-hydroxybutyl)-4-fluorophenyl)-4-cyclopropyl-2-(4-fluoro-2-methylphenoxy)-5-( trifluoromethyl) benzamide

Dissolve tert-butyl (1-(5-(4-cyclopropyl-2-(4-fluoro-2-methylphenoxy)-5-(trifluoromethyl) benzamido)-2-fluorophenyl)-3-hydroxybutyl) carbamate (18 mg, 0.028 mmol) in 2 mL hydrogen chloride-ethyl acetate solution (2 M), stirred at room temperature overnight. Remove the solvent by rotary evaporation and add 10 mL water, adjust the pH to alkaline by sodium carbonate, then extract with ethyl acetate. Dry the organic phase, remove the solvent by rotary evaporation, and purify by Prep-HPLC to give 10 mg as a white solid, with the yield of 67%.

LC/MS: m/z=535.2 [M+H]⁺.

1H NMR (400 MHz, d₆-DMSO) δ 0.56-0.58 (2H, m), 1.00-1.07 (5H, m), 1.55-1.57 (1H, m), 2.12-2.14 (1H, m), 2.16 (3H, s), 3.68-3.76 (2H, m), 4.19- 4.28 (1H, m), 6.35 (1H, s), 7.06-7.10 (3H, m), 7.22 (1H, dd, *J*= 9.2 Hz, 2.0 Hz), 7.49-7.55 (1H, m), 7.73-7.80 (1H, m), 7.91 (1H, s), 10.39 (1H, s).

### Example 21

### N-(3-(1-amino-3-fluoropropyl)-4-fluorophenyl)-4-cyclopropyl-2-(4-fluoro-2-methylphenoxy )-5-(trifluoromethyl) benzamide

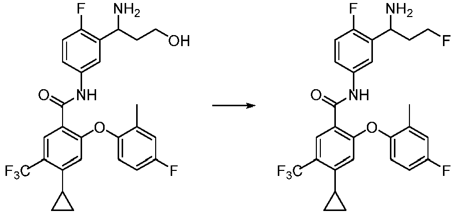

Dissolve N-(3-(1-amino-3-hydroxypropyl)-4-fluorophenyl)-4-cyclopropyl-2-(4-fluoro-2-methylphenoxy)-5 -(trifluoromethyl) benzamide (52 mg, 0.10 mmol) in 2 mL dichloromethane, add DAST (24 mg, 0.15 mmol), stir at room temperature overnight. Pour the reaction mixture into 10 mL of water, and extracte with ethyl acetate. Dry the organic phase, remove the solvent by rotary evaporation, and purify by Prep-TLC (DCM/MeOH=10/1) to give 20 mg as a white solid, with the yeild of 38%.

LC/MS: m/z=521.2 [M+H]⁺.

1H NMR (400 MHz, d₆-DMSO) δ 0.53-0.60 (2H, m), 1.00-1.06 (2H, m), 1.94-2.16 (7H, m), 4.31-4.33 (1H, m), 4.40-4.68 (1H, m), 6.34 (1H, s), 7.03- 7.10 (2H, m), 7.14-7.23 (2H, m), 7.49-7.56 (1H, m), 7.84-7.92 (2H, m), 10.45 (1H, s).

### Example 22

### N-(3-(1-Amino-2-fluoro-3-hydroxypropyl)-4-fluorophenyl)-4-cyclopropyl-2-(4-fluoro-2-met hylphenoxy)-5-(trifluoromethyl) benzamide

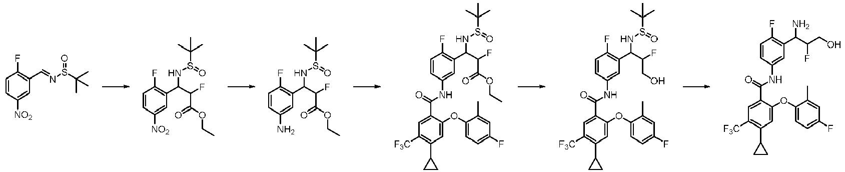

### Step 1: Synthesis of 3-((tert-butylsulfinyl) amino)-2-fluoro-3-(2-fluoro-5-nitrophenyl) propanoic acid ethyl ester

At -70 °C and under the protection of nitrogen, drop LiHMDS (1.3 M, 3.2 mL) into 5 mL anhydrous tetrahydrofuran solution of ethyl fluoroacetate (480 mg, 4.53 mmol), then stir for 0.5 h. Drop N-(2-fluoro-5-nitrobenzylidene)-2-methylpropane-2-sulfinacylamino (230 mg, 0.85 mmol) in 1 mL tetrahydrofuran to the reaction solution. The temperature of the system is controlled below -60 °C, stir for 1.5 h. Pour the reaction mixture into 20 mL of aqueous ammonium chloride solution, and extract with ethyl acetate. Dry the organic phase, remove the solvent by rotary evaporation, and purify by silica gel column (PE/EA=2/1) to give 216 mg of yellow solid, with the yield of 68%.

### Step 2: Synthesis of 3-(5-Amino-2-fluorophenyl)-3-((tert-butylsulfinyl) amino)-2-fluoropropionic acid ethyl ester

Add 3-((tert-Butylsulfinyl) amino)-2-fluoro-3-(2-fluoro-5-nitrophenyl) propanoic acid ethyl ester (216 mg, 0.57 mmol) and 5 mL of ethanol to a reaction flask, then add 10% Pd/C (30 mg). Stir the reaction mixture at room temperature under hydrogen atmosphere overnight. Filter, then wash the filter cake with ethanol, combine the filtrate and remove the solvent by rotary evaporation to obtain 200 mg of a yellow solid, with the yield of 100%.

### Step 3: Synthesis of 3-((tert-butylsulfinyl) amino)-3-(5-(4-cyclopropyl-2-(4-fluoro-2-methylphenoxy)-5-(trifluoromethyl) Benzamido)-2-fluorophenyl)-2-fluoropropionic acid ethyl ester

Add 4-cyclopropyl-2-(4-fluoro-2-methylphenoxy)-5-(trifluoromethyl) benzoic acid (82 mg, 0.23 mmol), 3-(5-Amino-2-fluorophenyl)-3-((tert-butylsulfinyl) amino)-2-fluoropropionic acid ethyl ester (80 mg, 0.23 mmol), DIPEA (106 mg, 0.82 mmol) and 5 mL of tetrahydrofuran to a reaction flask, then add HATU (163 mg, 0.42 mmol). Stir the reaction mixture at room temperature overnight. Pour the reaction mixture into 20 mL of water, and extract with ethyl acetate. Dry the organic phase, remove the solvent by rotary evaporation, and purify by Prep-TLC (DCM/MeOH=20/1) to give 35 mg of white solid, with the yeild of 29%.

### Step 4. Synthesis of N-(3-(1-((tert-butylsulfinyl) amino)-2-fluoro-3-hydroxypropyl)-4-fluorophenyl)-4-cyclopropyl-2-(4-fluoro-2-methylphenoxy)-5-(trifluoromethyl) benzamide

At room temperature, dissolve 3-((tert-butylsulfonyl) amino)-3-(5-(4-cyclopropyl-2-(4-fluoro-2-methylphenoxy)-5-(trifluoromethyl) benzamide)-2-fluorophenyl)-2-fluoropropionate ethyl ester (35 mg, 0.051 mmol) and 5 mg of lithium chloride in 2mL methanol, add 10 mg of sodium borohydride, stir at room temperature for 4 hours. Pour the reaction mixture into 10 mL of water, extract with ethyl acetate. Dry the organic phase and remove the solvent by rotary evaporation, and purify by Prep-TLC (DCM/MeOH=20/1) to obtain 15 mg of white solid, with the yield of 43%.

### Step 5: Synthesis of N-(3-(1-amino-2-fluoro-3-hydroxypropyl)-4-fluorophenyl)-4-cyclopropyl-2-(4-fluoro-2-methylph enoxy)-5-(trifluoromethyl) benzamide

Stir 2 mL HCl-ethyl acetate solution (2 M) of N-(3-(1-((tert-butylsulfinyl) amino)-2-fluoro-3-hydroxypropyl)-4-fluorophenyl)-4-cyclopropyl-2-(4-fluoro-2-methylphenoxy)-5-(trifluoromethyl) benzamide (15 mg, 0.023 mmol) at room temperature overnight. Remove the solvent by rotary evaporation, then transfer to 10 mL of water, adjust pH to alkalic by sodium carbonate, and extract with ethyl acetate. Dry the organic phase, remove the solvent by rotary evaporation, purify by Prep-HPLC to give 4 mg as a white solid, with the yield of 32%.

LC/MS: m/z=539.2 [M+H]⁺.

1H NMR (400 MHz, d₆-DMSO) δ 0.56-0.58 (2H, m), 0.99-1.07 (2H, m), 2.11-2.14 (1H, m), 2.16 (3H, s), 3.50-3.72 (2H, m), 4.26-4.30 (1H, m), 4.37- 4.45 (0.5H, m), 4.50-4.57 (0.5H, m), 4.95 (1H, brs), 6.35 (1H, s), 7.07-7.16 (3H, m), 7.22 (1H, d,*J*= 8.1 Hz), 7.53-7.61 (1H, m), 7.77-7.83 (1H, m), 7.92 (1H, s), 10.40-10.42 (1H, m).

### Example 23

### N-(3-(1-Amino-2,2-difluoro-3-hydroxypropyl)-4-fluorophenyl)-4-cyclopropyl-2-(4-fluoro-2-methylphenoxy)-5-(trifluoromethyl) benzamide

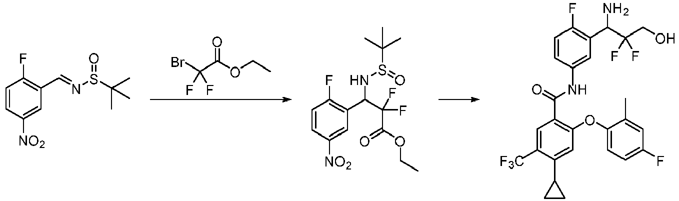

### Step 1. Synthesis of 3-((tert-butylsulfinyl) amino)-2,2-difluoro-3-(2-fluoro-5-nitrophenyl) propanoic acid ethyl ester

Add zinc powder (195 mg, 3.0 mmol) and ethyl 2-bromo-2,2-difluoroacetate (404 mg, 2.0 mmol) in anhydrous tetrahydrofuran, stir and reflux for 30 min. Add anhydrous THF solution of N-(2-fluoro-5-nitrobenzylidene)-2-methylpropane-2-sulfinacylamino (272 mg, 1.0 mmol) slowly to the previous mixture at this temperature. Stir and reflux for 1.5 h, filter, and the filtrate is concentrated. The crude product is purify by Prep-TLC (DCM/MeOH=50/1) to give 190 mg of white solid, with the yeild of 48%.

### Step 2: Synthesis of N-(3-(1-amino-2, 2-difluoro-3-hydroxypropyl)-4-fluorophenyl)-4-cyclopropyl-2-(4-fluoro-2-methylphenoxy)-5-(trif luoromethyl) benzamide

Product of Example 23 is obtained by the same method as in Example 22 and with the corresponding reagents

LC/MS: m/z=557.2 [M+H]⁺.

### Example 24

### 4-Cyclopropyl-N-(3-(1,3-diaminopropyl)-4-fluorophenyl)-2-(4-fluoro-2-methylphenoxy)-5-( trifluoromethyl) benzamide

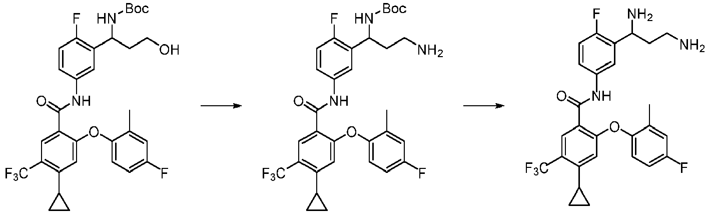

### Step 1: Synthesis of tert-butyl (3-amino-1-(5-(4-cyclopropyl-2-(4-fluoro-2-methylphenoxy)-5-(trifluoromethyl) benzamido)-2-fluorophenyl) propyl) carbamate

Add 1-(5--(4-(4-Cyclopropyl-2-(4-fluoro-2-methylphenoxy)-5-(trifluoromethyl) benzamido)-2-fluorophenyl)-3-hydroxypropyl) carbamic acid tert-butyl ester (100 mg, 0.16 mmol), DIPEA (41 mg, 0.32 mmol) and 5 mL of dichloromethane to a reaction flask, then drop methylflavonyl chloride (22 mg, 0.19 mmol) at room temperature. Stir the reaction mixture for 1 h at room temperature, remove the solvent by rotary evaporation, then dissolved in 5 mL of anhydrous tetrahydrofuran, and add ~10 mg of sodium acylamino. Keep the reaction at room temperature for 1 hour, then pour into 10 mL of ice water, and extract with ethyl acetate. Dry the organic phase, remove the solvent by rotary evaporation, and purify by Prep-HPLC to give 6 mg of yellow oil, with the yeild of 6%.

### Step 2: Synthesis of (4-cyclopropyl-N-(3-(1,3-diaminopropyl)-4-fluorophenyl)-2-(4-fluoro-2-methylphenoxy)-5-(triflu oromethyl) benzamide

Dissolve (tert-butyl (3-amino-1-(5-(4-cyclopropyl-2-(4-fluoro-2-methylphenoxy)-5-(trifluoromethyl) benzamido) -2-fluorophenyl) propyl) carbamate (6 mg, 0.01 mmol) in 2 mL hydrogen chloride-dioxane solution (2 M), stir at room temperature for 2 h, and remove the solvent by rotary evaporation, then add to 10 mL of water,. adjust pH to alkalic using sodium carbonate, extract with ethyl acetate and dry, remove the solvent by rotary evaporation, and purify by Prep-HPLC to give 2 mg of white solid, with the yield of 40%.

LC/MS: m/z=520.2 [M+H]⁺.

1H NMR (400 MHz, d₆-DMSO) δ 0.56-0.58 (2H, m), 0.99-1.07 (2H, m), 1.62-1.65 (2H, m), 2.11-2.16 (4H, m), 2.60-2.70 (2H, m), 4.12-4.14 (1H, m), 6.35 (1H, s), 7.02-7.16 (3H, m), 7.20-7.23 (1H, m), 7.48-7.55 (1H, m), 7.76-7.78 (1H, m), 7.91 (1H, s), 10.38 (1H, s).

### Example 25

### 2-(4-fluoro-2-methylphenoxy)-N-(4-fluoro-3-(hydroxyl (2-oxopyrrolidin-3-yl) methyl) phenyl)-5-(trifluoromethyl) benzamide

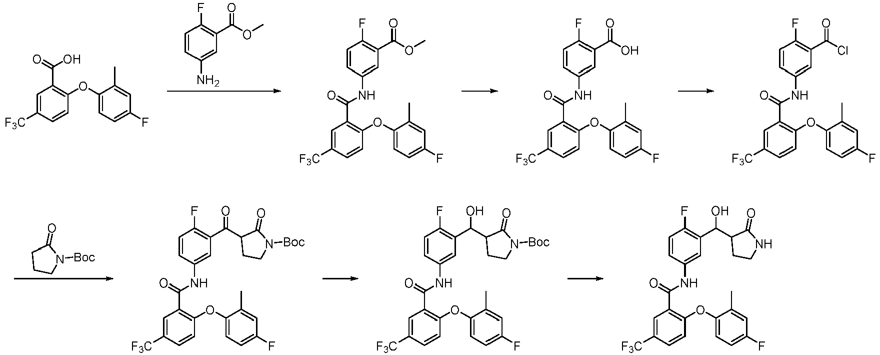

### Step 1: Synthesis of 2-fluoro-5-(2-(4-fluoro-2-methylphenoxy)-5-(trifluoromethyl) benzoyl-methyl benzoate

Add 2-(4-fluoro-2-methylphenoxy)-5-(trifluoromethyl) benzoic acid (314 mg, 1.0 mmol), 5-amino-2-fluorobenzoic acid methyl ester (169 mg, 1.0 mmol), DIPEA (258 mg, 2.0 mmol) and 5 mL of tetrahydrofuran to a reaction flask, then add HATU (456 mg, 1.2 mmol). Stir the reaction mixture at room temperature overnight. Pour the reaction mixture into 10 mL of water, and extract with ethyl acetate. Dry the organic phase, remove the solvent by rotary evaporation, and purify by silica gel column chromatography (PE/EA=3/1) to give 330 mg of pale yellow solid, with the yeild of 71%.

### Step 2: Synthesis of 2-fluoro-5-(2-(4-fluoro-2-methylphenoxy)-5-(trifluoromethyl) benzamido) benzoic acid

Add 2-fluoro-5 - (2- (4-fluoro-2-methylphenoxy) -5- (trifluoromethyl) benzoyl-methyl benzoate (330 mg, 0.71 mmol), sodium hydroxide (114 mg, 2.84 mmol), 5 mL of water and 5 mL of methanol to a reaction flask. Stir the reaction mixture at room temperature for 8 h. After TLC shows that the reaction is complete, pour the reaction mixture into 20 mL of water and adjust the pH to 5 with 1 M hydrochloric acid. Extract the resulting solution with ethyl acetate, dry the organic phase, and remove the solvent by rotary evaporation to give 320 mg of the target product, with the yeild of 100%.

### Step 3: Synthesis of 2-fluoro-5-(2-(4-fluoro-2-methylphenoxy)-5-(trifluoromethyl)benzamido) benzoyl chloride

Dissolve 2-fluoro-5-(2-(4-fluoro-2-methylphenoxy)-5-(trifluoromethyl) benzamido) benzoic acid (320 mg, 0.71 mmol) in 5 mL sulfoxide chloride, stir at 80 °C for 1 h. Remove the solvent by rotary evaporation, and extract twice with chloroform. The resulting solid is used directly in the next reaction step.

### Step 4: Synthesis of 3-(2-fluoro-5-(2-(4-fluoro-2-methylphenoxy)-5-(trifluoromethyl) benzamido) benzoyl)-2-oxopyrrolidine-1-carboxylic acid tert-butyl ester

At -70 °C and under the protection of nitrogen, drop n-butyllithium (2.5 M, 1.8 mL) into 10 mL anhydrous tetrahydrofuran solution of 2-oxopyrrolidine-1-carboxylate tert-butyl ester (657 mg, 3.55 mmol), stir for 0.5 h. Drop 2 mL tetrahydrofuran solution containing the solid obtained in the previous step to the reaction mixture, and keep the temperature below -60 °C. After stir of 1.5 h, pour the reaction mixture into 20 mL of aqueous ammonium chloride solution, extract with ethyl acetate. Dry the organic phase, remove the solvent by rotary evaporation, and purify by silica gel column (PE/EA=2/1) to obtain 180 mg of yellow solid, with the two-step yeild of 41%.

### Step 5: Synthesis of 3-((2-fluoro-5-(2-(4-fluoro-2-methylphenoxy)-5-(trifluoromethyl) benzamido) phenyl) (hydroxy) methyl)- 2-oxopyrrolidine-1-carboxylate tert-butyl ester

Dissolve 3-(2-fluoro-5-(2-(4-fluoro-2-methylphenoxy)-5-(trifluoromethyl) benzamido) benzoyl)-2-oxopyrrolidine -1-tert-butyl carboxylate (62 mg, 0.1 mmol) in 2 mL tetrahydrofuran, add 10 mg of sodium borohydride at room temperature. Stir for 4 h at room temperature, pour the mixture into 10 mL of water. Extract the organic phase with ethyl acetate, dry, remove the solvent by rotary evaporation, and purify by Prep-TLC (DCM/MeOH=20/1) to give 40 mg as a white solid, with the yeild of 65%.

### Step 6: Synthesis of 2-(4-fluoro-2-methylphenoxy)-N-(4-fluoro-3-(hydroxyl (2-oxopyrrolidin-3-yl) methyl) phenyl)-5-(trifluoromethyl) benzamide

Dissolve 3-((2-fluoro-5-(2-(4-fluoro-2-methylphenoxy)-5-(trifluoromethyl) benzamido) phenyl) (hydroxy) methyl)-2-oxopyrrolidine-1-carboxylate tert-butyl ester (40 mg, 0.065 mmol) in 2 mL hydrogen chloride-dioxane solution (2 M), stir at room temperature for 2 h. Remove the solvent by rotary evaporation and then add to 10 ml water. Adjust pH to alkalic with sodium carbonate. Extract the organic phase with ethyl acetate, dry, remove the solvent by rotary evaporation, and then purify by Prep-HPLC to give 25 mg as a white solid, with the yield of 74%.

LC/MS: m/z=521.1 [M+H]⁺.

1H NMR (400 MHz, d₆-DMSO) δ 1.62-1.72 (1H, m), 2.04-2.16 (4H, m), 3.10-3.18 (2H, m), 5.31 (1H, s), 5.59 (1H, d, *J*= 7.2 Hz), 6.83 (1H, d, *J*= 8.0 Hz), 7.10-7.25 (4H, m), 7.62-7.64 (2H, m), 7.78 (1H, d, *J*= 8.4 Hz), 7.89 (1H, d, *J*= 7.6 Hz), 7.97 (1H, s), 10.56 (1H, s).

### Example 26

### N-(3-(2-Amino-1-hydroxy-2-oxoethyl)-4-fluorophenyl)-2-(4-fluoro-2-methylphenoxy)-5-(tri fluoromethyl) benzamide

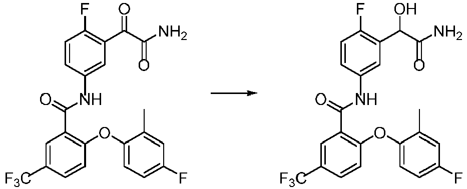

At room temperature, add 10 mg of sodium borohydride to 2 mL tetrahydrofuran solution of N-(3-(2-amino-2-oxoacetyl)-4-fluorophenyl)-2-(4-fluoro-2-methylphenoxy)-5-(trifluoromethyl) benzamide (48 mg, 0.10 mmol) , stir at room temperature for 4 hours. Pour the mixture into 10 mL of water, and extract with ethyl acetate, Dry the organic phase and remove the solvent by rotary evaporation, and purify by Prep-TLC (DCM/MeOH=20/1) to obtain 25 mg of white solid, with the yieldof 52%.

LC/MS: m/z=481.1 [M+H]⁺.

1H NMR (400 MHz, d₆-DMSO) δ 2.14 (3H, s), 5.08 (1H, d, *J*= 7.2 Hz), 6.24 (1H, d, *J*= 7.2 Hz), 6.80 (1H, d, *J*= 8.0 Hz), 7.12-7.26 (4H, m), 7.37 (1H, s), 7.45 (1H, s), 7.61-7.64 (1H, m), 7.75-7.80 (2H, m), 7.97 (1H, d, *J*= 2.4 Hz), 10.54 (1H, s).

### Example 27

### N-(3-(1 -Amino-1 -oxopropan-2-yl)-4-fluorophenyl)-2-(4-fluoro-2-methylphenoxy)-5-(trifluor omethyl) benzamide

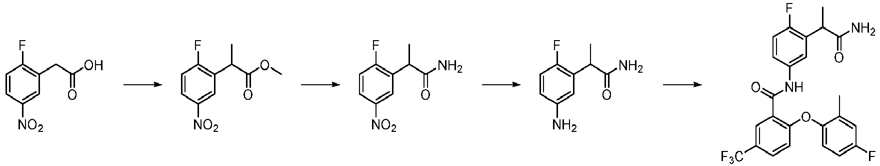

### Step 1: Synthesis of 2- (2-fluoro-5-nitrophenyl) methyl propionate

Dissolve 2-(2-fluoro-5-nitrophenyl) acetic acid (199 mg, 1.0 mmol) in 5 mL tetrahydrofuran, add potassium carbonate (414 mg, 3 mmol) and iodomethane (284 mg, 2.0 mmol), stir at room temperature overnight. Filter, collect the filtrate, remove the solvent by rotary evaporation, then purify by silica gel column chromatography (PE/EA=20/1) to give 80 mg of yellow solid, with the yeild of 35%.

### Step 2: Synthesis of 2-(2-fluoro-5-nitrophenyl) propionamide

Dissolve 2-(2-fluoro-5-nitrophenyl) methyl propionate (80 mg, 0.35 mmol) in 5 mL ammonia-methanol solution, stir overnight at 50°C. Remove the solvent in the mixture by rotary evaporation, then purify by Prep-TLC (PE/EA=5/1) to give 60 mg of yellow solid, with the yield of 80%.

### Step 3: Synthesis of 2-(5-amino-2-fluorophenyl) propionamide

Add 2-(2-fluoro-5-nitrophenyl) propionamide (60 mg, 0.28 mmol) and 5 mL of methanol to a reaction flask, then add 10%Pd/C (10 mg). Stir the reaction mixture at room temperature under hydrogen atmosphere overnight. Filter, wash the filter cake with methanol, combine the filtrate and remove the solvent by rotary evaporation to obtain 50 mg of light yellow solid, with the yield of 98%.

### Step 4: Synthesis of N-(3-(1-amino-1-oxopropan-2-yl)-4-fluorophenyl)-2-(4-fluoro-2-methylphenoxy)-5-(trifluoromet hyl) benzamide

Add 2-(4-fluoro-2-methylphenoxy)-5-(trifluoromethyl) benzoic acid (31 mg, 0.10 mmol), 2-(5-amino-2-fluorophenyl) propionamide (18 mg, 0.10 mmol), DIPEA (26 mg, 0.20 mmol) and 2 mL tetrahydrofuran to a reaction flask, then add HATU (57 mg, 0.15 mmol). Stir the reaction mixture at room temperature overnight, pour into 10 mL of water, and extract with ethyl acetate. Dry the organic phase, remove the solvent by rotary evaporation, and purify by Prep-TLC (PE/EA=2/1) to give 25 mg of white solid, with the yeild of 52%.

LC/MS: m/z=479.1 [M+H]⁺.

1H NMR (400 MHz, d₆-DMSO) δ 0.56-0.59 (2H, m), 1.02-1.04 (2H, m), 1.31 (3H, d,*J*= 7.6 Hz), 2.10-2.14 (1H, m), 2.16 (3H, s), 3.82 (1H, q, *J*= 7.6 Hz), 6.35 (1H, s), 6.94 (1H, s), 7.06-7.14 (3H, m), 7.21 (1H, d, *J*= 8.4 Hz), 7.41 (1H, s), 7.56-7.59 (1H, m), 7.66-7.68 (1H, m), 77.90 (1H, s), 10.42 (1H, s).

### Example 28

### 4-cyclopropyl-2-(4-fluoro-2-methylphenoxy)-N-(4-fluoro-3-(4-hydroxybutan-2-yl) phenyl)-5-(trifluoromethyl) benzamide

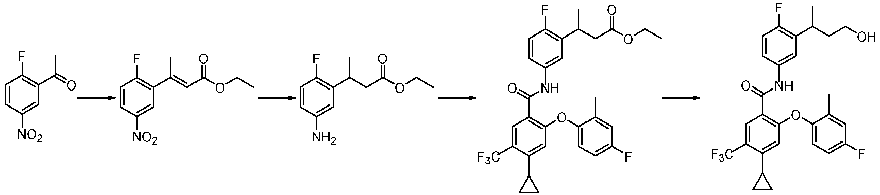

### Step 1: Synthesis of (E)-3-(2-fluoro-5-nitrophenyl) butyl-2-enoic acid ethyl ester

Suspend sodium hydrogen (308 mg, 7.7 mmol) at 60% content in anhydrous 5 mL tetrahydrofuran, cool to 0 °C under nitrogen protection, then drop triethyl phosphoacyl acetate (1.48 g, 6.6 mmol) into the reaction mixture slowly. Stir the mixture in ice bath for 30 min, drop 1-(2-fluoro-5-nitrophenyl) ethyl-1-ketone (1.0 g, 5.5 mmol) to the reaction mixture in ice bath, stir at room temperature for 2 h. The reaction mixture is quenched with ammonium chloride solution. Extract with ethyl acetate, dry the organic phase, remove the solvent by rotary evaporation, and purify by silica gel column chromatography (PE/EA=5/1) to give 900 mg of gray solid, with the yeild of 64%.

### Step 2: Synthesis of 3- (5-amino-2-fluorophenyl) ethyl butyrate

Dissolve (E)-3-(2-Fluoro-5-nitrophenyl) butyl-2-enoic acid ethyl ester (900 mg, 3.56 mmol) in 25 mL of methanol, and add palladium hydroxide (90 mg, 10% wt) after hydrogen gas is charged, stir under hydrogen atmosphere for 4 h. Filter, collect the filtrate and remove the solvent by rotary evaporation to give 750 mg of gray solid, with the yield of 95%.

### Step 3: Synthesis of 3-(5-(4-Cyclopropyl-2-(4-fluoro-2-methylphenoxy)-5-(trifluoromethyl) benzamido)-2-fluorophenyl) butanoic acid ethyl ester

Add 4-cyclopropyl-2-(4-fluoro-2-methylphenoxy)-5-(trifluoromethyl) benzoic acid (354 mg, 1 mmol), ethyl 3-(5-amino-2-fluorophenyl) butanoate (268 mg, 1.2 mmol), DIPEA (387 mg, 3 mmol) and 2 mL tetrahydrofuran to a reaction flask, then add HATU (456 mg 1.2 mmol), stir at room temperature overnight. Pour the reaction mixture into 10 mL of water, extract with ethyl acetate. Dry the organic phase, remove the solvent by rotary evaporation, and purify by silica gel column chromatography (PE/EA=2/1) to give 450 mg of white solid, with the yeild of 80%.

### Step 4: Synthesis of 4-cyclopropyl-2-(4-fluoro-2-methylphenoxy)-N-(4-fluoro-3-(4-hydroxybutan-2-yl) phenyl)-5-(trifluoromethyl) benzamide

Add 3-(5-(4-Cyclopropyl-2-(4-fluoro-2-methylphenoxy)-5-(trifluoromethyl) benzamido)-2-fluorophenyl) butanoic acid ethyl ester (450 mg, 0.8 mmol), anhydrous lithium chloride (13 mg, 0.3 mmol) and 15 mL of methanol to a reaction flask, then add sodium borohydride (152 mg, 4 mmol) in portions, stir for 2 h at room temperature. The reaction mixture is quenched with dilute hydrochloric acid, extract with ethyl acetate. Dry the organic phase, remove the solvent by rotary evaporation, and purify by silica gel column chromatography (DCM/MeOH=30/1) to give 370 mg of white solid, with the yield of 90%.

LC/MS: m/z=520.1 [M+H]⁺.

1H NMR (400 MHz, d₆-DMSO) δ 0.54-0.60 (2H, m), 1.02-1.08 (2H, m), 1.16 (3H, d, J= 6.8 Hz), 1.74-1.78 (2H, m), 2.07-2.19 (4H, m), 3.62- 3.65 (2H, m), 4.42-4.44 (1H, m), 5.77 (1H, s), 6.39 (1H, s), 7.01-7.14 (3H, m), 7.20 (1H, dd,J= 9.2 Hz, 2.8 Hz), 7.44-7.52 (1H, m), 7.56 (1H, dd, J= 6.6 Hz, 2.4 Hz), 7.91 (1H, s), 10.36 (1H, s).

### Example 29

### N-(3-(4-Aminobutan-2-yl)-4-fluorophenyl)-4-cyclopropyl-2-(4-fluoro-2-methylphenoxy)-5-( trifluoromethyl) benzamide

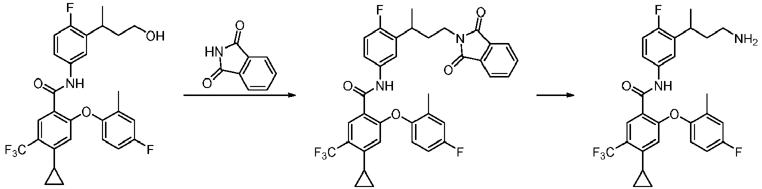

### Step 1: Synthesis of 4-cyclopropyl-N-(3-(4-(1,3-dioxoisoindolin-2-yl) butan-2-yl)-4-fluorophenyl)-2-(4-fluoro-2-methylphenoxy)-5-(trifluoromethyl) benzamide

Dissolve 4-Cyclopropyl-2-(4-fluoro-2-methylphenoxy)-N-(4-fluoro-3-(4-hydroxybutan-2-yl) phenyl)-5-(trifluoromethyl) benzamide (104 mg, 0.2 mmol), phthalimide (44 mg, 0.3 mmol) and triphenylphosphine (105 mg, 0.4 mmol) in 8 mL of tetrahydrofuran, drop DIAD (121 mg, 0.6 mmol) at room temperature under nitrogen protection. Stir the reaction solution at room temperature for 3 h. Add water, and extract with ethyl acetate. Dry the organic phase, remove the solvent by rotary evaporation and purify by silica gel column chromatography (PE/EA=3/1) to give 80 mg of gray solid, with the yield of 62%.

### Step 2: Synthesis of N-(3-(4-aminobutan-2-yl)-4-fluorophenyl)-4-cyclopropyl-2-(4-fluoro-2-methylphenoxy)-5-(triflu oromethyl) benzamide

Dissolve 4-cyclopropyl-N-(3-(4-(1,3-dioxoisoindolin-2-yl) butan-2-yl)-4-fluorophenyl)-2-(4-fluoro-2-methylphenoxy)-5-(trifluoromethyl) benzamide (80 mg, 0.12 mmol) in 8 mL of ethanol and 2 mL of hydrazine hydrate. Add water into the reaction mixture, and extract with ethyl acetate. Dry the organic phase, remove the solvent by rotary evaporation, and purify by Prep-TLC purification (DCM/MeOH=15/1) to obtain 25 mg of white solid, with the yeild of 40%.

LC/MS: m/z=519.1 [M+H]⁺.

1H NMR (400 MHz, d₆-DMSO) δ 0.54-0.60 (2H, m), 1.02-1.08 (2H, m), 1.18 (3H, d, J= 6.8 Hz), 1.65-1.75 (2H, m), 2.07-2.19 (4H, m), 2.55- 2.59 (2H, m), 3.05-3.10 (1H, m), 6.39 (1H, s), 7.04-7.13 (3H, m), 7.19 (1H, dd, *J*= 9.2 Hz, 2.8 Hz), 7.44-7.52 (1H, m), 7.61 (1H, dd, *J*= 6.6 Hz, 2.4 Hz), 7.91 (1H, s), 10.38 (1H, s).

### Example 30

### N-(3-(1-aminopropyl-2-yl)-4-fluorophenyl)-4-cyclopropyl-2-(4-fluoro-2-methylphenoxy)-5-( trifluoromethyl) benzamide

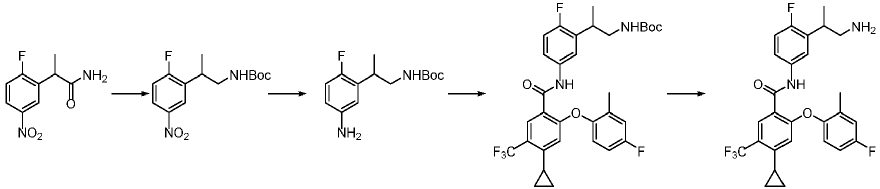

### Step 1: Synthesis of (2-(2-fluoro-5-nitrophenyl) propyl) carbamic acid tert-butyl ester

Add 2- (2-fluoro-5-nitrophenyl) propionamide (212 mg, 1.0 mmol), BH₃-THF (3 mL, 3 mmol, 1.0 M) in 10 mL of tetrahydrofuran, stir the solution at 50 °C for 2 h. The reaction is quenched by 15 mL of methanol, and stir the mixture for 30 min. then add (Boc)₂O (436 mg, 2.0 mmol), and stir for 1 h at room temperature. Rremove the solvent by rotary evaporation directly, and purify by silica gel column chromatography (PE/EA=3/1) to give 180 mg of off-white solid, with the yeild of 60%.

### Step 2. Synthesis of N-(3-(1-aminopropyl-2-yl)-4-fluorophenyl)-4-cyclopropyl-2-(4-fluoro-2-methylphenoxy)-5-(triflu oromethyl) benzamide

Example 30 is obtained by the same protocol as Example 3 and with the corresponding substrate as raw material.

LC/MS: m/z=505.2 [M+H]⁺.

1H NMR (400 MHz, d₆-DMSO) δ 0.54-0.61 (2H, m), 1.00-1.08 (2H, m), 1.18 (3H, d,*J*= 6.9 Hz), 2.07-2.19 (4H, m), 2.64-2.69 (1H, m), 2.72- 2.77 (1H, m), 2.94-3.01 (1H, m), 6.39 (1H, s), 7.01-7.14 (3H, m), 7.20 (1H, dd, *J*= 9.2 Hz, 2.8 Hz), 7.44-7.52 (1H, m), 7.57 (1H, dd, *J*= 6.6 Hz, 2.4 Hz), 7.91 (1H, s), 10.37 (1H, s).

### Example 31

### 4-Cyclopropyl-2-(4-fluoro-2-methylphenoxy)-N-(4-fluoro-3-(1-fluoro-3-hydroxypropyl) phenyl)-5-(trifluoromethyl) benzamide

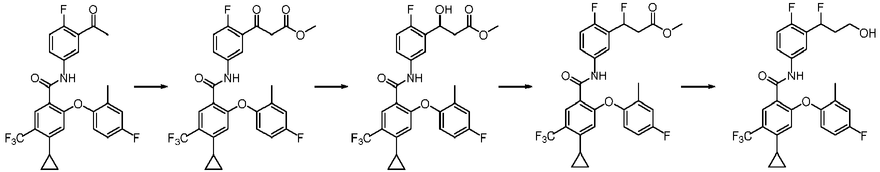

### Step 1: Synthesis of 3-(5-(4-cyclopropyl-2-(4-fluoro-2-methylphenoxy)-5 (trifluoromethyl) benzamido)-2-fluorophenyl)-3-oxypropionate methyl ester

Suspend Dimethyl carbonate (450 mg, 5 mmol) and 60% content of sodium hydrogen (100 mg, 2.5 mmol) in 15 mL of dried tetrahydofuran, then add N-(3-acetyl-4-fluorophenyl)-4-cyclopropyl-2-(4-fluoro-2-methylphenoxy)-5-(trifluoromethyl) benzamide (245 mg, 0.5 mmol) at room temperature. Stir at 70°C for 6 h, cool in an ice bath, and quench by adding ammonium chloride solution. Extract with ethyl acetate, dry the organic phase and concentrated to dryness, and purify by silica gel column chromatography (PE/EA=2/1) to give 165 mg of yellow solid, with the yeild of 60%.

### Step 2: Synthesis of methyl 3-(5-(4-cyclopropyl-2-(4-fluoro-2-methylphenoxy)-5-(trifluoromethyl) benzamido)-2-fluorophenyl)-3-hydroxypropanoate

Dissolve 3-(5-(4-cyclopropyl-2-(4-fluoro-2-methylphenoxy)-5-(trifluoromethyl) benzamido)-2-fluorophenyl)-3-oxypropionate methyl ester (165 mg, 0.3 mmol) in 5 mL of methanol, add sodium borohydride (57 mg, 1.5 mmol) in portions, stir at room temperature for 1 h, and the reaction is quenched by adding dilute hydrochloric acid, then extract with ethyl acetate. Dry the organic phase and concentrated to dryness, and purify by rapid silica gel column chromatography (PE/EA=1/1) to give 125 mg of yellow solid, with the yeild of 76%.

### Step 3: Synthesis of 3-(5-(4-cyclopropyl-2-(4-fluoro-2-methylphenoxy)-5-(trifluoromethyl) benzamido)-2-fluorophenyl)-3-methyl fluoropropionate

Add Methyl 3-(5-(4-cyclopropyl-2-(4-fluoro-2-methylphenoxy)-5-(trifluoromethyl) benzamido)-2-fluorophenyl)-3-hydroxypropanoate (125 mg, 0.23 mmol) and 5 mL of dichloromethane to a reaction flask, add DAST (74 mg, 0.46 mmol) to the reaction mixture at about -45 °C. Stir the reaction mixture at -45 °C for 2 hours, quench with water, extract with ethyl acetate. Dry the organic phase and concentrated to dryness, purify by silica gel column chromatography (PE/EA=2/1) to give 40 mg of off-white solid, with the yeild of 32%.

### Step 4: Synthesis of 4-cyclopropyl-2-(4-fluoro-2-methylphenoxy)-N-(4-fluoro-3-(1-fluoro-3-hydroxypropyl) phenyl)-5-(trifluoromethyl) benzamide

To the solution of methyl 3-(5-(4-cyclopropyl-2-(4-fluoro-2-methylphenoxy)-5-(trifluoromethyl) benzamido)-2-fluorophenyl)-3-fluoropropionate (40 mg, 0.07 mmol), anhydrous lithium chloride (4 mg, 0.1 mmol) and 3 mL of methanol, add sodium borohydride (27 mg, 0.7 mmol) in portions, stir at room temperature for 1 h, and quench by adding dilute hydrochloric acid. Extract the organic phase with ethyl acetate, dry and concentrate to dryness, and purify by silica gel Prep-TLC (DCM/MeOH=30/1) to give 25 mg of white solid, with the yeild of 66%.

LC/MS: m/z=524.2 [M+H]⁺.

### Example 32

### 4-Cyclopropyl-N-(3-(1,1-difluoro-3-hydroxypropyl)-4-fluorophenyl)-2-(4-fluoro-2-methylph enoxy)-5-(trifluoromethyl) benzamide

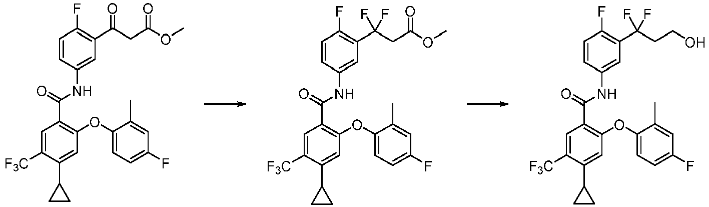

### Step 1: Synthesis of 3-(5-(4-cyclopropyl-2-(4-fluoro-2-methylphenoxy)-5-(trifluoromethyl) benzamido)-2-fluorophenyl)-3,3-methyl difluoropropionate

Add 3-(5-(4-cyclopropyl-2-(4-fluoro-2-methylphenoxy)-5-(trifluoromethyl) benzamido)-2-fluorophenyl)-3-methyl oxopropionate (200 mg, 0.37 mmol) and 15 mL of dichloromethane to a reaction flask, add DAST (119 mg, 0.74 mmol), stir for 2 hours, quench with water, and extract with ethyl acetate. Dry the organic phase and concentrated to dryness, purify by silica gel column chromatography (PE/EA=2/1) to give 45 mg of off-white solid, with the yeild of 21%.

### Step 2: Synthesis of 4-cyclopropyl-N-(3-(1,1-difluoro-3-hydroxypropyl)-4-fluorophenyl)-2-(4-fluoro-2-methylphenox y)-5-(trifluoromethyl) benzamide

Product of Example 32 is obtained by the same protocol as in Example 31 and with the corresponding substrate as raw material

LC/MS: m/z=542.2 [M+H]⁺.

### Example 33

### 4-Cyclopropyl-N-(3-(1,1-difluoro-2-((2-hydroxyethyl) amino)-2-oxoethyl)-4-fluorophenyl)-2-(4-fluoro-2-methylphenoxy)-5-(trifluoromethyl) benzamide

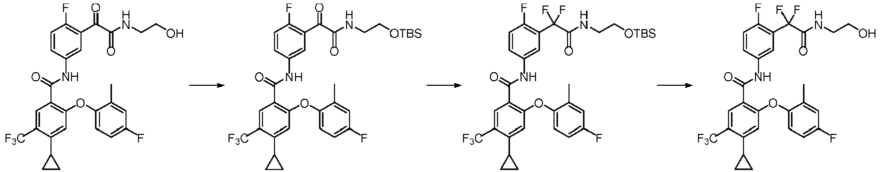

### Step 1: Synthesis of N-(3-(2-((2-((tert-butyldimethylmethylsilyl) oxy) ethyl) amino)-2-oxoacetyl)-4-fluorophenyl)-4-cyclopropyl-2-(4-fluoro-2-methylphenoxy)-5-(trifluorome thyl) benzamide

Dissolve 4-cyclopropyl-2-(4-fluoro-2-methylphenoxy)-N-(4-fluoro-3-(2-(((2-hydroxyethyl) amino)-2-oxoacetyl) phenyl)-5-(trifluoromethyl) benzamide (100 mg, 0.18 mmol) and TEA (101 mg, 1 mmol) in 10 mL of dichloromethane, then add TBSOTf (95 mg, 0.36 mmol) at room temperature. Remove the solvent by rotary evaporation directly, and purify by silica gel column chromatography (PE/EA=5/1) to give 90 mg, with the yeild of 74%.

### Step 2: Synthesis of N-(3-(2-(((2-((tert-butyldimethylsilyl) oxy) ethyl) amino)-1,1 -difluoro-2-oxoethyl)-4-fluorophenyl)-4-cyclopropyl-2-(4-fluoro-2-methylphenoxy)-5-(trifluoromethyl) benzamide

Add N-(3-(3-(2-((2-((tert-butyldimethylsilyl) oxy) ethyl) amino)-2-oxoacetyl)-4-fluorophenyl)-4-cyclopropyl-2-(4-fluoro-2-methylphenoxy)-5-(trifluorome thyl) benzamide (90 mg, 0.13 mmol) and 5 mL of dichloromethane to a reaction flask at room temperature, add DAST (65 mg, 0.4 mmol), stir at room temperature for 2 hours, quench with water, and extract with ethyl acetate. Dry the organic phase and concentrate to dryness, then purify by silica gel column chromatography (PE/EA=5/1) to give 18 mg of off-white solid, with the yeild of 19%.

### Step 3: Synthesis of 4-cyclopropyl-N-(3-(1,1-difluoro-2-((2-hydroxyethyl) amino)-2-oxoethyl)-4-fluorophenyl)-2-(4-fluoro-2-methylphenoxy)-5-(trifluoromethyl) benzamide

Add N-(3-(2-(((2-((tert-butyldimethylsilyl) oxy) ethyl) amino)-1,1 -difluoro-2-oxoethyl)-4-fluorophenyl)-4-cyclopropyl-2-(4-fluoro-2-methylphenoxy)-5-(trifluoromethyl) benzamide (18 mg), TBAF (0.2 mL, 1 M) and 2 mL of tetrahydrofuran to a reaction flask, stir at room temperature for 1 h. Add water, extract with ethyl acetate. Wash the organic phase twice with dilute hydrochloric acid, dry and concentrate, then purify by Prep-TLC (DCM/MeOH=20/1) to give 5 mg of off-white solid, with the yeild of 31%.

LC/MS: m/z=585.2 [M+H]⁺.

### Example 34

### N-(3-(2-aminoethoxy)-4-fluorophenyl)-4-cyclopropyl-2-(4-fluoro-2-methylphenoxy)-5-(trifl uoromethyl) benzamide

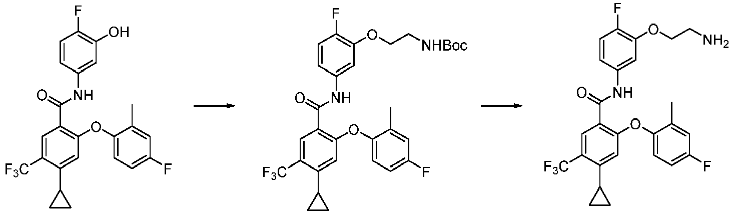

### Step 1: Synthesis of (2-(5-(4-(4-cyclopropyl-2-(4-fluoro-2-methylphenoxy)-5-(trifluoromethyl) benzamido)-2-fluorobenzeneoxy) ethyl) carbamate tert-butyl

Dissolve 4-cyclopropyl-2-(4-fluoro-2-methylphenoxy)-N-(4-fluoro-3-hydroxyphenyl)-5-(trifluoromethyl) benzamide (46 mg, 0.1 mmol), potassium carbonate (41 mg, 0.3 mmol) and (2-Bromoethyl) carbamate tert-butyl ester (33 mg, 0.15 mmol) in 3 mL acetonitrile, stir at 100 °C for 2 hours. Filter, collect the filtrate, remove the solvent by rotary evaporation to give 90 mg of yellow solid, the crude product was used directly for the next reaction step.

### Step 2: Synthesis of N-(3-(2-aminoethoxy)-4-fluorophenyl)-4-cyclopropyl-2-(4-fluoro-2-methylphenoxy)-5-(trifluoro methyl) benzamide

Dissolve (2-(5-(4-(4-cyclopropyl-2-(4-fluoro-2-methylphenoxy)-5-(trifluoromethyl) benzamide group)-2-fluorophenoxy) ethyl) tert-butyl carbamate (90 mg, crude) in 5 mL ethyl acetate-hydrochloric acid solution, stir at room temperature for 1 h. Remove the solvent by rotary evaporation, then add water, adjust pH to alkalic by sodium carbonate, and extract with ethyl acetate. Remove the solvent by rotary evaporation, and then purify by Prep-TLC purification (DCM/MeOH=12/1) to give 20 mg of white solid, with the two-step yeild of 39%.

LC/MS: m/z=507.2 [M+H]⁺.

1H NMR (400 MHz, d₆-DMSO) δ 0.56-0.61 (2H, m), 0.99-1.08 (2H, m), 2.11-2.13 (1H, m), 2.15 (3H, s), 2.91 (2H, d, *J*= 5.8 Hz), 3.94 (2H, d, *J*= 5.8 Hz), 6.38 (1H, s), 7.03-7.23 (5H, m), 7.52-7.61 (1H, m), 7.91 (1H, s), 10.41 (1H, s).

**Table I: The following compounds are prepared according to operations similar to those set forth in the above Examples, using the corresponding reagents as raw materials.**

| Example | Structure | MS (ESI) m/z | ¹H NMR | Synthesis Reference of Examples |
|---|---|---|---|---|
| 35 | 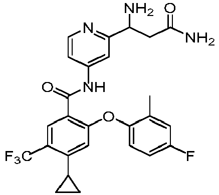 | 517.2 | NA | 1 |
| 36 | 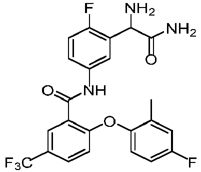 | 480.1 | 1H NMR (400 MHz, d₆-DMSO) δ 2.15 (3H, s), 4.50 (1H, s), 6.81 (1H, d, *J* = 8.4 Hz), 7.12-7.26 (5H, m), 7.49 (1H, s), 7.63-7.65 (1H, m), 7.75-7.78 (2H, m), 7.96 (1H, s), 10.56 (1H, s). | 9 |
| 37 | 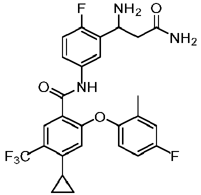 | 534.2 | 1H NMR (400 MHz, d₆-DMSO) δ 0.54-0.58 (2H, m), 1.01-1.06 (2H, m), 2.10-2.13 (1H, m), 2.15 (3H, s), 2.45-2.47 (2H, m), 4.51-4.54 (1H, m), 6.34 (1H, s), 6.92 (1H, s), 7.01-7.07 (3H, m), 7.22 (1H, d, *J* = 8.4 Hz), 7.47 (1H, s), 7.54-7.56 (1H, m), 7.87 (1H, d, *J* = 8.8 Hz), 7.91 (1H, s), 10.43 (1H, s). | 1 |
| 38 | 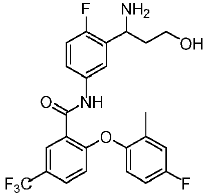 | 481.2 | 1H NMR (400 MHz, d₆-DMSO) δ 1.70-1.74 (2H, m), 2.15 (3H, s), 3.45-3.48 (2H, m), 4.20-4.24 (1H, m), 6.81 (1H, d, *J* = 8.4 Hz), 7.09-7.26 (5H, m), 7.54-7.58 (1H, m), 7.77 (1H, d, *J* = 8.4 Hz), 7.84 (1H, dd, *J* = 8.4 Hz, 2.8 Hz), 7.96 (1H, d, *J* = 2.4 Hz), 10.52 (1H, s). | 3 |
| 39 | 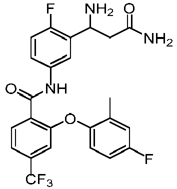 | 494.1 | 1H NMR (400 MHz, d₆-DMSO) δ 2.17 (3H, s), 2.38 (2H, d, *J* = 6.4 Hz), 4.46 (1H, t, *J* = 6.4 Hz), 6.87 (1H, s), 6.94 (1H, s), 7.11-7.15 (3H, m), 7.23 (1H, d, *J* = 8.4 Hz), 7.43 (1H, s), 7.54-7.60 (2H, m), 7.83-7.88 (2H, m), 10.58 (1H, s). | 1 |
| 40 | 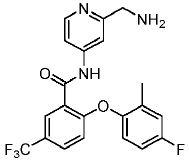 | 420.1 | NA | 12 |
| 41 | 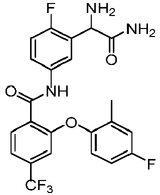 | 480.1 | 1H NMR (400 MHz, d₆-DMSO) δ 2.16 (3H, s), 4.49 (1H, s), 6.92 (1H, s), 7.10-7.23 (5H, m), 7.47 (1H, s), 7.57-7.59 (2H, m), 7.73 (1H, dd, *J* = 8.0 Hz, 4.4 Hz), 7.83 (1H, d, *J* = 8.4 Hz), 10.55 (1H, s). | 9 |
| 42 | 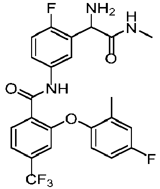 | 494.1 | NA | 9 |
| 43 | 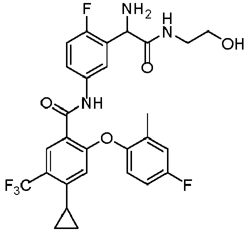 | 564.2 | 1H NMR (400 MHz, d₆-DMSO) δ 0.55-0.57 (2H, m), 1.01-1.03 (2H, m), 2.10-2.13 (1H, m), 2.14 (3H, s), 3.15-3.17 (2H, m), 3.35-3.40 (2H, m), 4.51 (1H, s), 4.70-4.73 (1H, m), 6.33 (1H, s), 7.07-7.11 (3H, m), 7.21 (1H, d, *J* = 8.4 Hz), 7.55-7.58 (1H, m), 7.63-7.65 (1H, m), 7.89 (1H, s), 8.10-8.13 (1H, m), 10.41 (1H, s). | 9 |
| 44 | 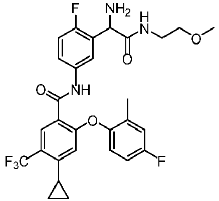 | 578.2 | 1H NMR (400 MHz, d₆-DMSO) δ 0.55-0.57 (2H, m), 1.01-1.03 (2H, m), 2.10-2.13 (1H, m), 2.14 (3H, s), 3.22 (3H, s), 3.24-3.27 (2H, m), 3.34-3.36 (2H, m), 4.51 (1H, s), 6.33 (1H, s), 7.07-7.13 (3H, m), 7.21 (1H, d, *J* = 8.4 Hz), 7.55-7.58 (1H, m), 7.65 (1H, dd, *J* = 7.2 Hz, 2.4 Hz), 7.89 (1H, s), 8.14-8.17 (1H, m), 10.41 (1H, s). | 9 |
| 45 | 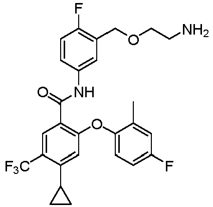 | 521.2 | NA | 34 |

### Example 46

### 4-Cyclopropyl-N-(3-(1,3-diaminobutyl)-4-fluorophenyl)-2-(4-fluoro-2-methylphenoxy)-5-(tr ifluoromethyl) benzamide

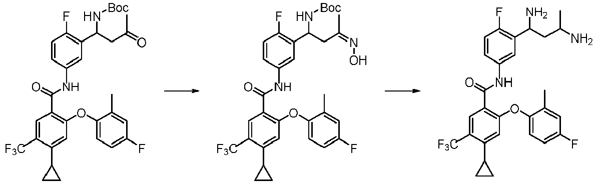

### Step 1: Synthesis of tert-butyl-(1-(5-(4-cyclopropyl-2-(4-fluoro-2-methylphenoxy)-5-(trifluoromethyl) benzamido)-2-fluorophenyl)-3-(hydroxyimino) butyl) carbamate

Dissolve (1-(5-(4-(4-cyclopropyl-2-(4-fluoro-2-methylphenoxy)-5-(trifluoromethyl) benzamido)-2-fluorobenzene base)-3-oxobutyl) tert-butyl carbamate (63 mg, 0.1 mmol), sodium acetate (16 mg, 0.2 mmol) and hydroxylamine hydrochloride (14 mg, 0.2 mmol) in 3 mL ethanol, stir at room temperature overnight. Pour the reaction mixture into 10 mL of water, extract with ethyl acetate, and dry the organic phase, remove the solvent by rotary evaporation to give 70 mg of yellow solid.

### Step 2: Synthesis of 4-cyclopropyl-N-(3-(1,3-diaminobutyl)-4-fluorophenyl)-2-(4-fluoro-2-methylphenoxy)-5-(trifluor omethyl) benzamide

Dissolve the crude product obtained from the reaction in the previous step in 5 mL of methanol, add 0.1 mL of concentrated hydrochloric acid and 50 mg of Pd/C (10%). Stir the reaction mixture under hydrogen atmosphere at 0.2 MPa pressure for 6 hours, filter. Remove the solvent by rotary evaporation, and purify by Prep-TLC (DCM/MeOH=10/1) to give 3 mg of white solid, with the two-step yeild of 5%.

LC/MS: m/z=534.2 [M+H]⁺.

### Example 47

### N-(3-(2-Amino-1-fluoroethyl)-4-fluorophenyl)-4-cyclopropyl-2-(4-fluoro-2-methylphenoxy) -5-(trifluoromethyl) benzamide

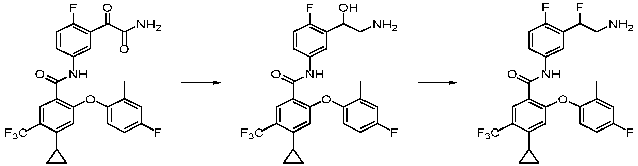

### Step 1: Synthesis of N-(3-(2-amino-1-hydroxyethyl)-4-fluorophenyl)-4-cyclopropyl-2-(4-fluoro-2-methylphenoxy)-5-( trifluoromethyl) benzamide

Dissolve N-(3-(2-amino-2-oxoacetyl)-4-fluorophenyl)-4-cyclopropyl-2-(4-fluoro-2-methylphenoxy)-5-(trifl uoromethyl) benzamide (104 mg, 0.2 mmol) in 3 mL of tetrahydrofuran, add lithium tetrahydroaluminum (23 mg, 0.6 mmol) at 0 °C. Stir the reaction mixture at room temperature overnight. Pour the reaction solution into 10 mL of water, extract with ethyl acetate. Dry the organic phase, remove the solvent by rotary evaporation, and purify by Prep-TLC (DCM/MeOH=10/1) to give 25 mg of yellow solid, with the yeild of 25%.

### Step 2: Synthesis of N-(3-(2-amino-1-fluoroethyl)-4-fluorophenyl)-4-cyclopropyl-2-(4-fluoro-2-methylphenoxy)-5-(tri fluoromethyl) benzamide

Dissolve N-(3-(2-amino-1-hydroxyethyl)-4-fluorophenyl)-4-cyclopropyl-2-(4-fluoro-2-methylphenoxy)-5-( trifluoromethyl) benzamide (25 mg, 0.05 mmol) in 3 mL of dichloromethane, cool the mixture to -20 °C, add diethylamino sulfur trifluoride (8 mg, 0.05 mmol), stir at 0 °C for 1 h. Pour the reaction solution into 10 mL of water, extracte with ethyl acetate. Remove the solvent by rotary evaporation, and purify by Prep-TLC (DCM/MeOH=15/1) to give 8 mg of a white-like solid, with the yeild of 32%.

LC/MS: m/z=509.2 [M+H]⁺.

1H NMR (400 MHz, d₆-DMSO) δ 0.58-0.60 (2H, m), 1.02-1.07 (2H, m), 1.18 (3H, d,J= 7.0 Hz), 2.13-2.17 (4H, m), 2.64-2.69 (1H, m), 2.72- 2.77 (1H, m), 2.94-3.01 (1H, m), 6.28 (1H, s), 7.05-7.13 (3H, m), 7.20 (1H, dd, *J=* 9.2 Hz, 2.8 Hz), 7.47-7.51 (1H, m), 7.57 (1H, dd, *J=* 6.6 Hz, 2.4 Hz), 7.91 (1H, s), 10.37 (1H, s).

### Example 48

### N-(3-(4-Amino-4-oxobutan-2-yl)-4-fluorophenyl)-4-cyclopropyl-2-(4-fluoro-2-methylpheno xy)-5-(trifluoromethyl) benzamide

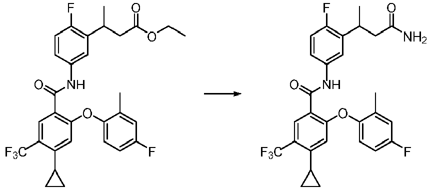

Dissolve 3-(5-(4-Cyclopropyl-2-(4-fluoro-2-methylphenoxy)-5-(trifluoromethyl)benzamido)-2-fluoropheny l) butanoic acid ethyl ester (56 mg, 0.1 mmol) in 5 mL of ammonia-methanol solution (7 M), stir at room temperature overnight, and remove the solvent by rotary evaporation directly, the crude product is slurried with a small amount of ethyl acetate to give 35 mg of white solid, with the yeild of 66%.

LC/MS: m/z=533.2 [M+H]⁺.

### Example 49

### 4-Cyclopropyl-2-(4-fluoro-2-methylphenoxy)-N-(4-fluoro-3-(3-hydroxy-1-(1H-imidazol-1-y l) propyl) phenyl)-5-(trifluoromethyl) benzamide

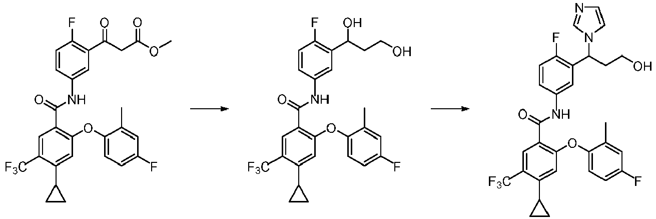

### Step 1: Synthesis of 4-cyclopropyl-N-(3-(1,3-dihydroxypropyl)-4-fluorophenyl)-2-(4-fluoro-2-methylphenoxy)-5-(trifl uoromethyl) benzamide

Dissolve 3-(5-(4-cyclopropyl-2-(4-fluoro-2-methylphenoxy)-5-(trifluoromethyl) benzamido)-2-fluorophenyl)-3-methyl oxopropionate (55 mg, 0.1 mmol) in 3 mL of methanol, add sodium borohydride (38 mg, 1.0 mmol) at room temperature. Stir the reaction mixture for 6 h at room temperature, then pour into 10 mL of water, and extract with ethyl acetate. Remove the solvent by rotary evaporation, then purify by Prep-TLC (DCM/MeOH=20/1) to give 30 mg of yellow solid, with the yeild of 58%.

### Step 2: Synthesis of 4-cyclopropyl-2-(4-fluoro-2-methylphenoxy)-N-(4-fluoro-3-(3-hydroxy-1-(1H-imidazol-1-yl) propyl) phenyl)-5-(trifluoromethyl) benzamide

Dissolve 4-cyclopropyl-N-(3-(1,3-dihydroxypropyl)-4-fluorophenyl)-2-(4-fluoro-2-methylphenoxy)-5-(trifl uoromethyl) benzamide (30 mg, 0.06 mmol) in 3 mL NMP, add carbonyl diimidazole (16 mg, 0.1 mmol). Stir the reaction mixture at 150 °C for two days. Direct purification by Prep-HPLC yield 8 mg white solid, with the yeild of 24%.

LC/MS: m/z=572.2 [M+H]⁺.

### Example 50

### 4-Cyclopropyl-2-(4-fluoro-2-methylphenoxy)-N-(4-fluoro-3-(2-oxo-1,4-diazol-5-yl) phenyl)-5-(trifluoromethyl) benzamide

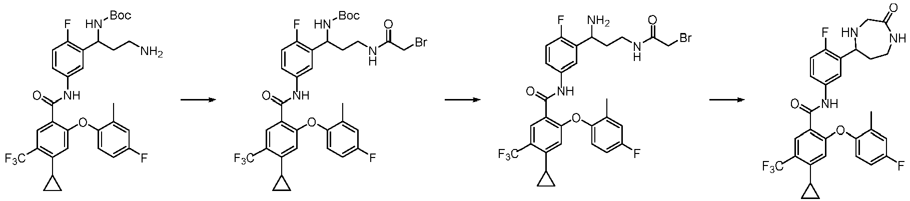

### Step 1: Synthesis of tert-butyl (3-(2-bromoacetylamino)-1-(5-(4-cyclopropyl-2-(4-fluoro-2-methylphenoxy)-5-(trifluoromethyl) benzamido)-2-fluorophenyl) propyl) carbamate

Dissolve Tert-butyl (3-amino-1-(5-(4-cyclopropyl-2-(4-fluoro-2-methylphenoxy)-5-(trifluoromethyl) benzamido)-2-fluorophenyl) propyl) carbamate (62 mg, 0.1 mmol) and triethylamine (20 mg, 0.2 mmol) in 5 mL of dichloromethane, cool the mixture to 0 °C, add bromoacetyl bromide (20 mg, 0.1 mmol). Stir the reaction solution at room temperature for 1 h and remove the solvent by rotary evaporation. The crude product is purify by Prep-TLC (PE/EA=2/1) to give 55 mg yellow solid, with the yeild of 74%.

### Step 2: Synthesis of N-(3-(1-amino-3-(2-bromoacetylamino) propyl)-4-fluorophenyl)-4-cyclopropyl-2-(4-fluoro-2-methylphenoxy)-5-(trifluoromethyl) benzamide

Dissolve Tert-butyl (3-(2-bromoacetamido)-1-(5-(4-cyclopropyl-2-(4-fluoro-2-methylphenoxy)-5-(trifluoromethyl) benzamido)-2-fluorophenyl) propyl) carbamate (55 mg, 0.07 mmol) in 5 mL of ethyl acetate-hydrochloric acid solution, stir for 1 h. Remove the solvent by rotary evaporation to give the hydrochloride salt of the target product, which is directly used in the next step.

### Step 3: Synthesis of 4-cyclopropyl-2-(4-fluoro-2-methylphenoxy)-N-(4-fluoro-3-(2-oxo-1,4-diazol-5-yl) phenyl)-5-(trifluoromethyl) benzamide

Dissolve the product of the previous step in 5 mL of tetrahydrofuran, add potassium carbonate (37 mg, 0.2 mmol), stir at room temperature overnight, and filter. Remove the solvent by rotary evaporation, then purify by Prep-TLC (DCM/MeOH=10/1) to give 16 mg of off-white solid, with the two-step yeild of 39%.

LC/MS: m/z=560.2 [M+H]⁺.

### Example 51

### 4-Cyclopropyl-2-(4-fluoro-2-methylphenoxy)-N-(4-fluoro-3-(2-oxohexahydropyrimidin-4-yl) phenyl)-5-(trifluoromethyl) benzamide

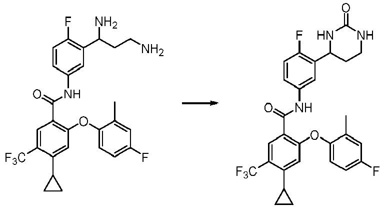

Dissolve 4-cyclopropyl-N-(3-(1,3-diaminopropyl)-4-fluorophenyl)-2-(4-fluoro-2-methylphenoxy)-5-(triflu oromethyl) benzamide (51 mg, 0.1 mmol) in 3 mL tetrahydrofuran, add potassium carbonate (37 mg, 0.2 mmol) and triphosgene (30 mg, 0.1 mmol), stir for 6 h at room temperature, then filter. Remove the solvent by rotary evaporation, and purify by Prep-TLC (DCM/MeOH=30/1) to give 20 mg of white solid, with the yeild of 36%.

LC/MS: m/z=546.2 [M+H]⁺.

1H NMR (400 MHz, d₆-DMSO) δ 0.57-0.60 (2H, m), 1.00-1.06 (2H, m), 1.70-1.77 (1H, m), 2.00-2.03 (1H, m), 2.12-2.16 (4H, m), 2.92-2.94 (1H, m), 3.09-3.11 (1H, m), 4.72-4.74 (1H, m), 6.34-6.37 (2H, m), 6.48 (1H, s), 7.05-7.23 (4H, m), 7.63-7.67 (2H, m), 7.89 (1H, s), 10.53 (1H, s).

### Example 52

### N-(3-Amino-4-fluorophenyl)-4-cyclopropyl-2-(4-fluoro-2-methylphenoxy)-5-(trifluorometh yl) benzamide

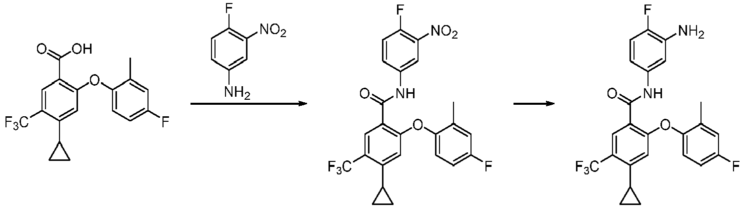

### Step 1: Synthesis of 4-cyclopropyl-2-(4-fluoro-2-methylphenoxy)-N-(4-fluoro-3-nitrophenyl)-5-(trifluoromethyl) benzamide

Add 4-cyclopropyl-2-(4-fluoro-2-methylphenoxy)-5-(trifluoromethyl) benzoic acid (35 mg, 0.1 mmol), 4-fluoro-3-nitroaniline (16 mg, 0.1 mmol), DIPEA (26 mg, 0.2 mmol) and 2 mL of tetrahydrofuran to a reaction flask, then add HATU (38 mg, 0.1 mmol). Stir the reaction mixture at room temperature overnight. Pour the reaction mixture into 10 mL of water, extract with ethyl acetate. Wash the organic phase twice with 1 M dilute hydrochloric acid, 1 M aqueous sodium hydroxide and saturated NaCl solution, respectively, then dry, remove the solvent by rotary evaporation to give 40 mg of yellow solid, with the yield of 82%.

### Step 2: Synthesis of N-(3-amino-4-fluorophenyl)-4-cyclopropyl-2-(4-fluoro-2-methylphenoxy)-5-(trifluoromethyl) benzamide

Dissolve 4-cyclopropyl-2-(4-fluoro-2-methylphenoxy)-N-(4-fluoro-3-nitrophenyl)-5-(trifluoromethyl) benzamide (40 mg, 0.08 mmol) in 5 mL of methanol, add 10 mg of Pd/C (10%). Stir the reaction mixture overnight under hydrogen atmosphere at 0.2 MPa pressure. Filter, remove the solvent by rotary evaporation and purify by Prep-TLC (DCM/MeOH=30/1) to give 26 mg of white solid, with the yeild of 68%.

LC/MS: m/z=463.1 [M+H]⁺.

1H NMR (400 MHz, d₆-DMSO) δ 0.55-0.58 (2H, m), 1.01-1.05 (2H, m), 2.08-2.15 (4H, m), 5.20 (2H, s), 6.34 (1H, s), 6.74-6.76 (1H, m), 6.90-6.92 (1H, m), 7.07-7.08 (2H, m), 7.19-7.21 (2H, m), 7.87 (1H, s), 10.13 (1H, s).

### Example 53

### N-(3-(4-Amino-5,5,5-trifluoropentan-2-yl)-4-fluorophenyl)-4-cyclopropyl-2-(4-fluoro-2-met hylphenoxy)-5-(trifluoromethyl) benzamide

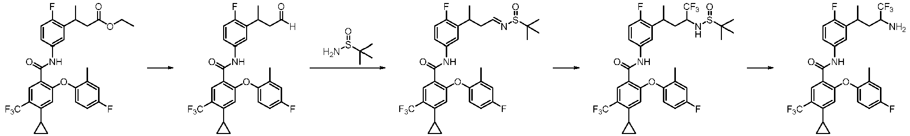

### Step 1: Synthesis of 4-cyclopropyl-2-(4-fluoro-2-methylphenoxy)-N-(4-fluoro-3-(4-oxobutan-2-yl) phenyl)-5-(trifluoromethyl) benzamide

Cool 3-(5-(4-Cyclopropyl-2-(4-fluoro-2-methylphenoxy)-5-(trifluoromethyl) benzamido)-2-fluorophenyl) butanoic acid ethyl ester (281 mg, 0.5 mmol) in 5 mL tetrahydrofuran to -70 °C under nitrogen protection, then drop diisobutylaluminium hydride (1 M hexane solution, 0.5 mL, 0.5 mmol). Stir the reaction mixture at -70 °C for 1 hour, pour into 20 mL of water, and extract with ethyl acetate. Dry the organic phase, remove the solvent by rotary evaporation, and purify by Prep-TLC (PE/EA=3/1) to give 140 mg of yellow solid, with the yeild of 54%.

### Step 2: Synthesis of N-(3-(4-((tert-butylsulfinyl) imino) butan-2-yl)-4-fluorophenyl)-4-cyclopropyl-2-(4-fluoro-2-methylphenoxy)-5-(trifluoromethyl) benzamide

Dissolve 4-cyclopropyl-2-(4-fluoro-2-methylphenoxy)-N-(4-fluoro-3-(4-oxobutan-2-yl) phenyl)-5-(trifluoromethyl) benzamide (140 mg, 0.27 mmol), cesium carbonate (88 mg, 0.27 mmol), and tert-butyl sulfinacylamino (33 mg, 0.27 mmol) in 3 mL dichloromethane, stir at 40 °C overnight. Filter, remove the solvent by rotary evaporation to give 165 mg of yellow solid, the crude product is used directly in the next reaction step.

### Step 3: Synthesis of N-(3-(4-((tert-butylsulfinyl) amino)-5,5,5-trifluoropentan-2-yl)-4-fluorophenyl)-4-cyclopropyl-2-(4-fluoro-2-methylphenoxy)-5-(trifluoromethyl) benzamide

Cool N-(3-(4-((tert-butylsulfinyl) imino) butan-2-yl)-4-fluorophenyl)-4-cyclopropyl-2-(4-fluoro-2-methylphenoxy)-5-(trifluoromethyl) benzamide (165 mg, 0.27 mmol) and tetrabutylammonium fluoride (26 mg, 0.1 mmol) in 5 mL tetrahydrofuran to 0 °C under nitrogen protection. C, add (trifluoromethyl) trimethylsilane (38 mg, 0.27 mmol), stir at 0 °C for 2 h. Pour the mixture into 20 mL of water, extract with ethyl acetate. Dry the organic phase, remove the solvent by rotary evaporation, and purify by Prep-TLC (PE/EA=1/1) to give 30 mg of yellow solid, with the two-step yeild of 16%.

### Step 4: Synthesis of N-(3-(4-amino-5,5,5-trifluoropentan-2-yl)-4-fluorophenyl)-4-cyclopropyl-2-(4-fluoro-2-methylph enoxy)-5-(trifluoromethyl) benzamide

Dissolve N-(3-(4-((tert-butylsulfinyl) amino)-5,5,5-trifluoropentan-2-yl)-4-fluorophenyl)-4-cyclopropyl-2-(4-fluoro-2-methylphenoxy)-5-(trifluoromethyl) benzamide (30 mg, 0.04 mmol) in 5 mL of ethyl acetate-hydrochloric acid solution, and stir for 1 h at room temperature, remove the solvent by rotary evaporation, then add water, adjust the pH to alkalic by sodium carbonate. Extract with ethyl acetate, remove the solvent by rotary evaporation, and purify by Prep-TLC (DCM/MeOH=10/1) to give 12 mg of off-white solid, with the yeild of 48%.

LC/MS: m/z=587.2 [M+H]⁺.

1H NMR (400 MHz, d₆-DMSO) δ 0.57-0.59 (2H, m), 1.02-1.04 (2H, m), 1.16-1.22 (3H, m), 1.75-1.77 (1H, m), 1.90-1.98 (2H, m), 2.10-2.15 (4H, m), 2.70-2.75 (0.5H, m), 3.21-3.26 (0.5H, m), 6.39 (1H, s), 7.00-7.19 (4H, m), 7.45-7.51 (1H, m), 7.55-7.60 (1H, m), 7.90 (1H, s), 10.37-10.38 (1H, m).

### Example 54

### N-(3-(5-(tert-butylamino)-4-hydroxypentan-2-yl)-4-fluorophenyl)-4-cyclopropyl-2-(4-fluoro -2-methylphenoxy)-5-(trifluoromethyl) benzamide

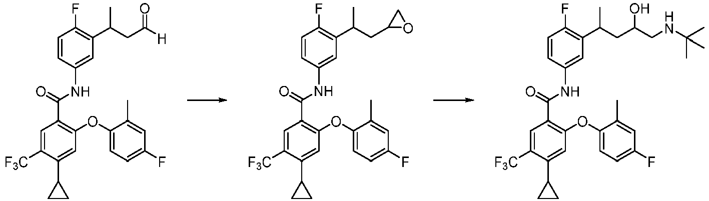

### Step 1: Synthesis of 4-cyclopropyl-2-(4-fluoro-2-methylphenoxy)-N-(4-fluoro-3-(1-(ethylene oxide-2-yl) propyl-2-yl) phenyl)-5-(trifluoromethyl) benzamide

Dissolve 4-cyclopropyl-2-(4-fluoro-2-methylphenoxy)-N-(4-fluoro-3-(4-oxobutan-2-yl) phenyl)-5-(trifluoromethyl) benzamide (45 mg, 0.087 mmol) in 2 mL of DMSO, and add sequentially with tert-butanol potassium (23 mg, 0.20 mmol) and trimethyl sulfoxide iodide (22 mg, 0.10 mmol). Stir the reaction solution at room temperature for 4 h. Pour into 10 mL of water, extract with ethyl acetate. Dry the organic phase, remove the solvent by rotary evaporation, and purify by Prep-TLC (PE/EA=2/1) to obtain 20 mg of white solid, with the yield of 43%.

### Step 2: Synthesis of N-(3-(5-(tert-butylamino)-4-hydroxypentan-2-yl)-4-fluorophenyl)-4-cyclopropyl-2-(4-fluoro-2-m ethylphenoxy)-5-(trifluoromethyl) benzamide

Dissolve 4-cyclopropyl-2-(4-fluoro-2-methylphenoxy)-N-(4-fluoro-3-(1-(oxiran-2-yl) propan-2-yl) phenyl)-5-(trifluoromethyl) benzamide (20 mg, 0.038 mmol) in 2 mL DMF, add tert-butylamine (15 mg, 0.20 mmol) and then heat to 100 °C and stir for 6 h. Pour the mixture into 10 mL of water, extract with ethyl acetate. Dry the organic phase, remove the solvent by rotary evaporation, purify by Prep-TLC (DCM/MeOH=10/1) to give 8 mg as a white solid, with the yeild of 35%.

LC/MS: m/z=605.3 [M+H]⁺.

1H NMR (400 MHz, d₆-DMSO) δ 0.56-0.58 (2H, m), 1.02-1.23 (14H, m), 1.60-1.68 (2H, m), 1.97-2.02 (1H, m), 2.10-2.14 (4H, m), 3.24-3.26 (2H, m), 3.60-3.62 (1H, m), 6.36-6.37 (1H, m), 7.06-7.12 (3H, m), 7.19-7.21 (1H, m), 7.41-7.44 (1H, m), 7.63-7.67 (1H, m), 7.89 (1H, s), 10.36-10.37 (1H, m).

### Example 55

### N-(3-(4-(tert-butylamino)-3-hydroxybutan-2-yl)-4-fluorophenyl)-4-cyclopropyl-2-(4-fluoro-2-methylphenoxy)-5-(trifluoromethyl) benzamide

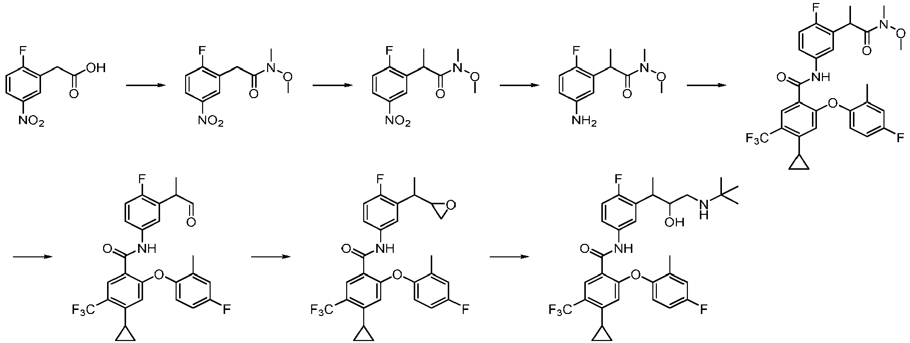

### Step 1: Synthesis of 2-(2-fluoro-5-nitrophenyl)-N-methoxy-N-methylacetamide

Add 2-fluoro-5-nitrophenylacetic acid (3000 mg, 15.1 mmol), N, O-dimethylhydroxylamine hydrochloride (1510 mg, 15.5 mmol), DIPEA (3870 mg, 30.0 mmol) and 50 mL tetrahydrofuran to a reaction flask, then add HATU (7600 mg, 20.0 mmol). Stir the reaction mixture for 4 h at room temperature, pour into 200 mL of water, extract with ethyl acetate, wash the organic phase twice with 1 M dilute hydrochloric acid, 1 M aqueous sodium hydroxide and saturated NaCl solution, respectively, then dry, remove the solvent by rotary evaporation to obtain 2900 mg of yellow solid, with the yield of 79%.

### Step 2: Synthesis of 2-(2-Fluoro-5-nitrophenyl)-N-methoxy-N-methylpropionamide

Dissolve 2-(2-fluoro-5-nitrophenyl)-N-methoxy-N-methylacetacylamino (1000 mg, 4.1 mmol) in 10 mL DMF, then add cesium carbonate (2608 mg, 8.0 mmol) and iodomethane (582 mg, 4.1 mmol) sequentially. Stir the reaction solution at room temperature overnight, pour into 100 mL of water, extract with ethyl acetate, wash the organic phase twice with NaCl solution, dry, remove the solvent by rotary evaporation to give 940 mg of yellow solid, with the yeild of 89%.

### Step 3: Synthesis of 2-(5-Amino-2-fluorophenyl)-N-methoxy-N-methylpropionamide

Dissolve 2-(2-Fluoro-5-nitrophenyl)-N-methoxy-N-methylpropionamide (940 mg, 3.7 mmol) in 20 mL of methanol, then add 100 mg of Pd/C (10%). Stir the reaction mixture overnight under hydrogen atmosphere at 0.2 MPa pressure. Filter, remove the solvent by rotary evaporation to give 700 mg of yellow solid, with the yeild of 84%.

### Step 4: Synthesis of 4-cyclopropyl-2-(4-fluoro-2-methylphenoxy)-N-(4-fluoro-3-(1-(methoxy (methyl) amino)-1-oxopropan-2-yl) phenyl)-5-(trifluoromethyl) benzamide

Add 4-cyclopropyl-2-(4-fluoro-2-methylphenoxy)-5-(trifluoromethyl) benzoic acid (210 mg, 0.6 mmol), 2-(5-amino-2-fluorophenyl)-N-methoxy-N-methylpropionacylamino (130 mg, 0.6 mmol), DIPEA (155 mg, 1.2 mmol) and 5 mL of tetrahydrofuran to a reaction flask, then add HATU (340 mg, 0.9 mmol). Stir the reaction mixture for 4 h at room temperature. Pour the reaction mixture into 20 mL of water, extract with ethyl acetate. Wash the organic phase twice with 1 M dilute hydrochloric acid, 1 M aqueous sodium hydroxide and saturated NaCl solution, respectively, then dry, remove the solvent by rotary evaporation to give 230 mg of yellow solid, with the yeild of 71%.

### Step 5: Synthesis of 4-cyclopropyl-2-(4-fluoro-2-methylphenoxy)-N-(4-fluoro-3-(1-oxopropan-2-yl) phenyl)-5-(trifluoromethyl) benzamide

Cool 5 mL tetrahydrofuran solution of 4-cyclopropyl-2-(4-fluoro-2-methylphenoxy)-N-(4-fluoro-3-(1-(methoxy(methyl)amino)-1-oxopr opan-2-yl) phenyl)-5-(trifluoromethyl) benzamide (230 mg, 0.4 mmol) to -70 °C under nitrogen protection, then drop diisobutylaluminium hydride (1 M n-hexane solution, 0.4 mL, 0.4 mmol). Stir the reaction mixture at -70 °C for 1.5 h. Pour into 20 mL of water, and extract with ethyl acetate. Dry the organic phase, remove the solvent by rotary evaporation to give 200 mg of yellow solid, with the yeild of 97%.

### Step 6: Synthesis of 4-cyclopropyl-2-(4-fluoro-2-methylphenoxy)-N-(4-fluoro-3-(1-(oxiran-2-yl) ethyl) phenyl)-5-(trifluoromethyl) benzamide

Dissolve 4-cyclopropyl-2-(4-fluoro-2-methylphenoxy)-N-(4-fluoro-3-(1-oxopropan-2-yl) phenyl)-5-(trifluoromethyl) benzamide (100 mg, 0.20 mmol) in 2 mL of DMSO, then add tert-butanol potassium (34 mg, 0.30 mmol) and trimethyl sulfoxide iodide (44 mg, 0.20 mmol) sequentially. Stir the reaction solution at room temperature overnight, then pour into 10 mL of water, and extract with ethyl acetate. Dry the organic phase, remove the solvent by rotary evaporation, and purify by Prep-TLC (PE/EA=2/1) to obtain 60 mg of white solid, with the yeild of 58%.

### Step 7: Synthesis of N-(3-(4-(tert-butylamino)-3-hydroxybutan-2-yl)-4-fluorophenyl)-4-cyclopropyl-2-(4-fluoro-2-met hylphenoxy)-5-(trifluoromethyl) benzamide

Dissolve 4-cyclopropyl-2-(4-fluoro-2-methylphenoxy)-N-(4-fluoro-3-(1-(oxiran-2-yl) ethyl) phenyl)-5-(trifluoromethyl) benzamide (52 mg, 0.10 mmol) in 2 mL DMF, add tert-butylamine (37 mg, 0.50 mmol), stir at 100 °C for 6 h. Pour the mixture into 10 mL of water, and extract with ethyl acetate. Dry the organic phase, remove the solvent by rotary evaporation, and purify by Prep-TLC (DCM/MeOH=10/1) to give 25 mg of white solid, with the yeild of 42%.

LC/MS: m/z=591.3 [M+H]⁺.

1H NMR (400 MHz, d₆-DMSO) δ 0.56-0.59 (2H, m), 0.96-1.05 (11H, m), 1.21-1.23 (3H, m), 2.10-2.14 (5H, m), 2.33-2.49 (2H, m), 3.00-3.04 (1H, m), 3.56 (1H, brs), 4.80 (1H, brs), 6.37 (1H, s), 7.04-7.09 (3H, m), 7.18-7.21 (1H, m), 7.49-7.52 (1H, m), 7.58-7.60 (1H, m), 7.89 (1H, s), 10.36 (1H, s).

### Example 56

### 4-cyclopropyl-2-(4-fluoro-2-methylphenoxy)-N-(4-fluoro-3-hydroxyphenyl)-5-(trifluoromet hyl) benzamide

### Example 57

### N-(3-(3-amino-2-hydroxypropoxy)-4-fluorophenyl)-4-cyclopropyl-2-(4-fluoro-2-methylphen oxy)-5-(trifluoromethyl) benzamide

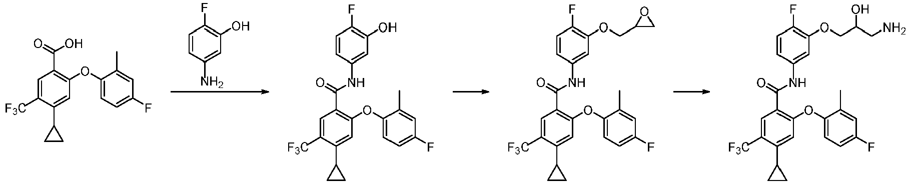

### Step 1: Synthesis of 4-cyclopropyl-2-(4-fluoro-2-methylphenoxy)-N-(4-fluoro-3-hydroxyphenyl)-5-(trifluoromethyl) benzamide

Add 4-cyclopropyl-2-(4-fluoro-2-methylphenoxy)-5-(trifluoromethyl) benzoic acid (210 mg, 0.6 mmol), 5-amino-2-fluorophenol (76 mg, 0.6 mmol), DIPEA (155 mg, 1.2 mmol) and 5 mL tetrahydrofuran to a reaction flask, then add HATU (340 mg, 0.9 mmol). Stir the reaction mixture at room temperature for 4 h. Pour into 20 mL of water, and extract with ethyl acetate. Wash the organic phase twice with 1 M dilute hydrochloric acid, 1 M aqueous sodium hydroxide and saturated NaCl solution, respectively, dry, remove the solvent by rotary evaporation to give 160 mg of yellow solid, with the yield of 58%.

LC/MS: m/z=464.1 [M+H]⁺.

1H NMR (400 MHz, d₆-DMSO) δ 0.55-0.59 (2H, m), 1.01-1.06 (2H, m), 2.10-2.15 (4H, m), 6.37 (1H, s), 6.96-7.00 (1H, m), 7.03-7.10 (3H, m), 7.20 (1H, dd, J= 9.2 Hz, 2.8 Hz), 7.45 (1H, dd, J= 8.4 Hz, 2.4 Hz), 7.88 (1H, s), 9.92 (1H, s), 10.29 (1H, s).

### Step 2: Synthesis of 4-cyclopropyl-2-(4-fluoro-2-methylphenoxy)-N-(4-fluoro-3-(oxiran-2-ylmethoxy) phenyl)-5-(trifluoromethyl) benzamide

Dissolve 4-cyclopropyl-2-(4-fluoro-2-methylphenoxy)-N-(4-fluoro-3-hydroxyphenyl)-5-(trifluoromethyl) benzamide (92 mg, 0.2 mmol) in 3 mL of acetonitrile, then add potassium carbonate (55 mg, 0.4 mmol) and epichlorohydrin (37 mg, 0.4 mmol). Stir the reaction solution at 60 °C overnight, then filter. Remove the solvent by rotary evaporation, and purify by Prep-TLC (PE/EA=2/1) to give 60 mg of off-white solid, with the yeild of 58%.

### Step 3: Synthesis of N-(3-(3-amino-2-hydroxypropoxy)-4-fluorophenyl)-4-cyclopropyl-2-(4-fluoro-2-methylphenoxy) -5-(trifluoromethyl) benzamide

Dissolve 4-cyclopropyl-2-(4-fluoro-2-methylphenoxy)-N-(4-fluoro-3-(oxiran-2-ylmethoxy) phenyl)-5-(trifluoromethyl) benzamide (52 mg, 0.10 mmol) in 2 mL DMF, add 0.5 mL ammonia, seal the container and heat to 100 °C, stir f 6 h. Pour the mixture into 10 mL water and extract with ethyl acetate. Dry the organic phase, remove the solvent by rotary evaporation, and purify by Prep-TLC (DCM/MeOH=10/1) to give 30 mg of white solid, with the yeild of 56%.

LC/MS: m/z=537.2 [M+H]⁺.

1H NMR (400 MHz, d₆-DMSO) δ 0.55-0.59 (2H, m), 1.01-1.05 (2H, m), 2.10-2.14 (4H, m), 2.86-2.89 (1H, m), 3.03-3.06 (1H, m), 3.97-4.00 (2H, m), 4.04-4.08 (1H, m), 5.83 (1H, d, *J*= 5.2 Hz), 6.36 (1H, s), 7.05-7.11 (3H, m), 7.17-7.22 (2H, m), 7.65-7.68 (1H, m), 7.89 (1H, s), 10.45 (1H, s).

### Example 58

### N-(3-(3-aminocyclobutoxy)-4-fluorophenyl)-4-cyclopropyl-2-(4-fluoro-2-methylphenoxy)-5 -(trifluoromethyl) benzamide

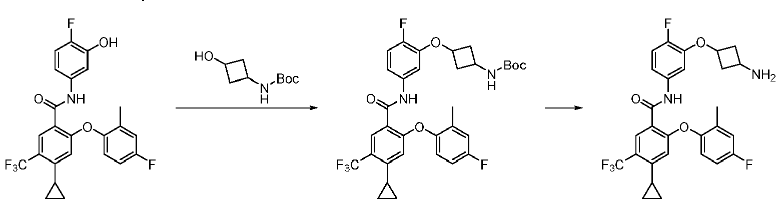

### Step 1: Synthesis of tert-butyl (3-(5-(4-cyclopropyl-2-(4-fluoro-2-methylphenoxy)-5-(trifluoromethyl) benzamido)-2-fluorophenoxy) cyclobutyl) carbamate

Dissolve 4-cyclopropyl-2-(4-fluoro-2-methylphenoxy)-N-(4-fluoro-3-hydroxyphenyl)-5-(trifluoromethyl) benzamide (93 mg, 0.2 mmol), (3-Hydroxycyclobutyl) carbamic acid tert-butyl ester (37 mg, 0.2 mmol) and triphenylphosphine (105 mg, 0.4 mmol) in 3 mL of tetrahydrofuran. Drop DIAD (80 mg, 0.4 mmol) at room temperature under the protection by nitrogen. Stir the reaction solution at room temperature for 3 h, then pour into water, and extracte with ethyl acetate. Dry the organic phase, remove the solvent by rotary evaporation, and purify by Prep-TLC (PE/EA=1/1) to give 85 mg of off-white solid, with the yeild of 67%.

### Step 2: Synthesis of N-(3-(3-aminocyclobutoxy)-4-fluorophenyl)-4-cyclopropyl-2-(4-fluoro-2-methylphenoxy)-5-(trifl uoromethyl) benzamide

Dissolve Tert-butyl (3-(5-(4-cyclopropyl-2-(4-fluoro-2-methylphenoxy)-5-(trifluoromethyl) benzamido)-2-fluorophenoxy) cyclobutyl) carbamate (63 mg, 0.1 mmol) in 5 mL ethyl acetate-hydrochloride acid solution and stir at room temperature for 1 h. Remove the solvent by rotary evaporation, and add water. Adjust pH to basic by sodium carbonate. Extract with ethyl acetate. dry the organic phase, remove the solvent by rotary evaporation, and purify by Prep-TLC (DCM/MeOH=10/1) to give 40 mg of off-white solid, with the yield of 75%.

LC/MS: m/z=533.2 [M+H]⁺.

1H NMR (400 MHz, d₆-DMSO) δ 0.57-0.61 (2H, m), 1.01-1.06 (2H, m), 1.75-1.81 (1H, m), 1.90-2.15 (7H, m), 2.25-2.31 (1H, m), 2.71-2.76 (1H, m), 2.98-3.02 (0.5H, m), 3.56-3.58 (0.5H, m), 4.22-4.25 (0.5H, m), 4.79-4.81 (0.5H, m), 6.40 (1H, s), 7.00-7.21 (5H, m), 7.32-7.41 (1H, m), 7.89 (1H, s), 10.38-10.39 (1H, m).

### Example 59

### 4-cyclopropyl-2-(4-fluoro-2-methylphenoxy)-N-(4-fluoro-3-(2-(methylsulfonamido) ethoxy) phenyl)-5-(trifluoromethyl) benzamide

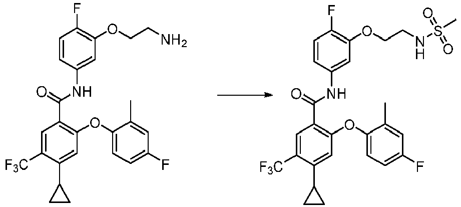

Dissolve N-(3-(2-aminoethoxy)-4-fluorophenyl)-4-cyclopropyl-2-(4-fluoro-2-methylphenoxy)-5-(trifluoro methyl) benzamide (51 mg, 0.1 mmol) and DIPEA (26 mg, 0.2 mmol) in 3 mL of tetrahydrofuran, and add methyl sulfonyl chloride (12 mg, 0.1 mmol) at room temperature. Stir the reaction solution at room temperature for 3 h. Pour into water, and extract with ethyl acetate. Dry the organic phase, remove the solvent by rotary evaporation, and purify by Prep-TLC (PE/EA=1/1) to give 45 mg as a white solid, with the yeild of 78%.

LC/MS: m/z=585.2 [M+H]⁺.

1H NMR (400 MHz, d₆-DMSO) δ 0.56-0.60 (2H, m), 1.01-1.05 (2H, m), 2.10-2.15 (4H, m), 2.96 (3H, s), 3.37-3.40 (2H, m), 4.03-4.06 (2H, m), 6.37 (1H, s), 7.05-7.07 (2H, m), 7.16-7.22 (3H, m), 7.33-7.34 (1H, m), 7.55-7.57 (1H, m), 7.90 (1H, s), 10.41 (1H, s).

### Example 60 4-cyclopropyl-2-(4-fluoro-2-methylphenoxy)-N-(4-fluoro-3-(3,3,3-trifluoro-2-hydroxypropo xy) phenyl)-5-(trifluoromethyl) benzamide

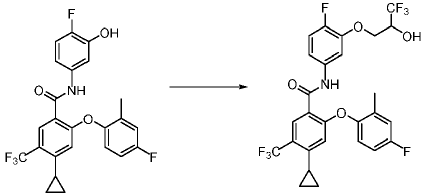

Dissolve 4-cyclopropyl-2-(4-fluoro-2-methylphenoxy)-N-(4-fluoro-3-hydroxyphenyl)-5-(trifluoromethyl) benzamide (46 mg, 0.1 mmol), potassium carbonate (41 mg, 0.3 mmol) and 3-bromo-1,1,1-trifluoropropan-2-ol (19 mg, 0.1 mmol) in 3 mL NMP, stir overnight at 110 °C. Pour the reaction mixture into 10 mL of water, extract with ethyl acetate. Dry the organic phase, remove the solvent by rotary evaporation, and purify by Prep-TLC (EA) to give 35 mg of white solid, with the yeild of 52%.

LC/MS: m/z=576.1 [M+H]⁺.

### Example 61

### 4-Cyclopropyl-2-(4-fluoro-2-methylphenoxy)-N-(4-fluoro-3-(2-oxo-2-(((2,2,2-trifluoroethyl) amino) ethoxy) phenyl)-5-(trifluoromethyl) benzamide

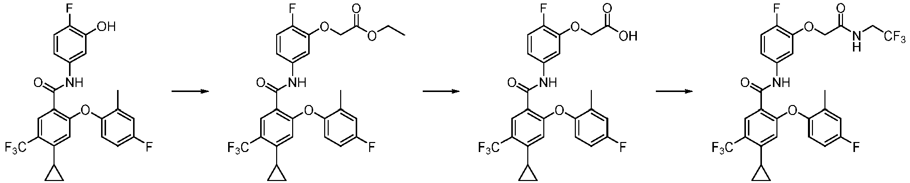

### Step 1: Synthesis of 2-(5-(4-cyclopropyl-2-(4-fluoro-2-methylphenoxy)-5-(trifluoromethyl) benzamide-(2-fluorophenoxy)-ethyl acetate

Dissolve 4-cyclopropyl-2-(4-fluoro-2-methylphenoxy)-N-(4-fluoro-3-hydroxyphenyl)-5-(trifluoromethyl) benzamide (46 mg, 0.1 mmol), potassium carbonate (28 mg, 0.2 mmol) and ethyl bromoacetate (17 mg, 0.1 mmol)in 3 mL of acetonitrile, stir at 60 °C for 2 hours.Filter, remove the solvent by rotary evaporation to obtain 55 mg yellow solid, the crude product is directly used in the next reaction.

### Step 2: Synthesis of 2-(5-(4-cyclopropyl-2-(4-fluoro-2-methylphenoxy)-5-(trifluoromethyl) benzamido)-2-fluorophenoxy) acetic acid

Dissolve 2-(5-(4-Cyclopropyl-2-(4-fluoro-2-methylphenoxy)-5-(trifluoromethyl) benzamido)-2-fluorophenoxy) acetic acid ethyl ester (55 mg, 0.1 mmol) in 2 mL of methanol, add 1 mL of water and lithium hydroxide monohydrate (17 mg, 0.4 mmol), stir at room temperature for 6 h. Pour the reaction mixture into 10 mL of water, adjust the pH to acidic by 1 M dilute hydrochloric acid. Extract the resulting reaction mixture with ethyl acetate, dry the organic phase, remove the solvent by rotary evaporation to obtain 45 mg of white solid, with the yeild of 87%.

### Step 3: Synthesis of 4-cyclopropyl-2-(4-fluoro-2-methylphenoxy)-N-(4-fluoro-3-(2-oxo-2-(((2,2,2-trifluoroethyl) amino) ethoxy) phenyl)-5-(trifluoromethyl) benzamide

Add 2-(5-(4-cyclopropyl-2-(4-fluoro-2-methylphenoxy)-5-(trifluoromethyl) benzamido)-2-fluorophenoxy) acetic acid (42 mg, 0.08 mmol), 2,2,2-trifluoroethylamine (10 mg, 0.1 mmol), DIPEA (26 mg, 0.2 mmol) and 3 mL of tetrahydrofuran to a reaction flask, then add HATU (38 mg, 0.1 mmol). Stir the reaction mixture for 4 h at room temperature, pour into 10 mL of water, and extract with ethyl acetate. Dry the organic phase, remove the solvent by rotary evaporation, and purify by Prep-TLC (PE/EA=1/1) to give 35 mg of white solid, with the yield of 73%.

LC/MS: m/z=603.2 [M+H]⁺.

1H NMR (400 MHz, d₆-DMSO) δ 0.56-0.58 (2H, m), 1.03-1.06 (2H, m), 2.10-2.14 (4H, m), 3.90-3.99 (2H, m), 4.65 (2H, s), 6.35 (1H, s), 7.04-7.11 (2H, m), 7.20, m), 7.20-7.22 (2H, m), 7.48 (1H, d, J= 7.6 Hz), 7.88 (1H, s), 7.17 (1H, t, J= 6.4 Hz), 10.44 (1H, s).

### Example 62

### (2-(5-(4-Cyclopropyl-2-(4-fluoro-2-methylphenoxy)-5-(trifluoromethyl) benzamido)-2-fluorophenoxy) ethyl) carbamate

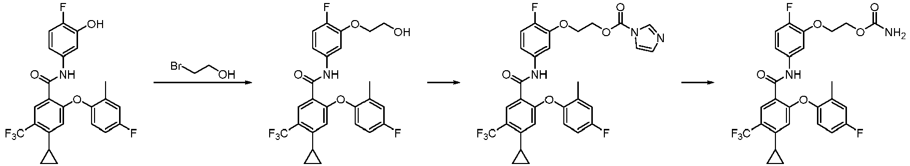

### Step 1: Synthesis of 4-cyclopropyl-2-(4-fluoro-2-methylphenoxy)-N-(4-fluoro-3-(2-hydroxyethoxy) phenyl)-5-(trifluoromethyl) benzamide

Stir the solution of 3 mL NMP containing 4-cyclopropyl-2-(4-fluoro-2-methylphenoxy)-N-(4-fluoro-3-hydroxyphenyl)-5-(trifluoromethyl) benzamide (92 mg, 0.2 mmol), potassium carbonate (41 mg, 0.3 mmol) and bromoethanol (25 mg, 0.2 mmol) at 110 °C for 5 hours. Pour the reaction mixture into 15 mL of water, extract with ethyl acetate. Dry the organic phase and remove the solvent by rotary evaporation to obtain 100 mg of yellow solid.

### Step 2: Synthesis of (2-(5-(4-cyclopropyl-2-(4-fluoro-2-methylphenoxy)-5-(trifluoromethyl) benzamido)-2-fluorophenoxy) ethyl) 1H-imidazole-1-carboxylate

Stir 4 mL tetrahydrofuran solution containing 4-cyclopropyl-2-(4-fluoro-2-methylphenoxy)-N-(4-fluoro-3-(2-hydroxyethoxy) phenyl)-5-(trifluoromethyl) benzamide (80 mg, 0.16 mmol) and CDI (32 mg, 0.2 mmol) for 4 h at 50 °C. Remove the solvent by rotary evaporation and the crude product obtained is used directly in the next step.

### Step 3: Synthesis of (2-(5-(4-cyclopropyl-2-(4-fluoro-2-methylphenoxy)-5-(trifluoromethyl) benzamido)-2-fluorophenoxy) ethyl) carbamate

Dissolve the product of the previous reaction step in 5 mL of ammonia-dioxane solution (1M), stir at 50°C for 4 h, and remove the solvent by rotary evaporation. The crude product is purified by Prep-HPLC to give 20 mg of white solid, with the two-step yeild of 23%.

LC/MS: m/z=551.2 [M+H]⁺.

1H NMR (400 MHz, d₆-DMSO) δ 0.57-0.59 (2H, m), 1.03-1.06 (2H, m), 2.10-2.15 (4H, m), 4.16-4.17 (2H, m), 4.25-4.26 (2H, m), 6.38 (1H, s), 7.05- 7.22 (5H, m), 7.55-7.57 (1H, m), 7.91 (1H, s), 10.41 (1H, s).

### Example 63

### 4-Cyclopropyl-N-(2-(2,3-dihydroxypropoxy) pyridin-4-yl)-2-(4-fluoro-2-methylphenoxy)-5-(trifluoromethyl) benzamide

### Example 64

### N-(2-(3-(tert-butylamino)-2-hydroxypropoxy) pyridin-4-yl)-4-cyclopropyl-2-(4-fluoro-2-methylphenoxy)-5-(trifluoromethyl) benzamide

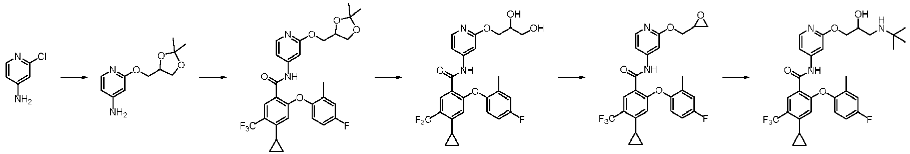

### Step 1: Synthesis of 2-(((2,2-dimethyl-1,3-dioxolan-4-yl) methoxy) pyridin-4-amine

Weigh 2-chloropyridin-4-amine (128 mg, 1.0 mmol) and acetone glycidol (264 mg, 2.0 mmol) into a flask, then add sodium (46 mg, 2.0 mmol), stir at 130 °C for 2 h. Cool the reaction mixture, and pour into 15 mL of water, extract with ethyl acetate. Dry the organic phase, remove the solvent by rotary evaporation and purify by column chromatography (PE/ EA=1/1) to obtain 100 mg of yellow oil, with the yeild of 44%.

### Step 2: Synthesis of 4-cyclopropyl-N-(2-(((2,2-dimethyl-1,3-dioxan-4-yl) methoxy) pyridin-4-yl)-2-(4-fluoro-2-methylphenoxy)-5-(trifluoromethyl) benzamide

Add 4-cyclopropyl-2-(4-fluoro-2-methylphenoxy)-5-(trifluoromethyl) benzoic acid (142 mg, 0.4 mmol), 2-(((2,2-dimethyl-1,3-dioxolan-4-yl) methoxy) pyridin-4-amine (100 mg, 0.44 mmol), DIPEA (103 mg, 0.8 mmol) and 5 mL tetrahydrofuran to a reaction flask, then add HATU (228 mg, 0.6 mmol). Stir the reaction mixture for 4 h at room temperature, pour into 20 mL of water, extract with ethyl acetate, dry the organic phase, remove the solvent by rotary evaporation, and purify by Prep-TLC (PE/EA=3/1) to give 130 mg of white solid, with the yeild of 58%.

### Step 3: Synthesis of 4-cyclopropyl-N-(2-(2,3-dihydroxypropoxy) pyridin-4-yl)-2-(4-fluoro-2-methylphenoxy)-5-(trifluoromethyl) benzamide

Dissolve 4-Cyclopropyl-N-(2-(((2,2-dimethyl-1,3-dioxolan-4-yl) methoxy) pyridin-4-yl)-2-(4-fluoro-2-methylphenoxy)-5-(trifluoromethyl) benzamide (130 mg, 0.23 mmol) in 5 mL ethyl acetate hydrochloride, stir at room temperature for 1 h, remove the solvent by rotary evaporation, and add water. Adjust the pH to alkalic by sodium carbonate, and extract with ethyl acetate. Dry the organic phase, remove the solvent by rotary evaporation, and purify by Prep-TLC (DCM/MeOH=10/1) to give 105 mg as a white solid, with the yield of 87%.

LC/MS: m/z=521.2 [M+H]⁺.

1H NMR (400 MHz, d₆-DMSO) δ 0.58-0.60 (2H, m), 1.02-1.06 (2H, m), 2.10-2.13 (4H, m), 3.40-3.50 (2H, m), 3.74-3.79 (1H, m), 4.08-4.13 (1H, m), 4.22-4.26 (1H, m), 6.39 (1H, s), 7.04-7.16 (5H, m), 7.91 (1H, s), 8.02 (1H, d, *J*= 5.6 Hz), 10.72 (1H, s).

### Step 4: Synthesis of 4-cyclopropyl-2-(4-fluoro-2-methylphenoxy)-N-(2-(oxirane-2-ylmethoxy) pyridin-4-yl)-5-(trifluoromethyl) benzamide

Stir 3 mL of acetonitrile solution containing 4-cyclopropyl-N-(2-(2,3-dihydroxypropoxy) pyridin-4-yl)-2-(4-fluoro-2-methylphenoxy)-5-(tri Fluoromethyl) benzamide (104 mg, 0.2 mmol), potassium carbonate (41 mg, 0.3 mmol) and methylxanthyl chloride (23 mg, 0.2 mmol) at room temperature for 1 hour, then heat to 60°C , stir for 2 hours. Filter, remove the solvent by rotary evaporation to obtain 120 mg of a yellow solid, and the crude product is directly used in the next reaction.

### Step 5: Synthesis of N-(2-(3-(tert-butylamino)-2-hydroxypropoxy) pyridin-4-yl)-4-cyclopropyl-2-(4-fluoro-2-methylphenoxy)-5-(trifluoromethyl) benzamide

Dissolve the product obtained in the previous step in 2 mL DMF, add tert-butylamine (73 mg, 1.0 mmol), stir at 100 °C for 6 h. Pour the mixture into 10 mL water, extract with ethyl acetate, dry the organic phase, remove the solvent by rotary evaporation, and purify by Prep-TLC (DCM/MeOH=10/1) to give 15 mg of off-white solid, with the two-step yeild of 13%.

LC/MS: m/z=576.2 [M+H]⁺.

### Example 65

### 4-Cyclopropyl-2-(4-fluoro-2-methylphenoxy)-N-(4-fluoro-3-(2-hydroxy-3-(2-hydroxyacetyl amino) propoxy) phenyl)-5-(trifluoromethyl) benzamide

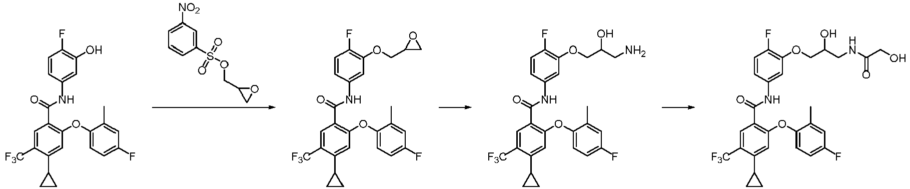

### Step 1: Synthesis of 4-cyclopropyl-2-(4-fluoro-2-methylphenoxy)-N-(4-fluoro-3-(oxiran-2-ylmethoxy) phenyl)-5-(trifluoromethyl) benzamide

Dissolve 4-Cyclopropyl-2-(4-fluoro-2-methylphenoxy)-N-(4-fluoro-3-hydroxyphenyl)-5-(trifluoromethyl) benzamide (92 mg, 0.2 mmol) in 3 mL DMF, then add potassium carbonate (55 mg, 0.4 mmol) and glycidyl m-nitrobenzenesulfonate (52 mg, 0.2 mmol). Stir the reaction solution at 60 °C for 6 hours. Pour the mixture into 20 mL of water, extract with ethyl acetate. Dry the organic phase, remove the solvent by rotary evaporation, and purify by Prep-TLC (PE/EA=3/1) to give 100 mg of off-white solid, with the yeild of 96%.

### Step 2: Synthesis of N-(3-(3-amino-2-hydroxypropoxy)-4-fluorophenyl)-4-cyclopropyl-2-(4-fluoro-2-methylphenoxy) -5-(trifluoromethyl) benzamide

Dissolve 4-Cyclopropyl-2-(4-fluoro-2-methylphenoxy)-N-(4-fluoro-3-(oxiran-2-ylmethoxy) phenyl)-5-(trifluoromethyl) benzamide (52 mg, 0.10 mmol) in 2 mL DMF, add 0.5 mL ammonia, seal the container and heat to 100 °C, stir for 6 h. Pour the mixture into 10 mL water and extract with ethyl acetate. Dry the organic phase, remove the solvent by rotary evaporation, and purify by Prep-TLC (DCM/MeOH=10/1) to give 30 mg of white solid, with the yeild of 56%.

### Step 3: Synthesis of 4-cyclopropyl-2-(4-fluoro-2-methylphenoxy)-N-(4-fluoro-3-(2-hydroxy-3-(2-hydroxyacetylamino) propoxy) phenyl)-5-(trifluoromethyl) benzamide

Add Hydroxyacetic acid (5 mg, 0.06 mmol), N-(3-(3-amino-2-hydroxypropoxy)-4-fluorophenyl)-4-cyclopropyl-2-(4-fluoro-2-methylphenoxy) -5-(trifluoromethyl) benzamide (30 mg, 0.06 mmol), DIPEA (26 mg, 0.2 mmol) and 3 mL of tetrahydrofuran to a reaction flask, then add HATU (38 mg, 0.1 mmol). Stir the reaction mixture for 4 h at room temperature, pour into 20 mL of water, and extract with ethyl acetate. Dry the organic phase, remove the solvent by rotary evaporation, and purify by Prep-TLC (DCM/MeOH=20/1) to give 20 mg of white solid, with the yield of 61%.

LC/MS: m/z=595.2 [M+H]⁺.

1H NMR (400 MHz, d₆-DMSO) δ 0.56-0.59 (2H, m), 1.01-1.06 (2H, m), 2.12-2.15 (4H, m), 3.18-3.23 (1H, m), 3.35-3.38 (1H, m), 3.81 (2H, d,J= 6.0 Hz), 3.85-3.92 (3H, m), 5.31 (1H, d,J= 4.8 Hz), 5.50 (1H, t,J= 5.6 Hz), 6.37 (1H, s), 7.05-7.08 (2H, m), 7.15-7.21 (3H, m), 7.54 (1H, d, J= 6.8 Hz), 7.70-7.73 (1H, m), 7.90 (1H, s), 10.40 (1H, s).

**Table II: The following compounds are prepared according to operations similar to those set forth in the above examples and with the corresponding reagents.**

| Exampl es | Structure | MS (ESI) m/z | ¹H NMR | Synthesis reference of examples |
|---|---|---|---|---|
| 66 | 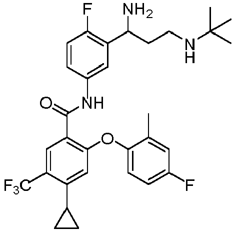 | 576.3 | 1H NMR (400 MHz, d₆-DMSO) δ 0.54-0.58 (2H, m), 1.02-1.05 (2H, m), 1.10 (9H, s), 1.74-1.76 (2H, m), 2.12-2.15 (4H, m), 2.70-2.73 (2H, m), 4.17-4.20 (1H, m), 6.34 (1H, s), 7.07-7.13 (3H, m), 7.21 (1H, d, *J =* 8.8 Hz), 7.50-7.53 (1H, m), 7.81-7.82 (1H, m), 7.89 (1H, s), 10.42 (1H, s). | 24 |
| 67 | 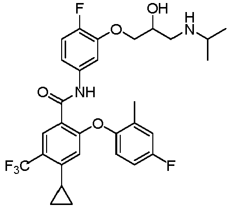 | 579.2 | 1H NMR (400 MHz, d₆-DMSO) δ 0.56-0.60 (2H, m), 0.96 (6H, d, *J* = 6.0 Hz), 1.01-1.06 (2H, m), 2.09-2.15 (4H, m), 2.55-2.58 (1H, m), 2.65-2.70 (2H, m), 3.88-3.91 (2H, m), 3.96-3.98 (1H, m), 5.04 (1H, brs), 6.38 (1H, s), 7.03-7.22 (5H, m), 7.53-7.55 (1H, m), 7.90 (1H, s), 10.39 (1H, s). | 57 |
| 68 | 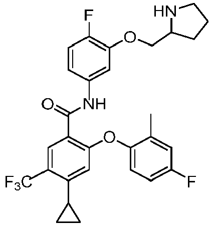 | 547.2 | 1H NMR (400 MHz, d₆-DMSO) δ 0.56-0.60 (2H, m), 1.01-1.06 (2H, m), 1.45-1.48 (1H, m), 1.62-1.71 (2H, m), 1.83-1.86 (1H, m), 2.10-2.15 (4H, m), 2.79-2.82 (2H, m), 3.40-3.45 (2H, m), 3.77-3.85 (2H, m), 6.38 (1H, s), 7.03-7.21 (5H, m), 7.54-7.56 (1H, m), 7.89 (1H, s), 10.38 (1H, s). | 58 |
| 69 | 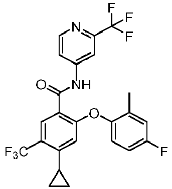 | 499.1 | 1H NMR (400 MHz, d₆-DMSO) δ 0.58-0.62 (2H, m), 1.02-1.07 (2H, m), 1.97-2.02 (1H, m), 2.13 (3H, s), 6.40 (1H, s), 7.03-7.10 (2H, m), 7.17-7.20 (1H, m), 7.82-7.85 (1H, m), 7.98 (1H, s), 8.14-7.15 (1H, m), 8.65 (1H, d, *J* = 5.6 Hz), 11.19 (1H, s). | 56 |
| 70 | 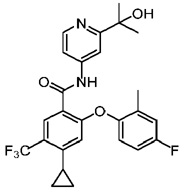 | 489.2 | 1H NMR (400 MHz, d₆-DMSO) δ 0.55-0.59 (2H, m), 1.01-1.06 (2H, m), 1.41 (6H, s), 2.10-2.14 (4H, m), 5.19 (1H, s), 6.34 (1H, s), 7.07-7.09 (2H, m), 7.19-7.21 (1H, m), 7.50-7.52 (1H, m), 7.90-7.92 (2H, m), 8.36 (1H, d, *J* = 5.2 Hz), 10.72 (1H, s). | 56 |
| 71 | 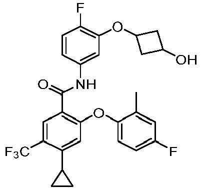 | 534.2 | 1H NMR (400 MHz, d₆-DMSO) δ 0.58-0.62 (2H, m), 1.02-1.07 (2H, m), 2.10-2.16 (4H, m), 2.28-2.38 (4H, m), 4.34-4.39 (1H, m), 4.80-4.83 (1H, m), 5.23 (1H, d, *J* = 5.2 Hz), 6.41 (1H, s), 7.03-7.22 (5H, m), 7.32-7.34 (1H, m), 7.90 (1H, s), 10.39 (1H, s). | 58 |
| 72 | 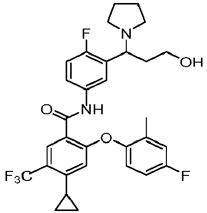 | 575.8 | NA | 8 |
| 73 | 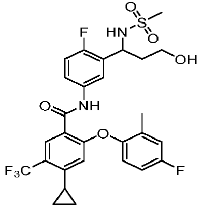 | 599.2 | NA | 6 |
| 74 | 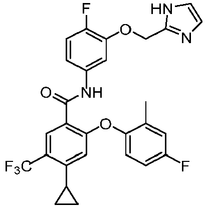 | 544.2 | NA | 58 |
| 75 | 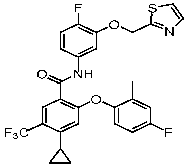 | 561.1 | NA | 58 |
| 76 | 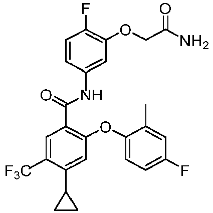 | 521.2 | NA | 58 |
| 77 | 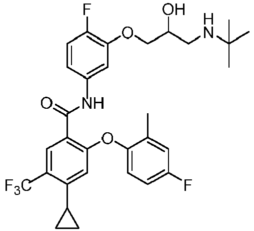 | 593.2 | NA | 57 |
| 78 | 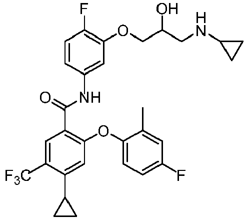 | 577.2 | 1H NMR (400 MHz, d₆-DMSO) δ 0.20-0.22 (2H, m), 0.35-0.37 (2H, m), 0.58-0.61 (2H, m), 1.02-1.07 (2H, m), 2.10-2.13 (2H, m), 3.15 (3H, s), 2.64-2.69 (1H, m), 2.73-2.77 (1H, m), 3.86-3.99 (3H, m), 5.02 (1H, s), 6.38 (1H, s), 7.03-7.22 (5H, m), 7.52-7.55 (1H, m), 7.90 (1H, s), 10.40 (1H, s). | 57 |
| 79 | 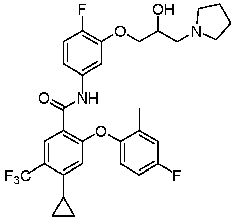 | 591.2 | 1H NMR (400 MHz, d₆-DMSO) δ 0.57-0.61 (2H, m), 1.02-1.07 (2H, m), 1.64-1.71 (4H, m), 2.13-2.15 (4H, m), 2.45-2.60 (5H, m), 2.64-2.68 (1H, m), 3.85-3.89 (1H, m), 3.96-4.04 (2H, m), 5.02 (1H, s), 6.39 (1H, s), 7.04-7.22 (5H, m), 7.54-7.56 (1H, m), 7.90 (1H, s), 10.40 (1H, s). | 57 |
| 80 | 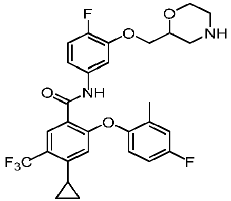 | 563.2 | NA | 58 |
| 81 | 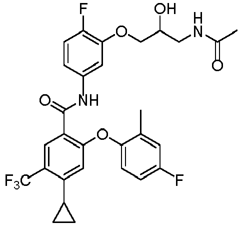 | 579.2 | 1H NMR (400 MHz, d₆-DMSO) δ 0.57-0.60 (2H, m), 1.02-1.06 (2H, m), 1.82 (3H, s), 2.10-2.16 (4H, m), 3.12-3.16 (1H, m), 3.24-3.29 (1H, m), 3.86-3.92 (3H, m), 5.25 (1H, d, *J =* 4.4 Hz), 6.38 (1H, s), 7.03-7.23 (5H, m), 7.54-7.58 (1H, m), 7.90 (1H, s), 7.93-7.96 (1H, m), 10.42 (1H, s). | 57 |
| 82 | 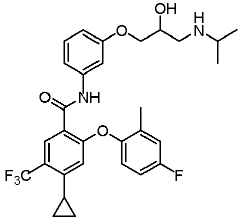 | 561.2 | NA | 57 |
| 83 | 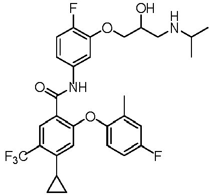 | 579.2 | 1H NMR (400 MHz, d₆-DMSO) δ 0.56-0.60 (2H, m), 0.97 (6H, d, *J =* 6.0 Hz), 1.01-1.06 (2H, m), 2.09-2.15 (4H, m), 2.54-2.59 (1H, m), 2.65-2.70 (2H, m), 3.88-3.91 (2H, m), 3.96-3.98 (1H, m), 5.05 (1H, brs), 6.38 (1H, s), 7.04-7.21 (5H, m), 7.53-7.55 (1H, m), 7.90 (1H, s), 10.39 (1H, s). | 57 |
| 84 | 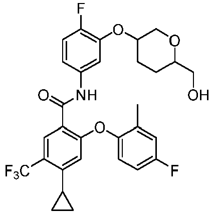 | 578.2 | 1H NMR (400 MHz, d₆-DMSO) δ 0.58-0.61 (2H, m), 1.02-1.06 (2H, m), 1.32-1.40 (1H, m), 1.54-1.61 (1H, m), 1.76-1.80 (1H, m), 2.10-2.15 (4H, m), 2.21-2.24 (1H, m), 3.24-3.42 (4H, m), 4.08-4.10 (1H, m), 4.17-4.24 (1H, m), 4.70 (1H, d, *J* = 5.6 Hz), 6.41 (1H, s), 7.00-7.10 (2H, m), 7.16-7.22 (3H, m), 7.60 (1H, d, *J* = 8.0 Hz), 7.91 (1H, s), 10.39 (1H, s). | 58 |
| 85 | 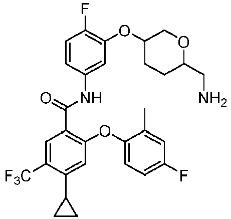 | 577.2 | 1H NMR (400 MHz, d₆-DMSO) δ 0.58-0.62 (2H, m), 1.02-1.07 (2H, m), 1.32-1.38 (1H, m), 1.58-1.62 (1H, m), 1.81-1.84 (1H, m), 2.10-2.15 (4H, m), 2.21-2.24 (1H, m), 2.61-2.70 (2H, m), 3.25-3.40 (3H, m), 4.11-4.14 (1H, m), 4.20-4.24 (2H, m), 6.40 (1H, s), 7.03-7.08 (2H, m), 7.16-7.22 (3H, m), 7.62-7.65 (1H, m), 7.90 (1H, s), 10.43 (1H, s). | 58 |
| 86 | 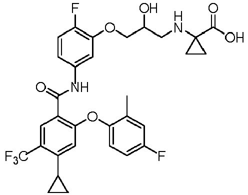 | 621.2 | NA | 57 |
| 87 | 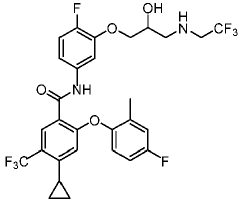 | 619.2 | 1H NMR (400 MHz, d₆-DMSO) δ 0.57-0.60 (2H, m), 1.02-1.05 (2H, m), 2.10-2.15 (4H, m), 2.33-2.39 (1H, m), 2.68-2.79 (2H, m), 3.27-3.31 (2H, m), 3.89-3.97 (3H, m), 5.15 (1H, d, *J* = 4.4 Hz), 6.38 (1H, s), 7.04-7.10 (2H, m), 7.15-7.22 (3H, m), 7.54-7.56 (1H, m), 7.90 (1H, s), 10.41 (1H, s). | 57 |
| 88 | 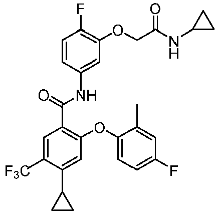 | 561.2 | 1H NMR (400 MHz, d₆-DMSO) δ 0.46-0.47 (2H, m), 0.57-0.60 (4H, m), 1.00-1.05 (2H, m), 2.11-2.15 (4H, m), 2.62-2.65 (1H, m), 4.47 (2H, s), 6.34 (1H, s), 7.03-7.10 (2H, m), 7.17-7.22 (3H, m), 7.46 (1H, d, *J* = 8.0 Hz), 7.88 (1H, s), 8.20 (1H, d, *J* = 4.0 Hz), 10.43 (1H, s). | 61 |
| 89 | 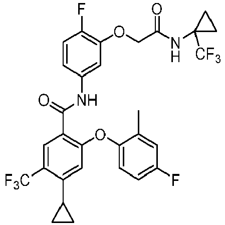 | 629.2 | 1H NMR (400 MHz, d₆-DMSO) δ 0.54-0.58 (2H, m), 1.00-1.07 (4H, m), 1.17-1.21 (2H, m), 2.10-2.14 (4H, m), 4.55 (2H, s), 6.34 (1H, s), 7.05-7.12 (3H, m), 7.16-7.22 (2H, m), 7.50 (1H, dd, *J* = 8.0 Hz, 2.4 Hz), 7.87 (1H, s), 8.99 (1H, s), 10.42 (1H, s). | 61 |
| 90 | 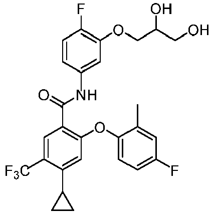 | 538.2 | 1H NMR (400 MHz, d₆-DMSO) δ 0.56-0.60 (2H, m), 1.01-1.05 (2H, m), 2.10-2.15 (4H, m), 3.45 (2H, t, *J* = 5.6 Hz), 3.79-3.89 (2H, m), 4.00 (1H, dd, *J* = 9.2 Hz, 4.0 Hz), 4.72 (1H, t, *J* = 5.6 Hz), 5.03 (1H, d, *J* = 4.8 Hz), 6.37 (1H, s), 7.04-7.22 (5H, m), 7.56 (1H, dd, *J =* 8.0 Hz, 2.0 Hz), 7.90 (1H, s), 10.40 (1H, s). | 57 |
| 91 | 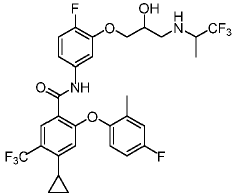 | 633.2 | NA | 57 |
| 92 | 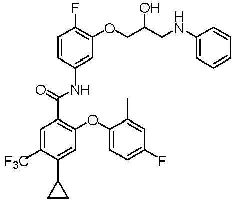 | 613.2 | 1H NMR (400 MHz, d₆-DMSO) δ 0.55-0.59 (2H, m), 1.01-1.06 (2H, m), 2.08-2.14 (4H, m), 3.05-3.11 (1H, m), 3.21-3.27 (1H, m), 3.94-4.02 (3H, m), 5.26 (1H, d, *J* = 4.0 Hz), 5.58 (1H, t, *J* = 6.0 Hz), 6.37 (1H, s), 6.49-6.52 (1H, m), 6.59-6.62 (2H, m), 7.03-7.20 (7H, m), 7.50 (1H, dd, *J* = 8.0 Hz, 2.0 Hz), 7.90 (1H, s), 10.41 (1H, s). | 57 |
| 93 | 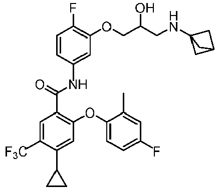 | 603.2 | NA | 57 |
| 94 | 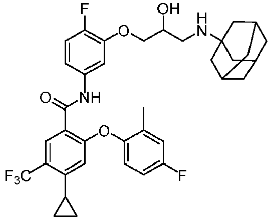 | 671.3 | 1H NMR (400 MHz, d₆-DMSO) δ 0.57-0.59 (2H, m), 1.01-1.05 (2H, m), 1.50-1.62 (12H, m), 1.99 (3H, s), 2.10-2.14 (4H, m), 2.61-2.67 (2H, m), 3.81 (1H, brs), 3.88-3.92 (1H, m), 3.98-4.01 (1H, m), 5.00 (1H, brs), 6.38 (1H, s), 7.04-7.21 (5H, m), 7.53-7.56 (1H, m), 7.90 (1H, s), 10.38 (1H, s). | 57 |
| 95 | 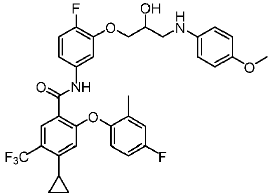 | 643.2 | 1H NMR (400 MHz, d₆-DMSO) δ 0.56-0.58 (2H, m), 1.01-1.06 (2H, m), 2.10-2.14 (4H, m), 3.02-3.05 (1H, m), 3.16-3.18 (1H, m), 3.62 (3H, s), 3.98-4.02 (3H, m), 5.22 (1H, d, *J =* 4.4 Hz), 5.32 (1H, t, *J* = 6.0 Hz), 6.37 (1H, s), 6.57 (2H, d, *J* = 8.8 Hz), 6.70 (2H, d, *J* = 8.8 Hz), 7.04-7.20 (6H, m), 7.56-7.59 (1H, m), 7.90 (1H, s), 10.38 (1H, s). | 57 |
| 96 | 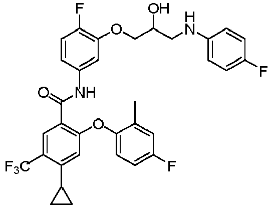 | 631.2 | 1H NMR (400 MHz, d₆-DMSO) δ 0.55-0.58 (2H, m), 1.02-1.06 (2H, m), 2.10-2.14 (4H, m), 3.02-3.05 (1H, m), 3.16-3.18 (1H, m), 3.98-4.03 (3H, m), 5.22 (1H, d, *J* = 4.4 Hz), 5.30 (1H, brs), 6.37 (1H, s), 6.85-6.88 (2H, m), 6.92-0.95 (2H, m), 7.05-7.22 (6H, m), 7.56-7.58 (1H, m), 7.91 (1H, s), 10.41 (1H, s). | 57 |
| 97 | 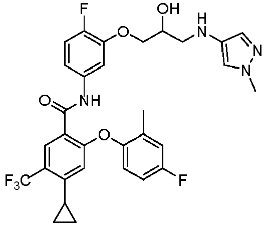 | 617.2 | 1H NMR (400 MHz, d₆-DMSO) δ 0.57-0.59 (2H, m), 1.02-1.05 (2H, m), 2.10-2.14 (4H, m), 2.89-2.92 (1H, m), 3.00-3.03 (1H, m), 3.66 (3H, s), 3.92-4.01 (3H, m), 4.33 (1H, brs), 5.17 (1H, d, *J* = 4.8 Hz), 6.37 (1H, s), 6.95 (1H, s), 7.02-7.21 (6H, m), 7.57 (1H, dd, *J* = 7.6 Hz, 1.6 Hz), 7.90 (1H, s), 10.39 (1H, s). | 57 |
| 98 | 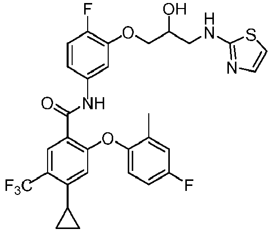 | 620.2 | 1H NMR (400 MHz, d₆-DMSO) δ 0.56-0.59 (2H, m), 1.01-1.06 (2H, m), 2.10-2.15 (4H, m), 3.87-4.03 (4H, m), 4.17-4.19 (1H, m), 6.37 (1H, s), 6.39 (1H, brs), 7.05-7.21 (7H, m), 7.58-7.61 (1H, m), 7.90 (1H, s), 10.41 (1H, s). | 57 |
| 99 | 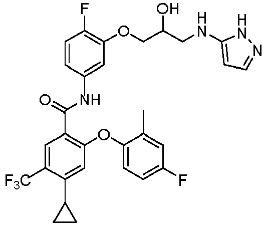 | 603.2 | 1H NMR (400 MHz, d₆-DMSO) δ 0.56-0.59 (2H, m), 1.01-1.06 (2H, m), 2.10-2.14 (4H, m), 3.09-3.13 (1H, m), 3.21-25 (1H, m), 3.92-4.01 (3H, m), 5.10-5.30 (2H, m), 5.47 (1H, d, *J* = 1.6 Hz), 6.37 (1H, s), 7.04-7.20 (6H, m), 7.34 (1H, s), 7.54-7.56 (1H, m), 7.90 (1H, s), 10.38 (1H, s), 11.56 (1H, brs). | 57 |
| 100 | 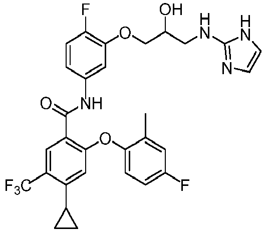 | 603.2 | NA | 57 |
| 101 | 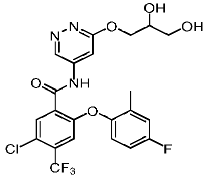 | 516.1 | 1H NMR (400 MHz, d₆-DMSO) δ 2.17 (3H, s), 3.44-3.47 (2H, m), 3.82-86 (1H, m), 4.28-4.33 (1H, m), 4.44-4.48 (1H, m), 4.69 (1H, t, *J* = 5.6 Hz), 5.00 (1H, d, *J* = 5.2 Hz), 7.09-7.11 (2H, m), 7.15 (1H, s), 7.22 (1H, d, *J* = 9.2 Hz), 7.51 (1H, s), 8.13 (1H, s), 8.93 (1H, s), 11.29 (1H, s). | 63 |
| 102 | 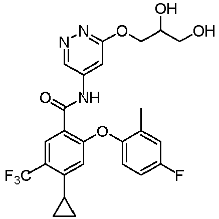 | 522.2 | 1H NMR (400 MHz, d₆-DMSO) δ 0.58-0.62 (2H, m), 1.03-1.07 (2H, m), 2.10-2.14 (4H, m), 3.43-3.46 (2H, m), 3.81-85 (1H, m), 4.26-4.31 (1H, m), 4.42-4.45 (1H, m), 4.69 (1H, t, *J* = 5.6 Hz), 5.00 (1H, d, *J* = 5.2 Hz), 6.41 (1H, s), 7.04-7.07 (2H, m), 7.20 (1H, dd, *J* = 9.2 Hz, 2.8 Hz), 7.50 (1H, d, *J* = 2.0 Hz), 7.97 (1H, s), 8.94 (1H, d, *J* = 2.0 Hz), 11.06 (1H, s). | 63 |
| 103 | 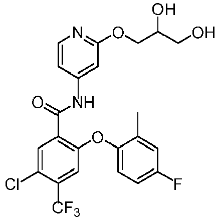 | 515.1 | 1H NMR (400 MHz, d₆-DMSO) δ 2.16 (3H, s), 3.40-3.43 (2H, m), 3.75-3.79 (1H, m), 4.09-4.13 (1H, m), 4.23-4.27 (1H, m), 4.64 (1H, t, *J* = 5.6 Hz), 4.92 (1H, d, *J* = 5.2 Hz), 7.09-7.16 (5H, m), 7.21 (1H, d, *J* = 9.2 Hz), 8.05 (1H, d, *J* = 5.6 Hz), 8.08 (1H, s), 10.94 (1H, s). | 63 |
| 106 | 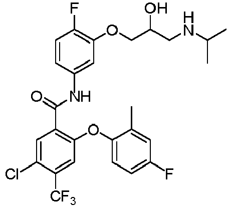 | 573.2 | NA | 57 |
| 107 | 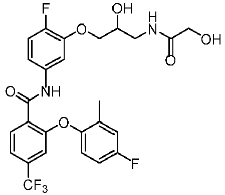 | 555.2 | 1H NMR (400 MHz, d₆-DMSO) δ 2.18 (3H, s), 3.17-3.24 (1H, m), 3.33-3.39 (1H, m), 3.81-3.83 (2H, m), 3.88-3.93 (3H, m), 5.33 (1H, d, *J =* 4.8 Hz), 5.52 (1H, t, *J* = 5.6 Hz), 6.97 (1H, s), 7.09-7.14 (2H, m), 7.17-7.23 (3H, m), 7.57-7.61 (2H, m), 7.71-7.74 (1H, m), 7.85 (1H, d, *J* = 7.6 Hz), 10.56 (1H, s). | 65 |
| 110 | 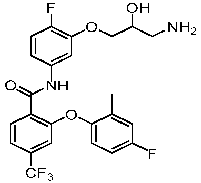 | 497.2 | NA | 57 |

### Example 113

### (S)-5-chloro-N-(2-(2,3-dihydroxypropoxy) pyridin-4-yl)-2-(4-fluoro-2-methylphenoxy)-4-(trifluoromethyl) benzamide

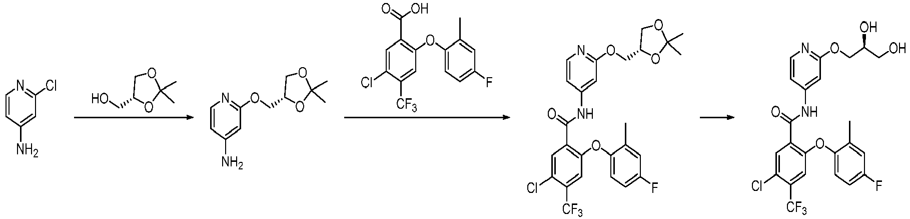

### Step 1: Synthesis of (R)-2-((2,2-dimethyl-1,3-dioxolane-4-yl) methoxy) pyridin-4-amine

Weigh 2-Chloropyridin-4-amine (128 mg, 1.0 mmol) and (R)-(-)-glycerol acetonide (264 mg, 2.0 mmol) to a flask, add sodium (46 mg, 2.0 mmol), stir the resulting mixture at 130 °C for 2 h. Cool the reaction mixture and pour into 15 mL of water, extract with ethyl acetate. Dry the organic phase, remove the solvent by rotary evaporation, and purify by by column chromatography (PE/EA=1/1) to obtain 120 mg of yellow oil, with the yeild of 54%.

### Step 2: Synthesis of (R)-5-chloro-N-(2-((2,2-dimethyl-1,3-dioxolan-4-yl) methoxy) pyridin-4-yl)-2-(4-fluoro-2-methylphenoxy))-4-(trifluoromethyl) benzamide

Add 5-chloro-2-(4-fluoro-2-methylphenoxy)-4-(trifluoromethyl) benzoic acid (186 mg, 0.54 mmol), (R)-2-((2,2-dimethyl-1,3-dioxolan-4-yl) methoxy) pyridin-4-amine (120 mg, 0.54 mmol), DIPEA (129 mg, 1.00 mmol) and 5 mL of tetrahydrofuran to a reaction flask, then add HATU (266 mg, 0.7 mmol), stir the reaction mixture at room temperature for 4 h. Pour the reaction mixture into 20 mL of water, extract with ethyl acetate. Dry the organic phase, remove the solvent by rotary evaporation, and purify by Prep-TLC (PE/EA=3/1) to give 180 mg of white solid, with the yeild of 60%.

### Step 3: Synthesis of (S)-5-chloro-N-(2-(2,3-dihydroxypropoxy) pyridin-4-yl)-2-(4-fluoro-2-methylphenoxy)-4-(trifluoromethyl) benzamide

Stir 5 mL ethyl acetate-hydrochloric acid solution of (R)-5-chloro-N-(2-((2,2-dimethyl-1,3-dioxolan-4-yl) methoxy) pyridin-4-yl)-2-(4-fluoro-2-methylphenoxy))-4-(trifluoromethyl) benzamide (100 mg, 0.18 mmol) at room temperature for 1 h. Remove the solvent by rotary evaporation, then add water, adjust the pH to alkalic by sodium carbonate, and extract with ethyl acetate. Dry the organic phase, remove the solvent by rotary evaporation, and purify by Prep-TLC (DCM/MeOH=10/1) to give 80 mg as a white solid, with the yield of 86%.

LC/MS: m/z=515.1 [M+H]⁺.

1H NMR (400 MHz, d₆-DMSO) δ 2.16 (3H, s), 3.40-3.43 (2H, m), 3.75-3.79 (1H, m), 4.09-4.13 (1H, m), 4.23-4.27 (1H, m), 4.64 (1H, t, J = 5.6 Hz), 4.92 (1H, d, J = 5.2 Hz), 7.09-7.16 (5H, m), 7.21 (1H, d, J = 9.2 Hz), 8.05 (1H, d, J = 5.6 Hz), 8.08 (1H, s), 10.94 (1H, s).

### Example 114

### (R)-5-chloro-N-(2-(2,3-dihydroxypropoxy) pyridin-4-yl)-2-(4-fluoro-2-methylphenoxy)-4-(trifluoromethyl) benzamide

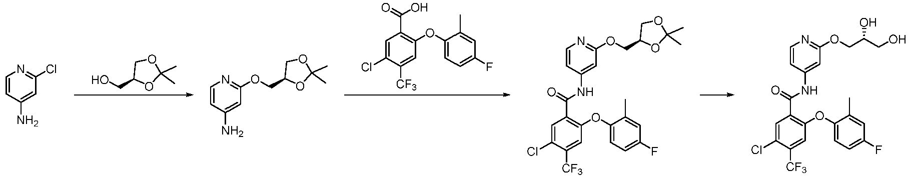

### Step 1: Synthesis of (S)-2-((2,2-dimethyl-1,3-dioxolane-4-yl) methoxy) pyridin-4-amine

Weigh 2-Chloropyridin-4-amine (128 mg, 1.0 mmol) and (S)-(+)-glycerol acetonide (264 mg, 2.0 mmol) to a flask, add sodium (46 mg, 2.0 mmol), and stir the resulting mixture at 130 °C for 2 h. Cool the reaction mixture, then pour into 15 mL of water, and extract with ethyl acetate. Dry the organic phase, remove the solvent by roary evaporation, and purify by column chromatography (PE/EA=1/1) to obtain 130 mg of yellow oil, with the yeild of 58%.

### Step 2: Synthesis of (S)-5-chloro-N-(2-((2,2-dimethyl-1,3-dioxolan-4-yl) methoxy) pyridin-4-yl)-2-(4-fluoro-2-methylphenoxy))-4-(trifluoromethyl) benzamide

Add 5-chloro-2-(4-fluoro-2-methylphenoxy)-4-(trifluoromethyl) benzoic acid (186 mg, 0.54 mmol), (S)-2-((2,2-dimethyl-1,3-dioxolan-4-yl) methoxy) pyridin-4-amine (120 mg, 0.54 mmol), DIPEA (129 mg, 1.00 mmol) and 5 mL of tetrahydrofuran to a reaction flask, then add HATU (266 mg, 0.7 mmol), stir the reaction mixture at room temperature for 4 h, pour into 20 mL of water, and extract with ethyl acetate. Dry the organic phase, remove the solvent by rotary evaporation and purify by Prep-TLC (PE/EA=3/1) to give 205 mg of white solid, with the yeild of 68%.

### Step 3: Synthesis of (R)-5-chloro-N-(2-(2,3-dihydroxypropoxy) pyridin-4-yl)-2-(4-fluoro-2-methylphenoxy)-4-(trifluoromethyl) benzamide

Stir the solution of (S)-5-chloro-N-(2-((2,2-dimethyl-1,3-dioxolan-4-yl) methoxy) pyridin-4-yl)-2-(4-fluoro-2-methylphenoxy)-4-(trifluoromethyl) benzamide (100 mg, 0.18 mmol) in 5 mL ethyl acetate-hydrochloride acid at room temperature for 1 hour, remove the solvent by rotary evaporation, then add water. Adjust the pH to alkalic by sodium carbonate, and extract with ethyl acetate. Dry the organic phase, remove the solvent by rotary evaporation, and purify by Prep-TLC (DCM/MeOH=10/1) to obtain 75 mg of white solid, with the yield of 81%.

LC/MS: m/z=515.1 [M+H]⁺.

1H NMR (400 MHz, d₆-DMSO) δ 2.16 (3H, s), 3.40-3.43 (2H, m), 3.75-3.79 (1H, m), 4.09-4.13 (1H, m), 4.23-4.27 (1H, m), 4.64 (1H, t, J = 5.6 Hz), 4.92 (1H, d, J = 5.2 Hz), 7.09-7.16 (5H, m), 7.21 (1H, d, J = 9.2 Hz), 8.05 (1H, d, J = 5.6 Hz), 8.08 (1H, s), 10.94 (1H, s).

### Example 117

### (R)-5-chloro-2-(4-fluoro-2-methylphenoxy)-N-(4-fluoro-3-(2-hydroxy-3-(2-hydroxyacetami do) propoxy) phenyl)-4-(trifluoromethyl)) benzamide

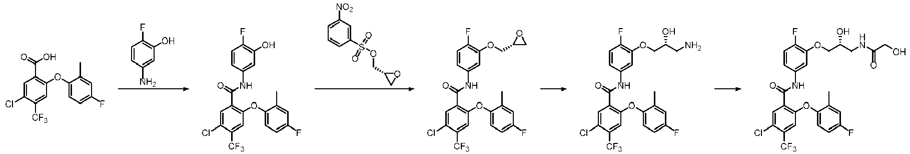

### Step 1: Synthesis of 5-chloro-2-(4-fluoro-2-methylphenoxy)-N-(4-fluoro-3-hydroxyphenyl)-4-(trifluoromethyl) benzamide

Add 5-chloro-2-(4-fluoro-2-methylphenoxy)-4-(trifluoromethyl) benzoic acid (209 mg, 0.6 mmol), 5-amino-2-fluorophenol (76 mg, 0.6 mmol), DIPEA (155 mg, 1.2 mmol) and 5 mL tetrahydrofuran to a reaction flask, then add HATU (340 mg, 0.9 mmol). Stir the reaction mixture for 4 h at room temperature, then pour into 20 mL of water, extract with ethyl acetate. Wash the organic phase twice with 1 M dilute hydrochloric acid, 1 M aqueous sodium hydroxide and saturated NaCl solution in turn, dry, remove the solvent by rotary evaporation to give 185 mg of yellow solid, with the yeild of 68%.

### Step 2: Synthesis of (R)-5-chloro-2-(4-fluoro-2-methylphenoxy)-N-(4-fluoro-3-(oxiran-2-ylmethoxy) phenyl)-4-(trifluoromethyl) benzamide

Dissolve 5-Chloro-2-(4-fluoro-2-methylphenoxy)-N-(4-fluoro-3-hydroxyphenyl)-4-(trifluoromethyl) benzamide (91 mg, 0.2 mmol) in 3 mL DMF, then add potassium carbonate (55 mg, 0.4 mmol) and (R)-(-)-m-nitrobenzenesulfonic acid glycidyl ester (52 mg, 0.2 mmol). Stir the reaction solution for 6 h at 60 °C, pour the mixure into 20 mL of water, extract with ethyl acetate. Dry the organic phase, remove the solvent by rotary evaporation, and purify by Prep-TLC (PE/EA=3/1) to give 100 mg of off-white solid, with the yeild of 96%.

### Step 3: Synthesis of (R)-N-(3-(3-amino-2-hydroxypropoxy)-4-fluorophenyl)-5-chloro-2-(4-fluoro-2-methylphenoxy)-4-(trifluoromethyl) benzamide

Dissolve (R)-5-chloro-2-(4-fluoro-2-methylphenoxy)-N-(4-fluoro-3-(oxiran-2-ylmethoxy) phenyl)-4-(trifluoromethyl) benzamide (100 mg, 0.19 mmol) in 2 mL DMF, add 0.5 mL ammonia, seal the container, then heat to 100 °C and stir for 6 h. Pour the mixture into 10 mL water, extracte with ethyl acetate. Dry the organic phase, remove the solvent by rotary evaporation, and purify by Prep-TLC (DCM/MeOH=10/1) to give 75 mg of white solid, with the yeild of 73%.

### Step 4: Synthesis of (R)-5-chloro-2-(4-fluoro-2-methylphenoxy)-N-(4-fluoro-3-(2-hydroxy-3-(2-hydroxyacetylamino) propoxy) phenyl)-4-(trifluoromethyl)) benzamide

Add Hydroxyacetic acid (10 mg, 0.12 mmol), (R)-N-(3-(3-amino-2-hydroxypropoxy)-4-fluorophenyl)-5-chloro-2-(4-fluoro-2-methylphenoxy)-4-(trifluoromethyl) benzamide (64 mg, 0.12 mmol), DIPEA (52 mg, 0.4 mmol) and 3 mL tetrahydrofuran to a reaction flask, then add HATU (76 mg, 0.2 mmol). Stir the reaction mixture at room temperature for 4 h. Pour the reaction mixture into 20 mL of water, and extract with ethyl acetate. Dry the organic phase, remove the solvent by rotary evaporation, and purify by Prep-TLC (DCM/MeOH=20/1) to give 45 mg of white solid, with the yeild of 63%.

LC/MS: m/z=589.1 [M+H]⁺.

1H NMR (400 MHz, d₆-DMSO) δ 2.18 (3H, s), 3.17-3.24 (1H, m), 3.34-3.39 (1H, m), 3.81-3.83 (2H, m), 3.88-3.93 (3H, m), 5.33 (1H, d, J = 4.8 Hz), 5.52 (1H, t, J = 5.6 Hz), 7.09-7.23 (6H, m), 7.53 (1H, dd, J = 7.6 Hz, 2.0 Hz), 7.72-7.75 (1H, m), 8.04 (1H, s), 10.63 (1H, s).

### Example 118

### (S)-5-chloro-2-(4-fluoro-2-methylphenoxy)-N-(4-fluoro-3-(2-hydroxy-3-(2-hydroxyacetamid o) propoxy) phenyl)-4-(trifluoromethyl)) benzamide

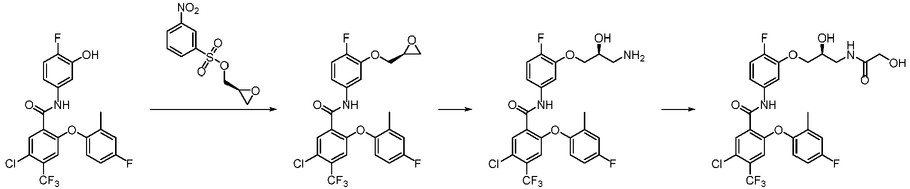

### Step 1: Synthesis of (S)-5-chloro-2-(4-fluoro-2-methylphenoxy)-N-(4-fluoro-3-(oxiran-2-ylmethoxy) phenyl)-4-(trifluoromethyl) benzamide

Dissolve 5-Chloro-2-(4-fluoro-2-methylphenoxy)-N-(4-fluoro-3-hydroxyphenyl)-4-(trifluoromethyl) benzamide (91 mg, 0.2 mmol) in 3 mL DMF, then add potassium carbonate (55 mg, 0.4 mmol) and (S)-(+)-m-nitrobenzenesulfonic acid glycidyl ester (52 mg, 0.2 mmol). Stir the reaction solution for 6 h at 60 °C. Pour the mixture into 20 mL of water, and extract with ethyl acetate. Dry the organic phase, remove the solvent by rotary evaporation, and purify by Prep-TLC (PE/EA=3/1) to give 95 mg of off-white solid, with the yeild of 92%.

### Step 2: Synthesis of (S)-N-(3-(3-amino-2-hydroxypropoxy)-4-fluorophenyl)-5-chloro-2-(4-fluoro-2-methylphenoxy)-4 -(trifluoromethyl) benzamide

Dissolve (S)-5-chloro-2-(4-fluoro-2-methylphenoxy)-N-(4-fluoro-3-(oxiran-2-ylmethoxy) phenyl)-4-(trifluoromethyl) benzamide (95 mg, 0.19 mmol) in 2 mL DMF, then add 0.5 mL ammonia, seal, stir for 6 h at 100 °C. Pour the mixture into 10 mL water, and extract with ethyl acetate. Dry the organic phase, remove the solvent by rotary evaporation, and purify by Prep-TLC (DCM/MeOH=10/1) to give 80 mg of white solid, with the yeild of 82%.

### Step 3: Synthesis of (S)-5-chloro-2-(4-fluoro-2-methylphenoxy)-N-(4-fluoro-3-(2-hydroxy-3-(2-hydroxyacetylamino) propoxy) phenyl)-4-(trifluoromethyl)) benzamide

Add Hydroxyacetic acid (10 mg, 0.12 mmol), (S)-N-(3-(3-amino-2-hydroxypropoxy)-4-fluorophenyl)-5-chloro-2-(4-fluoro-2-methylphenoxy)-4 -(trifluoromethyl) benzamide (64 mg, 0.12 mmol), DIPEA (52 mg, 0.4 mmol) and 3 mL tetrahydrofuran to a reaction flask, then add HATU (76 mg, 0.2 mmol). Stir the reaction mixture for 4 h at room temperature, pour into 20 mL of water, and extract with ethyl acetate. Dry the organic phase, remove the solvent by rotary evaporation and purify by Prep-TLC (DCM/MeOH=20/1) to give 50 mg of white solid, with the yeild of 70%.

LC/MS: m/z=589.1 [M+H]⁺.

1H NMR (400 MHz, d₆-DMSO) δ 2.18 (3H, s), 3.17-3.24 (1H, m), 3.34-3.39 (1H, m), 3.81-3.83 (2H, m), 3.88-3.93 (3H, m), 5.33 (1H, d, J = 4.8 Hz), 5.52 (1H, t, J = 5.6 Hz), 7.09-7.23 (6H, m), 7.53 (1H, dd, J = 7.6 Hz, 2.0 Hz), 7.72-7.75 (1H, m), 8.04 (1H, s), 10.63 (1H, s).

**Table III: The following compounds are prepared according to operations similar to those set forth in the above Examples and with the corresponding reagents.**

| Example | Structure | MS (ESI) m/z | ¹H NMR | Synthesis Reference Example |
|---|---|---|---|---|
| 104 | 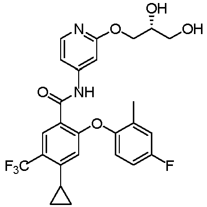 | 521.2 | 1H NMR (400 MHz, d₆-DMSO) δ 0.58-0.60 (2H, m), 1.02-1.06 (2H, m), 2.10-2.13 (4H, m), 3.40-3.50 (2H, m), 3.74-3.79 (1H, m), 4.08-4.13 (1H, m), 4.22-4.26 (1H, m), 4.63 (1H, t, *J* = 5.6 Hz), 4.91 (1H, d, *J* = 5.2 Hz), 6.39 (1H, s), 7.04-7.16 (5H, m), 7.91 (1H, s), 8.02 (1H, d, J = 5.6 Hz), 10.72 (1H, s). | 114 |
| 105 | 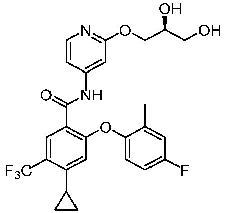 | 521.2 | 1H NMR (400 MHz, d₆-DMSO) δ 0.58-0.60 (2H, m), 1.02-1.06 (2H, m), 2.10-2.13 (4H, m), 3.40-3.50 (2H, m), 3.74-3.79 (1H, m), 4.08-4.13 (1H, m), 4.22-4.26 (1H, m), 4.63 (1H, t, *J* = 5.6 Hz), 4.91 (1H, d, *J* = 5.2 Hz), 6.39 (1H, s), 7.04-7.16 (5H, m), 7.91 (1H, s), 8.02 (1H, d, J = 5.6 Hz), 10.72 (1H, s). | 113 |
| 108 | 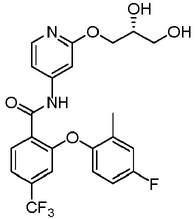 | 481.1 | 1H NMR (400 MHz, d₆-DMSO) δ 2.16 (3H, s), 3.41-3.44 (2H, m), 3.77-3.78 (1H, m), 4.10-4.14 (1H, m), 4.24-4.28 (1H, m), 4.63 (1H, t, *J* = 5.6 Hz), 4.90 (1H, d, *J* = 5.2 Hz), 6.99 (1H, s), 7.09-7.11 (2H, m), 7.16-7.18 (2H, m), 7.22 (1H, d, *J* = 8.8 Hz), 7.62 (1H, d, J = 8.0 Hz), 7.87 (1H, d, *J* = 7.6 Hz), 8.05 (1H, d, *J* = 5.6 Hz), 10.87 (1H, s). | 114 |
| 109 | 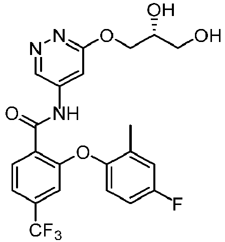 | 482.1 | 1H NMR (400 MHz, d₆-DMSO) δ 2.16 (3H, s), 3.44-3.47 (2H, m), 3.82-3.86 (1H, m), 4.28-4.33 (1H, m), 4.44-4.48 (1H, m), 4.68 (1H, t, *J* = 5.6 Hz), 4.99 (1H, d, *J* = 5.2 Hz), 7.03 (1H, s), 7.09-7.11 (2H, m), 7.22 (1H, dd, *J* = 8.8 Hz, 0.6 Hz), 7.53 (1H, d, *J* = 1.6 Hz), 7.64 (1H, d, *J* = 8.0 Hz), 7.91 (1H, d, *J* = 7.6 Hz), 8.95 (1H, d, *J* = 2.0 Hz), 11.21 (1H, s). | 114 |
| 111 | 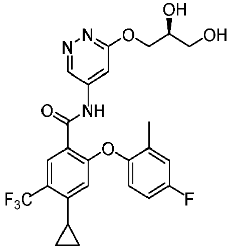 | 522.2 | 1H NMR (400 MHz, d₆-DMSO) δ 0.58-0.62 (2H, m), 1.03-1.07 (2H, m), 2.10-2.14 (4H, m), 3.43-3.46 (2H, m), 3.81-85 (1H, m), 4.26-4.31 (1H, m), 4.42-4.45 (1H, m), 4.69 (1H, t, *J* = 5.6 Hz), 5.00 (1H, d, *J* = 5.2 Hz), 6.41 (1H, s), 7.04-7.07 (2H, m), 7.20 (1H, dd, *J =* 9.2 Hz, 2.8 Hz), 7.50 (1H, d, *J* = 2.0 Hz), 7.97 (1H, s), 8.94 (1H, d, *J* = 2.0 Hz), XML/workb/2333/20168093.1 | 113 |
| 112 | 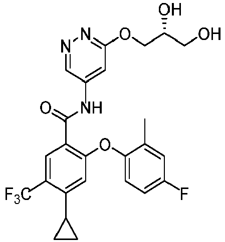 | 522.2 | 1H NMR (400 MHz, d₆-DMSO) δ 0.58-0.62 (2H, m), 1.03-1.07 (2H, m), 2.10-2.14 (4H, m), 3.43-3.46 (2H, m), 3.81-85 (1H, m), 4.26-4.31 (1H, m), 4.42-4.45 (1H, m), 4.69 (1H, t, *J* = 5.6 Hz), 5.00 (1H, d, *J* = 5.2 Hz), 6.41 (1H, s), 7.04-7.07 (2H, m), 7.20 (1H, dd, *J =* 9.2 Hz, 2.8 Hz), 7.50 (1H, d, *J* = 2.0 Hz), 7.97 (1H, s), 8.94 (1H, d, *J* = 2.0 Hz), 11.06 (1H, s). | 114 |
| 115 | 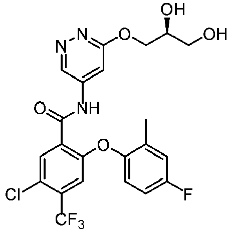 | 516.1 | 1H NMR (400 MHz, d₆-DMSO) δ 2.17 (3H, s), 3.44-3.47 (2H, m), 3.82-86 (1H, m), 4.28-4.33 (1H, m), 4.44-4.48 (1H, m), 4.69 (1H, t, *J* = 5.6 Hz), 5.00 (1H, d, *J* = 5.2 Hz), 7.09-7.11 (2H, m), 7.15 (1H, s), 7.22 (1H, d, *J* = 9.2 Hz), 7.51 (1H, s), 8.13 (1H, s), 8.93 (1H, s), 11.29 (1H, s). | 113 |
| 116 | 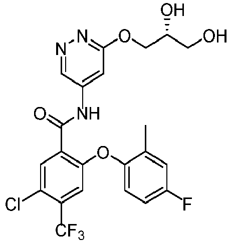 | 516.1 | 1H NMR (400 MHz, d₆-DMSO) δ 2.17 (3H, s), 3.44-3.47 (2H, m), 3.82-86 (1H, m), 4.28-4.33 (1H, m), 4.44-4.48 (1H, m), 4.69 (1H, t, *J* = 5.6 Hz), 5.00 (1H, d, *J* = 5.2 Hz), 7.09-7.11 (2H, m), 7.15 (1H, s), 7.22 (1H, d, *J* = 9.2 Hz), 7.51 (1H, s), 8.13 (1H, s), 8.93 (1H, s), 11.29 (1H, s). | 114 |
| 119 | 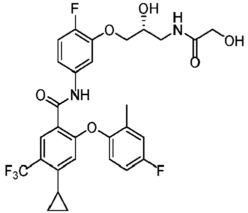 | 595.2 | 1H NMR (400 MHz, d₆-DMSO) δ 0.56-0.59 (2H, m), 1.01-1.06 (2H, m), 2.12-2.15 (4H, m), 3.18-3.23 (1H, m), 3.35-3.38 (1H, m), 3.81 (2H, d, J = 6.0 Hz), 3.85-3.92 (3H, m), 5.31 (1H, d, J = 4.8 Hz), 5.50 (1H, t, J = 5.6 Hz), 6.37 (1H, s), 7.05-7.08 (2H, m), 7.15-7.21 (3H, m), 7.54 (1H, d, J = 6.8 Hz), 7.70-7.73 (1H, m), 7.90 (1H, s), 10.40 (1H, s). | 117 |
| 120 | 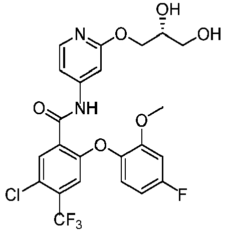 | 531.1 | 1H NMR (400 MHz, d₆-DMSO) δ 3.41-3.44 (2H, m), 3.75 (3H, s), 3.77-3.79 (1H, m), 4.11-4.15 (1H, m), 4.25-4.29 (1H, m), 4.63 (1H, t, *J* = 5.6 Hz), 4.90 (1H, d, *J* = 5.2 Hz), 6.83-6.88 (1H, m), 6.99 (1H, s), 7.13-7.19 (3H, m), 7.28-7.31 (1H, m), 8.03 (1H, s), 8.07 (1H, d, *J* = 5.6 Hz), 10.88 (1H, s). | 114 |
| 121 | 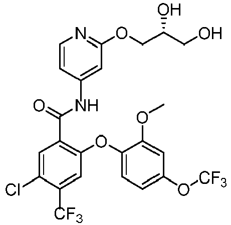 | 597.1 | NA | 114 |
| 122 | 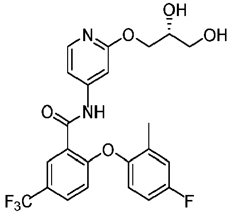 | 481.2 | 1H NMR (400 MHz, d₆-DMSO) δ 2.15 (3H, s), 3.41-3.44 (2H, m), 3.77-3.78 (1H, m), 4.10-4.14 (1H, m), 4.24-4.28 (1H, m), 4.63 (1H, t, *J* = 5.6 Hz), 4.90 (1H, d, *J* = 5.2 Hz), 6.85 (1H, d, *J* = 8.8 Hz), 7.11-7.26 (5H, m), 7.81 (1H, dd, *J* = 8.8 Hz, 2.0 Hz), 8.01 (1H, d, *J* = 1.6 Hz), 8.05 (1H, d, *J* = 6.0 Hz), 10.85 (1H, s). | 114 |

### Pharmacological test

### Test case 1: Compound inhibition activity on human NaV1.8 ion channel in HEK293 cells

### 1: Preparation of test solution

The reagents are purchased from Sigma (St. Louis, MO) except for NaOH and KOH used for acid-base titration. The final concentrations of the test compounds are prepared on the same day and then dissolved in extracellular solution. Extracellular solution (mM) contains: NaCl, 137; KCl, 4; CaCl₂, 1.8; MgCl₂, 1; HEPES, 10; glucose 10; pH 7.4 (NaOH titration). The intracellular solution (mM) contains: Aspartic acid, 140; MgCl₂, 2; EGTA 11; HEPES, 10; pH 7.2 (CsOH titration). All test solutions contain 1 µM TTX.

The test solution are stored at a concentration of 3 mM in dimethyl sulfoxide (DMSO). The solutions will be dissolved in extracellular solution on the day of testing and prepared to the required concentration.

Solvents for test compound

| | |
|---|---|
| Name | Dimethyl Sulfoxide (DMSO) |
| Source | Sigma |

| | |
|---|---|
| Molecular weight | 78.1 g/mol |
| Purity | 99.9% |
| Storage condition | Room temperature |

### 2: Test Method

### 2.1: Cells

All experiments were performed at room temperature. Each cell itself is taken as the corresponding control.

### 2.1.1: Testing of compounds

Compounds are all perfused using a perfusion system that utilizes the gravity. At least one cell is tested per concentration. After current stabilization (or 5 minutes), the change in current magnitude before and after the use of the compound is compared to calculate the blocking effect of the compound.

### 2.1.2: Test cells

HEK293 cell steady expressing NaV1.8 ion channel.

### 2.1.3: Experimental apparatus

Patch clamp amplifier: patch clamp PC-505B (WARNER instruments)/MultiClamp 700A (Axon instrument)
Digital-to-analog converter: Digidata 1440A (Axon CNS)/Digidata 1550A (Axon instruments)
Micromanipulator: MP-225 (SUTTER instrument)
Inverted microscope: TL4 (Olympus)
Glass microelectrode puller: PC-10 (NARISHIGE)
Microelectrode glass capillary: B12024F (Wuhan Microprobe Scientific Instruments Co., Ltd)

### 2.2: Electrophysiology

Transfer Cells to the perfusion tank and perfuse with extracellular fluid. The intracellular fluid (mM) contains: Aspartic acid, 140; MgCl₂, 2; EGTA 11; HEPES, 10; pH 7.2 (CsOH titration). The intracellular fluid is stored in small quantities in portions in a -80°C refrigerator, and melt on the day of the experiment. Electrodes are drawn with PC-10 (Narishige, Japan). Recording is performed with whole-cell diaphragm clamp. Noise is filtered with one-fifth of the sampling frequency.

### 2.3: Test voltage equation (resting) and the results

The cells are clamped at -80 mV and then depolarized to 10 mV with a sustained 10 ms square wave to give a Naᵥ1.8 current. This procedure is repeated every 5 seconds. The maximum current triggered by the square wave is detected, and when the current is stabilized, the test compound is perfused. The strength of the block is calculated when the response is stabilized.

**Table IV: Inhibition ratio of Nav1.8 by compounds (inhibition ratio at 100 nm concentration, shown as %: A>=80, 80>B>=50, 50>C>=20, D<20)**

| Examples | Activity of inhibition | Examples | Activity of inhibition | Examples | Activity of inhibition |
|---|---|---|---|---|---|
| 1 | B | 2 | D | 3 | A |
| 4 | B | 5 | B | 6 | B |
| 7 | A | 8 | A | 9 | A |
| 10 | A | 11 | A | 12 | B |
| 13 | C | 14 | B | 15 | C |
| 16 | D | 17 | D | 18 | D |
| 19 | A | 20 | B | 21 | B |
| 22 | A | 23 | B | 24 | A |
| 25 | C | 26 | C | 27 | A |
| 28 | C | 29 | B | 30 | B |
| 31 | B | 32 | C | 33 | D |
| 34 | B | 35 | A | 36 | A |
| 37 | A | 38 | B | 39 | A |
| 40 | B | 41 | A | 42 | A |
| 43 | B | 44 | B | 45 | NA |
| | | | | VX-150 | B |

**Table V (continuation of IV): Inhibition ratio of compounds against Nav1.8 (inhibition ratio at 100 nm concentration, shown as %: A>=80, 80>B>=50, 50>C>=20, D<20)**

| Examples | Activity of inhibition | Examples | Activity of inhibition | Examples | Activity of inhibition |
|---|---|---|---|---|---|
| 46 | A | 47 | B | 48 | A |
| 49 | B | 50 | C | 51 | A |
| 52 | B | 53 | D | 54 | B |
| 55 | A | 56 | B | 57 | A |
| 58 | B | 59 | C | 60 | D |
| 61 | C | 62 | B | 63 | B |
| 64 | C | 65 | A | 66 | A |
| 67 | A | 68 | C | 69 | C |
| 70 | C | 71 | B | 72 | B |
| 73 | C | 74 | B | 75 | B |
| 76 | A | 77 | A | 78 | A |
| 79 | C | 80 | B | 81 | B |
| 82 | A | 83 | A | 84 | C |
| 85 | C | 86 | C | 87 | B |
| 88 | B | 89 | B | 90 | B |
| 91 | C | 92 | C | 93 | B |
| 94 | B | 95 | C | 96 | C |
| 97 | B | 98 | A | 99 | B |
| 100 | B | 101 | B | 102 | B |
| 103 | A | 104 | A | 105 | B |
| 106 | A | 107 | B | 108 | B |
| 109 | C | 110 | B | 111 | B |
| 112 | C | 113 | A | 114 | A |
| 115 | B | 116 | B | 117 | A |
| 118 | B | 119 | A | 120 | A |
| 121 | A | 122 | B | | |

Several examples of this patent have a more significant activity advantage over VX-150 (clinical phase II), and some other examples have comparable activity.

### Example 2: Inhibition activity test of compound on hERG ion channels in HEK293 cells

This experiment is used as a cardiac safety test of compound.
**1:** Test apparatus
   Patch clamp instrument: PC-505B, MC-700A
   Micromanipulator: MP-225
   Microelectrode puller: PC-10 (Narishige, Japan)
**2:** Solution preparation

The reagents are purchased from Sigma (St. Louis, MO) except NaOH and KOH for acid-base titration. Final concentrations of tested solutions are prepared on the testing day and dissolved in extracellular fluid. The extracellular fluid (mM) contains: NaCl, 137; KCl, 4; CaCl2, 1.8; MgCl2, 1; HEPES, 10; glucose 10; pH 7.4 (NaOH titration). All the test solutions and the control solutions contain 0.3% DMSO. Intracellular fluid (mM) contains: K Aspartate, 130; MgCl2, 5; EGTA 5; HEPES, 10; Tris-ATP 4; pH 7.2 (KOH titration).

### 3: Experimental methods

Cells: all experiments is performed at room temperature. Each cell itself is taken as the corresponding control.

Compounds are all perfused using a perfusion system that utilizes the gravity. At least two cells are tested per concentration. After current stabilization (or 5 minutes), the change in current magnitude before and after the use of the compound is compared to calculate the blocking effect of the compound.

Positive control: The determination of the concentration of Cisapride in positive control is based on its sensitivity to cells, and the concentration with blocking rate about 90% is the optimal concentration used in the positive control. After testing, when Cisapride is 100nM, the blocking rate is about 90%, so the concentration of positive control Cisapride is set at 100nM. The method of operation is the same as that of the test compound.

Electrophysiology: Transfer the Cells to the perfusion tank and perfuse with extracellular fluid. The intracellular fluid (mM) contains: K Aspartate, 130; MgCl2, 5; EGTA 5; HEPES, 10; Tris-ATP 4; pH 7.2 (KOH titration). The intracellular fluid is stored in small quantities in portions in a -80°C refrigerator, and melt on the day of the experiment. Electrodes are drawn with PC-10 (Narishige, Japan). Recording is performed with whole-cell diaphragm clamp. Noise is filtered with one-fifth of the sampling frequency.

Test procedure and the results: Cells are clamped at-80 mV, then depolarized to 40mV with sustained 4-second square waves, and then hyperpolarized to -40mV with sustained 2-second square waves to give hERG tail current (Tail current is given in the attached figure). This process is repeated every 20 seconds. The hERG tail current is the pure hERGcurrent. The maximum tail current triggered by the second square wave is detected, and when the current is stabilized, the test compound is perfused. The strength of the block is calculated when the response is stabilized.

**Table VI: IC50 results for hERG inhibited by compounds**

| Examples | IC50 (uM) | Examples | IC50 (uM) |
|---|---|---|---|
| 1 | 3.2 | 36 | 10.5 |
| 38 | 3.0 | 104 | 3.8 |
| 114 | 22.3 | 116 | 15.6 |
| 117 | 6.0 | 119 | 4.2 |
| VX-150 | 7.0 | | |

The strong inhibition of the compounds on hERG poses serious cardiac safety risks such as prolongation of the cardiac QT interval. As shown from the data in Table 9: The inhibition of some compounds on hERG in this patent is significantly weaker than VX-150 (clinical phase II) and therefore are likely to be of a better cardiac safety profile.

### Test case 3: Compound selectivity test for Nav ion channel subtypes

The aim of this test is to investigate effect of compounds on human Na ion channel cells expressed in steady-state (NaV1.1, NaV1.3, NaV1.4, NaV1.5, NaV1.6, NaV1.7). After Na ion currents is stabilized, the effect can be obtained by comparing the magnitude of Na channel currents before and after the application of the compounds.

### 1: Preparation of test solution

The reagents are purchased from Sigma (St. Louis, MO) except NaOH and KOH for acid-base titrations. Final concentrations of test solutions are prepared on the testing day and dissolved in extracellular fluid. The extracellular fluid (mM) contains: NaCl, 137; KCl, 4; CaCl2, 1.8; MgCl2, 1; HEPES, 10; glucose 10; pH 7.4 (NaOH titration).

The test compound is stored at a concentration of 9 mM in dimethyl sulfoxide (DMSO), and on the day of testing, dissolve in extracellular solution, then prepare to a screening concentration of 1 uM.

### 2: Test Methods

### 2.1: cells

All experiments are performed at room temperature. Each cell itself is taken as the corresponding control.

### 2.1.1 Testing of compounds

Solutions are all perfused using a perfusion system that utilizes the gravity. At least one cell is tested per concentration. After current stabilization (or 5 minutes), the change in current magnitude before and after the use of the compound is compared to calculate the blocking effect of the compound.

### 2.1.2 Experimental cells/reagents

HEK293cells steadily expressing NaV1.1-, NaV1.3-, NaV1.4-, NaV1.5-, NaV1.6- ion channels; CHO cells expressing steadily NaV1.7 ion channel.

### 2.1.3 Experimental apparatus

Patch clamp amplifier: patch clamp PC-505B (WARNER instruments)/MultiClamp 700A (Axon instrument)
Digital-to-analog converter: Digidata 1440A (Axon CNS)/Digidata 1550A (Axon instruments)
Micromanipulator: MP-225 (SUTTER instrument)
Inverted microscope: TL4 (Olympus)
Glass microelectrode puller: PC-10 (NARISHIGE)
Microelectrode glass capillary: B12024F (Wuhan Microprobe Scientific Instruments Co., Ltd.)

### 2.2 Electrophysiology

Transfer the cells to the perfusion tank and perfuse with extracellular fluid. The intracellular fluid (mM) is: Aspartic acid, 140; MgCl₂, 2; EGTA 11; HEPES, 10; pH 7.2 (CsOH titration). The intracellular fluid is stored in small quantities in portions in a -80°C refrigerator, and melte on the day of the experiment. Electrodes are drawn with PC-10 (Narishige, Japan). Recording is performed with whole-cell diaphragm clamp. Noise is filtered with one-fifth of the sampling frequency.

### 2.3 Test voltage equation (resting) and the results

Cells are clamped at -80 mV and then depolarized to -10 mV with a sustained 10 ms square wave to obtain Na ion channel currents (see the figure). This process is repeated every 5 seconds. The maximum current triggered by the square wave is detected, and when the current is stabilized, the test compound is perfused. The strength of the block is calculated after the response is stabilized.

**Table VII: Inhibition ratioes of compounds on Nav subtypes at a concentration of 1uM**

| Examples | Nav1.1 | Nav1.3 | Nav1.4 | Nav1.5 | Nav1.6 | Nav1.7 |
|---|---|---|---|---|---|---|
| 1 | <1% | 13.4% | <1% | 7.4% | <1% | 9.5% |
| 104 | 1.5% | 4.3% | <1% | <1% | 2.8% | 1.3% |
| 114 | 4.8% | 4.4% | 2.7% | 5.9% | 8.3% | <1% |
| 116 | 1.2% | 3.7% | <1% | <1% | 2.6% | <1% |
| 117 | <1% | 1.2% | <1% | 5.0% | <1% | <1% |
| 119 | <1% | <1% | 1.4% | 2.8% | 5.1% | <1% |
| VX-150 | <1% | 2.2% | 3.6% | 15.4% | 1.7% | 8.7% |

It can be seen that some compounds of this patent have good selectivity to Nav ion channel subtypes. In particular, inhibition of Nav1.5 may cause cardiac side effects. The inhibitory effect of some compounds in this patent on Nav1.5 is significantly weaker than that of VX-150 (clinical phase II), and therefore are likely to show a better safety.

### Test case 4: In vivo pharmacokinetic testing of compounds in rats

All SD rats in the test are male and are purchased from Shanghai Sippe-Bk Lab Animal Co., Ltd. Each compound is administered orally (100 mg/kg, 3 rats per group) at single dose to SD rats for pharmacokinetic study. The solutions are prepared on the same day of administration, e.g., the tested compound is suspended in 5% DMSO + 95% saline, get vortexed for 2 min, and sonicated for 5 min to prepare a drug delivery system. The animals are fasted for 10-14 hours before oral administration, and resumed food 4 hours after administration. After oral administration by gavage in SD rats, pharmacokinetic samples are collected from jugular vein at the following time points: 0, 15 min, 30 min, 1 h, 2 h, 4 h, 6 h, 8 h and 24 h. Three whole blood samples are collected at each time point in a volume of about 0.2 mL and anticoagulated using sodium heparin. Blood samples are placed on ice immediately after collection and plasma is separated by centrifugation within 1 h (centrifugation conditions: 6800 rpm, 6 min, 2-8°C). The collected plasma is stored in a -80°C refrigerator prior to analysis.

**Table VIII: Results of pharmacokinetic tests of some compounds at a dose of 100 mg/kg**

| Examples | T1/2 (h) | Cmax (ng/ml) | AUC (ng/ml*h) |
|---|---|---|---|
| 104 | 5.1 | 5145 | 58002 |
| 112 | 6.3 | 8266 | 114951 |
| 114 | 11.7 | 7635 | 153626 |
| 116 | 8.7 | 2566 | 42454 |
| 117 | 3.0 | 9567 | 113972 |
| 119 | 1.9 | 5192 | 44501 |
| VX-150 | 2.2 | 1176 | 13859 |

In pharmacokinetic tests in rat, the control compound VX-150 is a prodrug (WO2015089361, compound 9), after administration, analysis is performed to test its active form (WO2014120808, compound 10). The aforementioned *in vitro* pharmacological tests are performed using the active ingredient (WO2014120808, compound 10).

Active form of VX-150 has very poor solubility and absorption of oral solid dosage form shows unsatisfactory outcome, thus it was developed as a prodrug to solve the solubility problem. Some of the compounds in this patent, however, shows significant pharmacokinetic characteristics in rat without pro-drug modification.

According to the above data, it can be seen that the compounds of the present invention have significant inhibitory effects on Nav1.8 ion channel activity. And some of the compounds have significant advantages over VX-150 in terms of cellular activity, ion channel selectivity, hERG safety, pharmacokinetics in rats, etc. They have broad applications as Nav1.8 inhibitors in the treatment of analgesia, atrial fibrillation, and Buga syndrome.

## Claims

1. A compound is **characterized by** having the structure shown in formula I or its tautomers, mesomers, racemates, enantiomers, diastereoisomers or its mixture form, and pharmaceutically acceptable hydrates, solvates or salts: 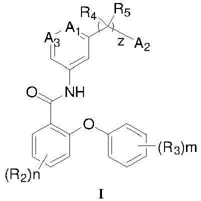
A₁ and A₃ are independently selected from CR₁ or N;
A₂ is selected from NR₁₀R₁₁ or OR₁₂;
R₁, R₂, R₃, R₄, R₅ are independently selected from hydrogen, halogen, hydroxyl, cyano, amino, substituted or unsubstituted alkyl, substituted or unsubstituted cycloalkyl, substituted or unsubstituted heteroalkyl, substituted or unsubstituted heterocycloalkyl, substituted or unsubstituted aryl, substituted or unsubstituted heteroaryl, substituted or unsubstituted acylamino, ester, acyl, carboxyl, sulfonyl, sulfonamido, boronic acid group, boronic ester group, phosphoryl, substituted or unsubstituted alkenyl, substituted or unsubstituted alkynyl;
R₁₀, R₁₁, R₁₂ are independently selected from hydrogen, halogen, hydroxy, cyano group, amino, substituted or unsubstituted alkyl, substituted or unsubstituted cycloalkyl, substituted or unsubstituted heteroalkyl, substituted or unsubstituted heterocycloalkyl, substituted or unsubstituted aryl, substituted or unsubstituted heteroaryl, ester, acyl, carboxyl, acylamino, sulfonyl, sulfonamido, boronic acid group, boronic acid ester group, and phosphoryl group;
Or, R₄, R₅ and their connected atoms constitute a substituted or non-substituted 3 to 10-membered cycloalkyls and =O; or, R₄, R₅ and its connected atoms constitute substituted or non-substituted 3 to 10-membered heterocycloalkyls; or R₅, Rn and their connected atoms constitute substituted or non-substituted 3 to 10-membered heterocycloalkyls, or R₅, R₁₂ and their connected atoms constitute substituted or non-substituted 3 to 10-membered heterocycloalkyls;
Wherein, R₁, R₂, R₃, R₄, R₅, R₁₀, R₁₁, R₅, R₁₁ and the substituents in the heterocycloalkyl groups formed by the atoms attached thereto are independently selected from deuterium, halogen, hydroxyl, amino, alkyl, heteroalkyl, cycloalkyl, heterocycloalkyl, cyano group, ester, acyl, carboxyl, amido, aryl, heteroaryl, sulfonyl, sulfonamido;
Z is selected from 0, 1, 2, 3, 4, and 5, n is selected from 0, 1, 2, 3, and 4, and m is selected from 0, 1, 2, 3, 4 and 5;
Further, n is selected from 0, 1, and 2, m is selected from 0, 1, and 2; further, z is selected from 0, 1, 2, and 3.

2. The compound stated in claim 1 is **characterized by** having the structure shown in formula II or II' or their tautomers, mesomeres, racemates, enantiomers, diastereoisomers or its mixture form, and pharmaceutically acceptable hydrates, solvates or salts:

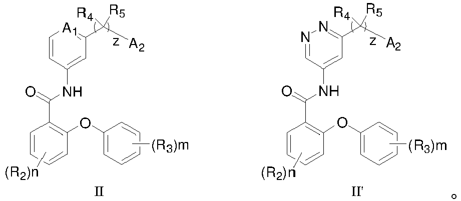

3. The compound stated in claim 1 or 2 is **characterized by** having the structure shown in formula III or III' or their isomers, tautomers, mesomeres, racemates, enantiomers, diastereoisomers or its mixture form, and pharmaceutically acceptable hydrates, solvates or salts: 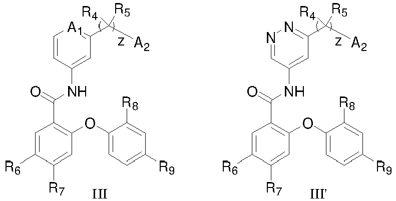
A₁ is selected from CR₁ or N; A₂ is selected from NR₁₀R₁₁ or OR₁₂;
R₁ is selected from hydrogen, halogen, hydroxyl, cyano group, amino, substituted or unsubstituted alkyl, substituted or unsubstituted cycloalkyl, substituted or unsubstituted heteroalkyl, substituted or unsubstituted heterocycloalkyl, ester group, acyl, carboxyl, acylamino, sulfonyl, sulfonamido, boronic group, boronic ester group, phosphoryl;
R₆, R₇, R₈, R₉ are independently selected from hydrogen, halogen, hydroxyl, cyano group, amino, substituted or unsubstituted alkyl, substituted or unsubstituted cycloalkyl, substituted or unsubstituted heteroalkyl, substituted or unsubstituted heterocycloalkyl, substituted or unsubstituted aryl, substituted or unsubstituted substituted heteroaryl, ester group, acyl, carboxyl group, acylamino group, sulfonyl group, sulfonamido group, boronic acid group, boronic acid ester group, phosphoryl group, substituted or non-substituted alkenyl group, and substituted or non-substituted alkynyl group; wherein the substituent is selected from deuterium, halogen, hydroxyl group, amino group, alkyl group, heteroalkyl group, cycloalkyl group, heterocycloalkyl group, cyano group, ester group, acyl group, carboxyl group, acylamino group, aryl group, heteroaryl group, sulfonyl group, sulfonamido group.

4. The compound stated in claim 2 or 3 is **characterized by** that,
Each occurrence of R₄ and R₅ is independently selected from hydrogen, halogen, hydroxyl, cyano group, amino, substituted or unsubstituted alkyl, substituted or unsubstituted cycloalkyl, substituted or unsubstituted heteroalkyl, substituted or unsubstituted heterocycloalkyl, substituted or unsubstituted acylamino, ester, acyl, carboxyl, sulfonyl, sulfonamido; or, R₄, R₅ and their connected atoms constitute substituted or non-substituted 3 to 6-membered cycloalkyls; or, R₄, R₅ and their connected atoms constitute substituted or non-substituted 3 to 6-membered heterocycloalkyls; or R₅, Rn and their connected atoms constitute a substituted or unsubstituted 3 to 6-membered heterocycloalkyl, or R ₄ and R ₅ and their connected atoms form =O; or R₅, R₁₂ and their connected atoms constitute substituted or unsubstituted 3 to 6-membered heterocycloalkyls; wherein the substituents are selected from halogen, hydroxyl, amino, C1-C6 alkyls, 3 to 6-membered cycloalkyls, 2 to 7-membered heteroalkyls, 3 to 6-membered heterocycloalkyls, cyano group, ester, acyl, carboxyl, amido, aryl, heteroaryl, sulfonyl, sulfonamido; at least one of R₄ and R₅ is H;
Further, each occurrence of R₄, R₅ is independently selected from hydrogen, halogen, hydroxyl, cyano group, amino, substituted or non-substituted alkyl, substituted or non-substituted cycloalkyl, substituted or non-substituted heteroalkyl, substituted or non-substituted heterocycloalkyl, substituted or non-substituted acylamino; or, R₄, R₅ and their connected atoms constitute substituted or non-substituted 3 to 6-membered cycloalkyls and =O; or, R₄, R₅ and their connected atoms constitute substituted or non-substituted 3 to 6-membered heterocycloalkyls, or R₅, R₁₁ and their connected atoms constitute substituted or non-substituted 3 to 6-membered heterocycloalkyls, wherein, the substituent is selected from halogen, hydroxyl, amino, C1-C6 alkyl, 3 to 6-membered cycloalkyls, 2 to 7-membered heteroalkyls, 3 to 6-membered heterocycloalkyls, cyano group, ester, acyl, carboxyl, amido, sulfonyl, sulfonamido; at least one of R₄ and R₅ is H.

5. The compound stated in claim 4 is **characterized by** that, each occurrence of R₄ and R₅ is independently selected from hydrogen, halogen, substituted or unsubstituted C1-C6 alkyl, substituted or unsubstituted 3 to 6-membered cycloalkyls, substituted or unsubstituted 2 to 7-membered heteroalkyls, substituted or unsubstituted 3 to 6-membered heterocyclic alkyls, substituted or unsubstituted acylamino groups; or, R₄, R₅ and their connected atoms constitute =O; or, R₅, R₁₁ and their connected atoms constitute substituted or unsubstituted 3 to 6-membered heterocycloalkyls, wherein, the substituent is selected from halogen, hydroxyl, amino, C1-C6 alkyl, 3 to 6-membered cycloalkyls, 2 to 7-membered heteroalkyls, 3 to 6-membered heterocycloalkyls, acyl, carboxyl, and acylamino;
Further, R₄ and R₅, at each occurrence, are independently selected from hydrogen, halogen, substituted or unsubstituted C1-C3 alkyl, substituted or unsubstituted 2- to 4-membered heteroalkyls, substituted or unsubstituted 5-membered heterocycloalkyls, substituted or unsubstituted acylamino; or R₄, R₅ and their connected atoms constitute =O; or, R₅, Rn and their connected atoms constitute substituted or nonsubstituted 5- or 6-membered heterocycloalkyls, wherein, the substituent is selected from halogen, hydroxyl, amino, C1-C6 alkyl, 3 to 6-membered cycloalkyls, 2 to 7-membered alkoxy groups, 3 to 6-membered heterocycloalkyl, acyl, carboxyl, acylamino; at least one of R₄ and R₅ is H.

6. The compound stated in claim 5 is **characterized by** that R₄ and R₅, at each occurrence, is independently selected from hydrogen, halogen, substituted or unsubstituted C1-C3 alkyl, substituted or unsubstituted 2- to 4-membered oxoalkyls, substituted or unsubstituted acylamino group, and 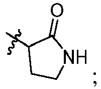 or, R₄, R₅ and their connected atoms sonstitute = O; or, R₅, R₁₁ and their connected atoms constitute substituted or non-substituted 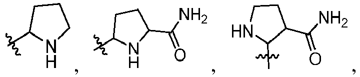 or 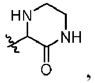 wherein, the substituent is selected from halogen, hydroxyl, amino, C1-C3 alkyl, 2-to 4-membered alkoxy groups, carboxyl, and acylamino group; at least one of R₄ and R₅ is H;
Further, R₄ and R₅, at each occurrence, is independently selected from hydrogen, halogen, methyl, 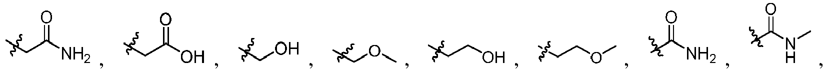 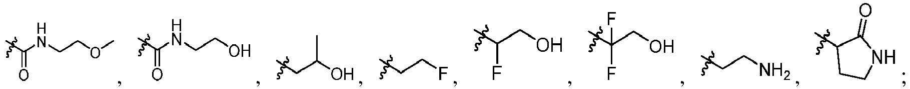 or, R₄, R₅ and their connected atoms constitute = O; or, R₅, R₁₁ and their connected atoms constitute 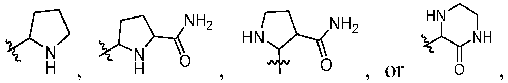 wherein, the substituents are selected from halogen, hydroxyl, amino, C1-C3 alkyl, 2- to 4-membered alkoxy groups, 3 to 6-membered heterocycloalkyls, acyl, carboxyl, acylamino;
Further, R₄ and R₅, at each occurrence, is independently selected from hydrogen, methyl, 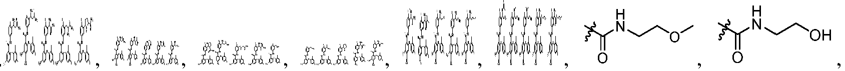 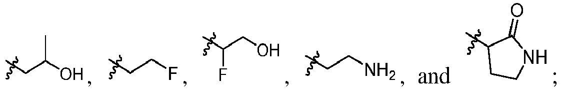 or, R₄ and R₅ and their connected atoms constitute =O; wherein the substituents are selected from halogen, hydroxyl, amino, C1-C3 alkyl, 2- to 4-membered alkoxy groups, acyl, acylamino; at least one of R₄ and R₅ is H;
Further, R₄ and R₅, each time they exist, are independently selected from hydrogen, methyl, 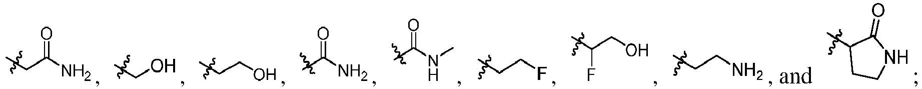 or, R₄, R₅ and their attached atoms constitute = O; at least one of R₄ and R₅ is H.

7. The compound stated in one of claims 1 to 6 is **characterized by** that when A₂ is selected from NR₁₀R₁₁, z is selected from 1, 2, and 3, preferably 1 or 2; When A₂ is selected from OR₁₂, z is selected from 0, 1, 2, and 3, preferably 0.

8. The compound stated in one of claims 3 to 7 is **characterized by** that R₁₀, Rn, and R₁₂ are independently selected from hydrogen, halogen, hydroxyl, cyano group, substituted or unsubstituted alkyl, substituted or unsubstituted cycloalkyl, substituted or unsubstituted heteroalkyl, substituted or unsubstituted substituted heterocycloalkyl, ester, acyl, acylamino, sulfonyl, sulfonamido, boronic acid group, boronic acid ester group, phosphoryl group, wherein, the substituents are selected from halogen, hydroxyl group, amino group, C1-C6 alkyl group, 3 to 6-membered cycloalkyls, 2 to 7-membered heteroalkyls, 3 to 6-membered heterocycloalkyls, cyano group, ester, acyl, carboxyl, acylamino, aryl, heteroaryl, sulfonyl, sulfonamido;
Or, Rn and R₅ and their connected atoms form a ring-substituted or unsubstituted heterocycloalkyl group, from which limites scope in the referenced claim.

9. The compound stated in claim 8 is **characterized by** that R₁₀, Rn, and R₁₂ are independently selected from hydrogen, halogen, hydroxyl, cyano group, substituted or non-substituted C1-C6 alkyl, substituted or non-substituted 3 to 6-membered cycloalkyls, substituted or non-substituted 2 to 7-membered metaheteroalkyls, substituted or unsubstituted 3 to 6 metaheterocycloalkyls, ester group, acyl group, acylamino, wherein the substituent is selected from halogen, hydroxyl, amino, C1-C6 alkyl, 3 to 6 meta-cycloalkyl, 2 to 7 metaheteroalkyl, 3 to 6 metaheterocycloalkyl, cyano group, ester, acyl, carboxyl, acylamino, sulfonyl, sulfonamido;
Or, the scope of substituted or non-substituted heterocycloalkyl constituted by Rn, R₅ and their connected atoms is limited from the claims cited therein.

10. The compound stated in claim 9 is **characterized by** that R₁₀, R₁₁, and R₁₂ are independently selected from hydrogen, substituted or non-substituted C1-C3 alkyl, substituted or non-substituted 2- to 4-membered heteroalkyls, acyl, amido, wherein the substituent is selected from halogen, hydroxyl, amino, C1-C6 alkyl, 3 to 6-membered cycloalkyls, 2 to 7-membered heteroalkyls, 3 to 6-membered heterocycloalkyls, cyano group, ester, acyl, acylamino, sulfonyl, sulfonamido;
Or, the scope of substituted or non-substituted heterocycloalkyl constituted by Rn, R₅ and their connected atoms is limited from the claims cited therein;
Further, R₁₀ and Rn are independently selected from hydrogen, substituted or non-substituted C1-C3 alkyl, acyl, wherein, the substituent is selected from halogen, hydroxyl, amino, C1-C6 alkyl, 3 to 6-membered cycloalkyls, 2 to 7-membered heteroalkyls, 3 to 6-membered heterocycloalkyls, ester group, acyl, acylamino;
Or, the scope of substituted or non-substituted heterocycloalkyl constituted by Rn, R₅ and their connected atoms is limited from the claims cited therein;
R₁₂ is selected from hydrogen, substituted or non-substituted C1-C3 alkyl;
The substituents in R₁₀, Rn, R₁₂ are selected from halogen, hydroxyl, amino, C1-C6 alkyl, 3 to 6-membered cycloalkyls, 2 to 7-membered heteroalkyls, 3 to 6-membered heterocycloalkyls, ester group, acyl, acylamino;
Further, R₁₀ and Rn are independently selected from hydrogen, methyl, 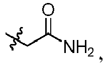 and 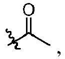 preferably seclected from hydrogen, methyl, 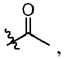 more preferably seclected from hydrogen, methyl; R₁₂ is selected from hydrogen, 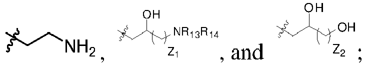 wherein, R₁₃ and R₁₄ are independently selected from hydrogen, acyl, substituted or unsubstituted alkyl, substituted or unsubstituted cycloalkyl, substituted or unsubstituted heterocycloalkyl, substituted or unsubstituted heteroaryl, and Z1 and Z2 are independently 1-5; further, R₁₃ and R₁₄ are independently selected from hydrogen, acyl, 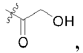 C1-C5 alkyl or cycloalkyl, and thiazole.

11. The compound stated in any claims 3 to 10 is **characterized by** that at least one of R₁₀ and R₁₁ is H.

12. The compound stated in claim 1 is **characterized by** having the structure shown in formula IV, IV' or IV" or their isomers, tautomers, mesomers, racemates, enantiomers, diastereoisomers or its mixture form, and pharmaceutically acceptable hydrates, solvates or salts: 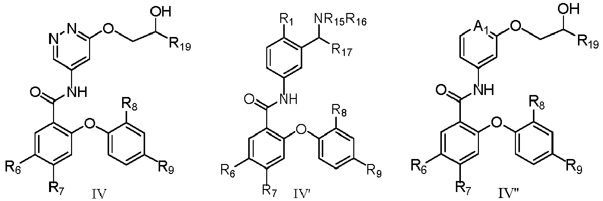 Wherein,
A₁ is selected from N or -CR₁; R₁₅ and R₁₆ are independently selected from hydrogen, C1-C3 alkyl, further selected from hydrogen, and methyl;
R₁₇ is selected from C1-C5 acylamino, 4-membered or 5-membered heterocyclic ring containing lactam, C1-C5 alkyl substituted by at least hydroxyl or amino; or, one of R₁₅ and R₁₆, composing R₁₇ and its connected atoms, constitute 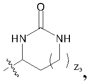 Z₃ is selected from 0, 1 or 2; further, R₁₇ is selected from C1-C3 acylamino, 4-membered or 5-membered heterocyclic ring containing lactam, C1-C3 alkyl substituted by at least hydroxyl or amino; or, either R₁₅ or R₁₆, R₁₇ and their attached atoms constitute 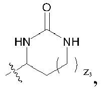 Z₃ is selected from 0, 1 or 2;
R₁₉ is selected from 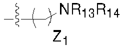 and 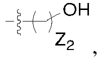 R₁₃ and R₁₄ are independently selected from hydrogen, acyl, substituted or non-substituted alkyl, substituted or non-substituted cycloalkyl, substituted or non-substituted heterocycloalkyl, substituted or non-substituted heteroaryl, Z₁ and Z₂ are independently 1-5; further, R₁₃ and R₁₄ are independently selected from hydrogen, acyl, 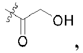 C1-C5 alkyl or cycloalkyl, thiazole.

13. The compound stated in claims 3 to 12 is **characterized by** that R₆ and R₇ are independently selected from hydrogen, halogen, hydroxyl, cyano group, amino, substituted or unsubstituted alkyl, substituted or unsubstituted cycloalkyl, substituted or unsubstituted heteroalkyl, substituted or unsubstituted heterocycloalkyl, ester group, acyl, carboxyl, acylamino, sulfonyl, sulfonamido, boronic acid group, boronic acid ester group, phosphoryl, substituted or unsubstituted alkenyl, and substituted or unsubstituted alkynyl;
Wherein, the substituents are selected from halogen, hydroxyl, amino, C1-C6 alkyl, 3 to 6-membered cycloalkyls, 2 to 7-membered heteroalkyls, 3 to 6-membered heterocycloalkyls, cyano group, ester, acyl, amido, aryl, heteroaryl, sulfonyl, sulfonamido.

14. The compound stated in claim 13 is **characterized by** that R₆ and R₇ are independently selected from hydrogen, halogen, hydroxyl, cyano group, amino, substituted or unsubstituted C1-C6 alkyl, substituted or unsubstituted 3 to 10-membered cycloalkyls, substituted or unsubstituted 2 to 7-membered heteroalkyls, substituted or unsubstituted 3 to 10-membered heterocycloalkyls, ester group, acyl, phosphoryl, substituted or unsubstituted alkenyl, substituted or unsubstituted alkynyl;
Wherein, the substituents are selected from halogen, hydroxyl, amino, C1-C6 alkyl, 2 to 7-membered heteroalkyls, cyano group, ester, acyl, amido, aryl, heteroaryl, sulfonyl, sulfonamido;
Further, R₆ and R₇ are independently selected from hydrogen, halogen, substituted or non-substituted C1~C3 alkyl, substituted or non-substituted cyclopropyl, and R₆ and R₇ are not simultaneously H;
Further, R₆ and R₇ are independently selected from hydrogen, halogen, trifluoromethyl, substituted or non-substituted cyclopropyl, and R₆ and R₇ are not simultaneously H.

15. The compound stated in any one of claims 3 to 14 is **characterized by** that R₈ and R₉ are independently selected from hydrogen, halogen, substituted or unsubstituted alkyl, substituted or unsubstituted cycloalkyl, substituted or unsubstituted heteroalkyl, substituted or unsubstituted heterocycloalkyl, hydroxy, cyano group, amino, ester, acyl, amido, sulfonamido, wherein, the substituents are selected from deuterium, alkyl, heteroalkyl, cycloalkyl, heterocycloalkyl, halogen, hydroxy, cyano group, amino, acyl, and sulfonamido.

16. The compound stated in claim 15 is **characterized by** that R₈ and R₉ are independently selected from hydrogen, halogen, substituted or non-substituted alkyl, substituted or non-substituted cycloalkyl, substituted or non-substituted heteroalkyl, substituted or non-substituted heterocycloalkyl, respectively, wherein, the substituents are selected from deuterium, C1-C6 alkyl, 2 to 7-membered heteroalkyls, 3 to 6-membered cycloalkyls, 3 to 6-membered heterocycloalkyls, halogen, hydroxyl, and amido;
Further, R₈ and R₉ are independently selected from hydrogen, halogen, substituted or non-substituted C1~C6 alkyl, substituted or non-substituted C1~C6 alkoxy, wherein, the substituents are selected from deuterium, halogen, C1~C6 alkyl, 2 to 7-membered heteroalkyls, 3 to 6-membered cycloalkyls, 3- to 6- membered heterocycloalkyls, hydroxyl, and acylamino.

17. The compound stated in claim 16 is **characterized by** that R₈ is selected from halogen, substituted or non-substituted C1-C3 alkyl, substituted or non-substituted C1-C3 alkoxy; preferably selected from substituted or non-substituted C1-C3 alkyl; more preferably selected from methyl;
R₉ is selected from halogen, methyl, trifluoromethyl, and substituted or non-substituted alkoxy; preferably selected from F, Cl, Br, methyl, trifluoromethyl, methoxy, and trifluoromethoxy; more preferably selected from F.

18. The compound stated in claim 1 is **characterized by** that the compound has the structure shown in formula V or V' or their isomers, tautomers, mesomers, racemates, enantiomers, diastereoisomers or its mixture form, and pharmaceutically acceptable hydrates, solvates or salts: 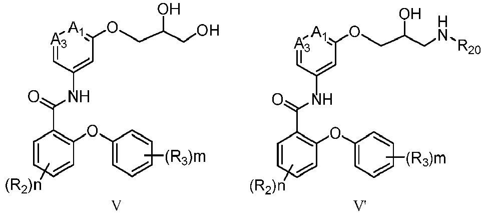
Wherein, A₁ and A₃ are selected from CR1; or, A₁ is selected from N and A₃ is selected from N or CR1;
R₂₀ is independently selected from hydrogen, acyl, 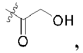 C1-C5 alkyl or cycloalkyl, and thiazole.

19. The compound stated in claim 18 is **characterized by** that R₁ is selected from hydrogen, halogen, substituted or non-substituted C1-C3 alkyl, and substituted or non-substituted 2- to 4-membered heteroalkyls; wherein, the substituent is selected from halogen, hydroxyl, amino, C1-C3 alkyl, 2- to 4-membered heteroalkyls, and acyl;
Further, R₁ is selected from hydrogen, halogen, preferably H and F.

20. The compound stated in one of claims 1, 2 or 18 is **characterized by** that: n is 1 or 2, each R₂ is independently selected from hydrogen, halogen, substituted or non-substituted C1-C3 alkyl, and substituted or non-substituted cyclopropyl; further, each R₂ is independently selected from hydrogen, halogen, trifluoromethyl, and substituted or non-substituted cyclopropyl; when n is 1, R₂ is not H; When n is 2, the two R₂s are not H simultaneously.

21. The compound stated in one of claims 1, 2 or 18 is **characterized by** that: m is 2 or 3, each R₃ is independently selected from halogen, substituted or non-substituted alkyl, substituted or non-substituted cycloalkyl, substituted or non-substituted heteroalkyl, and substituted or non-substituted heterocycloalkyl, wherein, the substituents are selected from deuterium, C1-C6 alkyl, 2 to 7-membered hetero alkyls, 3 to 6-membered cycloalkyls, 3 to 6-membered heterocycloalkyls, halogen, hydroxyl, and acylamino; each R₃ is different;
Further, each R₃ is independently selected from hydrogen, halogen, substituted or non-substituted C1-C6 alkyl, and substituted or non-substituted C1-C6 alkoxy, wherein, the substituents are selected from deuterium, halogen, C1-C6 alkyl, 2 to 7-membered heteroalkyls, 3 to 6-membered cycloalkyls, 3 to 6-membered heterocycloalkyls, hydroxyl, and acylamino;
Even further, each R₃ is independently selected from halogen, substituted or non-substituted C1-C3 alkyl, and substituted or non-substituted C1-C3 alkoxy.

22. The compound stated in one of claims 1-21 is **characterized by** that: the stated halogen is selected from F, Cl and Br; the substituted or non-substituted C1-C3 alkyl is selected from methyl and trifluoromethyl; and the substituted or non-substituted C1-C3 alkoxy is selected from methoxy and trifluoromethoxy.

23. The compound stated in claim 1 is **characterized by** that the stated compound structure is selected from one of the following:

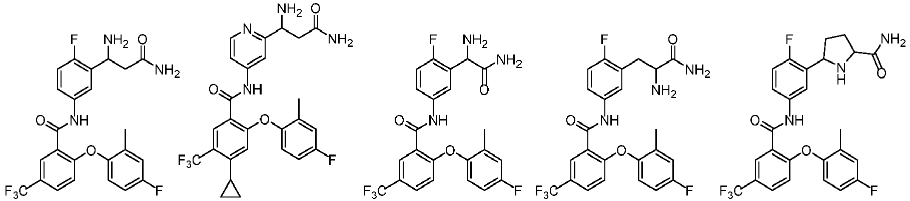

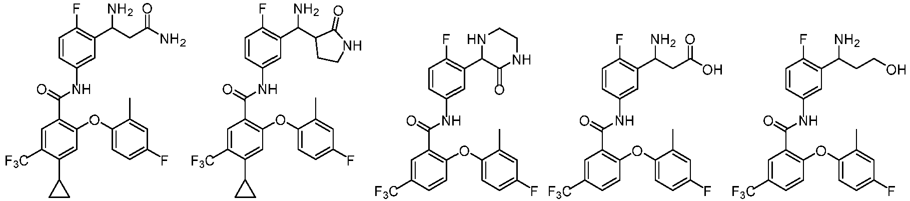

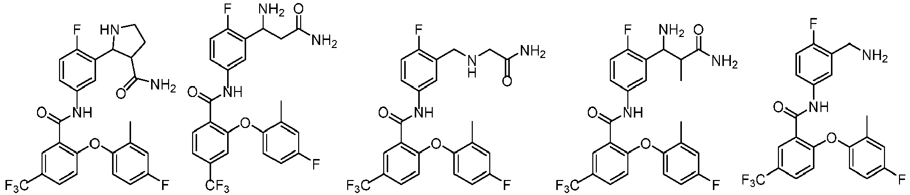

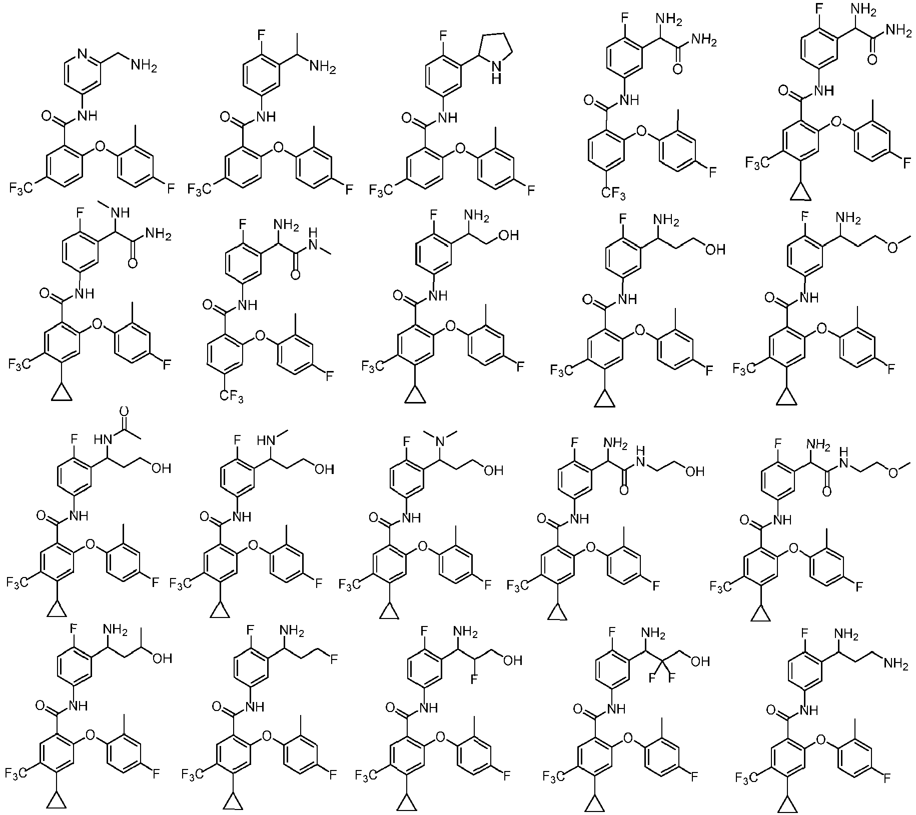

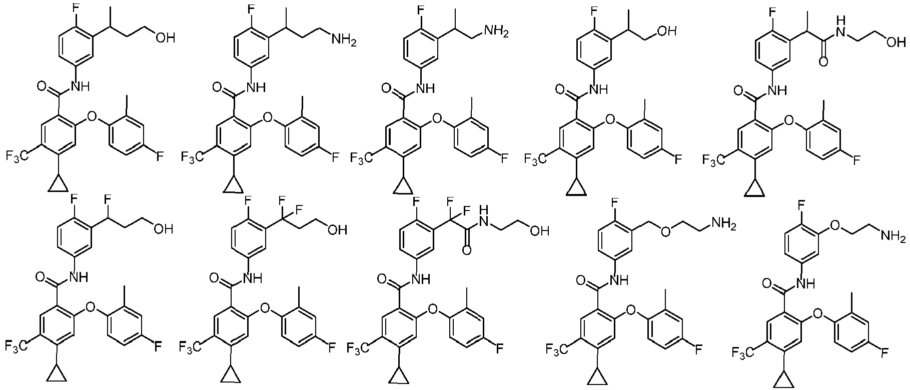

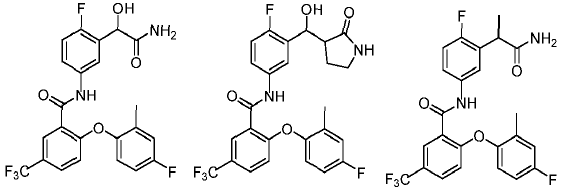

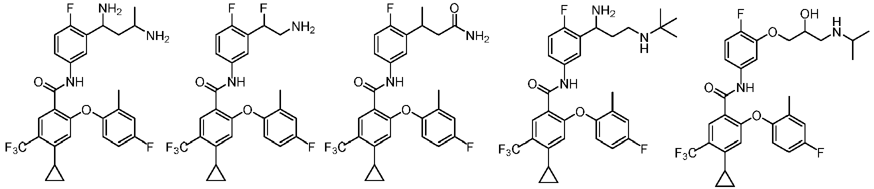

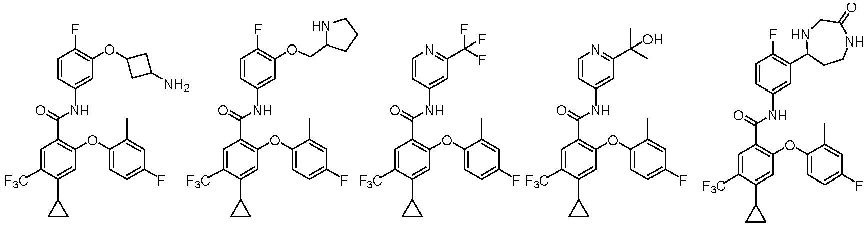

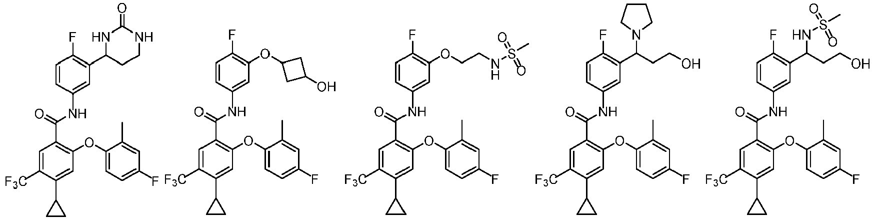

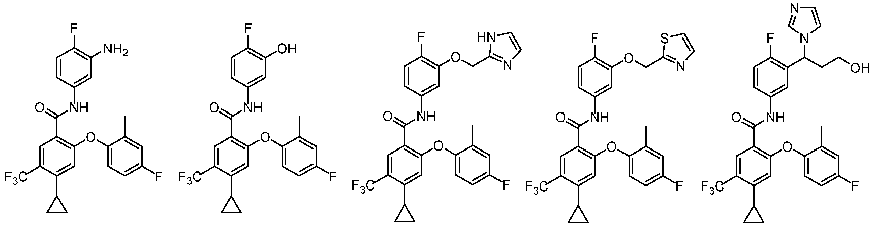

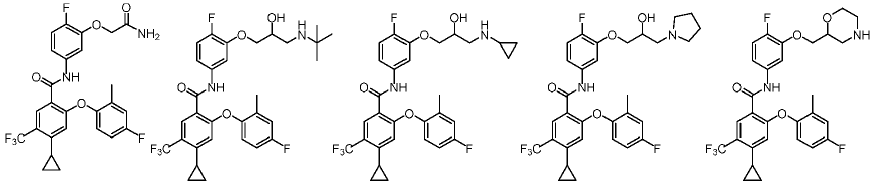

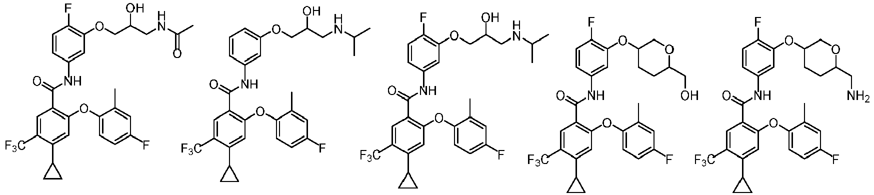

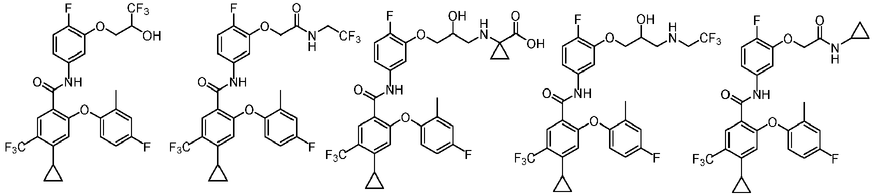

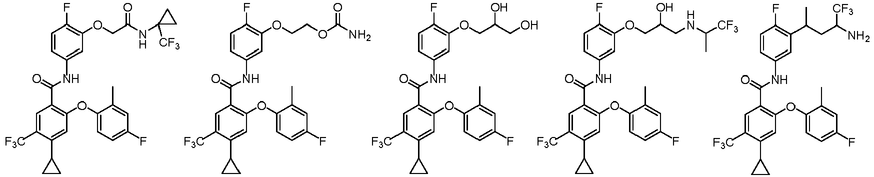

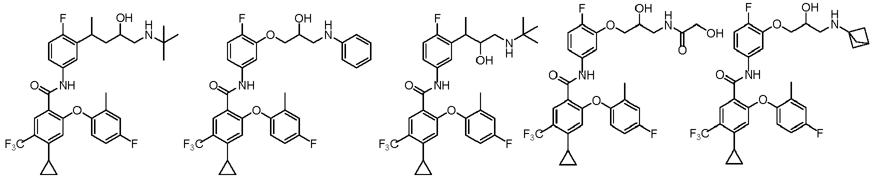

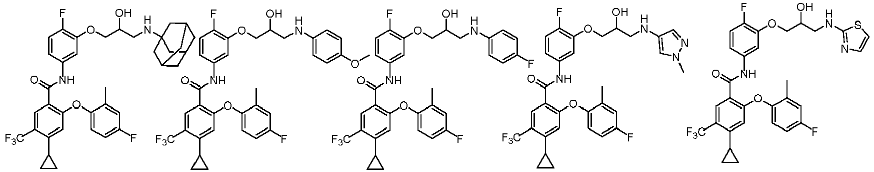

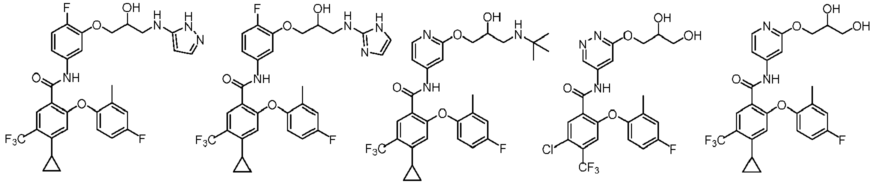

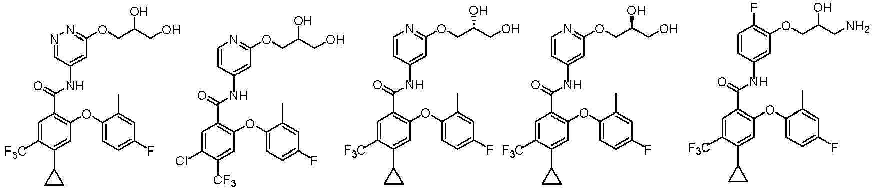

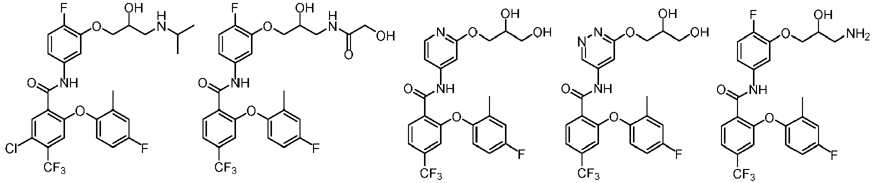

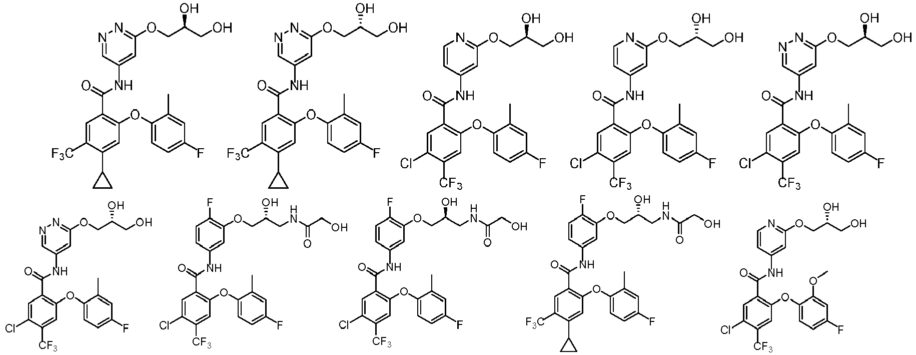

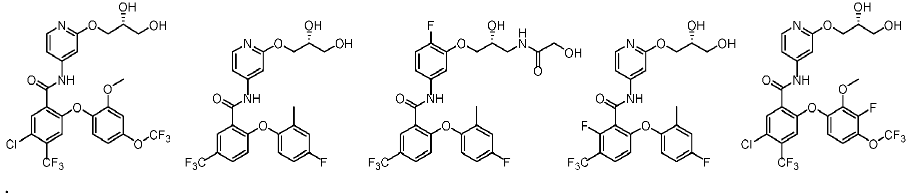

24. A pharmaceutical composition is **characterized by** that the active ingredient of the pharmaceutical composition is a combination from one or more the following stated in claims 1 to 25: the compounds or their stereoisomers, solvates, hydrates, and pharmaceutically acceptable salts or co-crystals.

25. Use of the compounds described in any one of claims 1 to 23 or their stereoisomers, solvates, hydrates, and pharmaceutically acceptable salts or co-crystals in the preparation of sodium channel regulator; further, the stated sodium channel regulator is a Nav1.8 inhibitor.

26. Use of the compounds described in any one of claims 1 to 23 or their stereoisomers, solvates, hydrates, and pharmaceutically acceptable salts or co-crystals in the preparation of drugs for the treatment of diseases causing Nav1.8 overexpression.

27. Use of the compounds described in any one of claims 1 to 23 or their stereoisomers, solvates, hydrates, and pharmaceutically acceptable salts or co-crystals in the preparation of drugs for the treatment of diseases caused by Nav1.8 overexpression.

28. The compound described in any one of claims 1 to 23 or their stereoisomers, solvates, hydrates, and pharmaceutically acceptable salts or co-crystals are prepared for the treatment of the following diseases: chronic pain, intestinal pain, neuropathic pain, musculoskeletal pain, acute pain, inflammatory pain, cancer pain, primary pain, multiple sclerosis, Charcot-Marie-Tooth syndrome, incontinence, and cardiac arrhythmia.

29. The use stated in claim 28 is **characterized by** that the stated neuropathic pains are selected from one or more of the following diseases: postherpetic neuralgia, diabetic neuralgia, painful HIV-associated sensory neuropathy, trigeminal neuralgia, burning mouth syndrome, post-amputation pain, phantom pain, painful neuroma, traumatic neuroma, Morton's neuroma, nerve crush injury, spinal canal stenosis, carpal tunnel syndrome, radiculalgia, sciatica, nerve avulsion injury, brachial plexus avulsion, complex regional pain syndrome, neuralgia caused by drug therapy, cancer chemotherapy and antiretroviral therapy, pain after spinal cord injury, primary small fiber neuropathy, primary sensory neuropathy, trigeminal autonomic headache;
The stated musculoskeletal pains are selected from one or more of the following: osteoarthritis pain, back pain, cold pain, burn pain, and dental pain;
The stated inflammatory pain is selected from rheumatoid arthritis pain and/or vulvodynia;
The stated primary pain is selected from fibromyalgia.
